# EUROPEAN PATENT SPECIFICATION

(11) **EP 4 342 986 B1**
(45) Date of publication and mention of the grant of the patent: **19.08.2026**
(21) Application number: 22807909.1
(22) Date of filing: 13.05.2022
(51) Int. Cl.: C12N 15/10, C12N 15/113, C12N 9/22, C12N 15/63

(54) **COMPOSITION AND METHOD FOR TREATMENT OF LCA10 USING RNA-GUIDED NUCLEASE**
ZUSAMMENSETZUNG UND VERFAHREN ZUR BEHANDLUNG VON LCA10 UNTER VERWENDUNG VON RNA-GEFÜHRTER NUKLEASE
COMPOSITION ET MÉTHODE DE TRAITEMENT DE LCA10 À L'AIDE D'UNE NUCLÉASE GUIDÉE PAR ARN

(30) Priority: 14.05.2021 KR 20210063023
(43) Date of publication of application: 27.03.2024
(73) Proprietor: Genkore Inc, Yuseong-gu, Daejeon 34141 (KR)
(72) Inventor: KIM, Yong-Sam, Daejeon 34118 (KR); KIM, Do Yon, Daejeon 34069 (KR); PARK, Seyeon, Daejeon 34141 (KR)
(74) Representative: Dehns
(86) International application number: PCT/KR2022/006940
(87) International publication number: WO 2022/240262

(56) References cited:
- WO-A1-2020/027982
- WO-A1-2020/223634
- WO-A1-2022/075808
- WO-A1-2022/075816
- CN-A- 111 363 763
- KR-A- 20190 058 358
- KR-A- 20210 053 228
- US-A1- 2020 172 886
- MAEDER MORGAN L ET AL: "Development of a gene-editing approach to restore vision loss in Leber congenital amaurosis type 10", NATURE MEDICINE(AUTHOR MANUSCRIPT ), NATURE PUBLISHING GROUP US, NEW YORK, vol. 25, no. 2, 21 January 2019 (2019-01-21), pages 229 - 233, XP036693196, ISSN: 1078-8956, [retrieved on 20190121], DOI: 10.1038/S41591-018-0327-9
- KIM DO YON ET AL: "Efficient CRISPR editing with a hypercompact Cas12f1 and engineered guide RNAs delivered by adeno-associated virus", NATURE BIOTECHNOLOGY, NATURE PUBLISHING GROUP US, NEW YORK, vol. 40, no. 1, 2 September 2021 (2021-09-02), pages 94 - 102, XP037667066, ISSN: 1087-0156, [retrieved on 20210902], DOI: 10.1038/S41587-021-01009-Z
- BIN MOON SU, LEE JEONG MI, KANG JEONG GU, LEE NAN-EE, HA DAE-IN, KIM DO YON, KIM SUN HEE, YOO KWANGSUN, KIM DAESIK, KO JEONG-HEON,: "Highly efficient genome editing by CRISPR-Cpf1 using CRISPR RNA with a uridinylate-rich 3′-overhang", NATURE COMMUNICATIONS, vol. 9, no. 1, 1 December 2018 (2018-12-01), XP055785455, DOI: 10.1038/s41467-018-06129-w
- LUCAS B. HARRINGTON, DAVID BURSTEIN, JANICE S. CHEN, DAVID PAEZ-ESPINO, ENBO MA, ISAAC P. WITTE, JOSHUA C. COFSKY, NIKOS C. KYRPID: "Programmed DNA destruction by miniature CRISPR-Cas14 enzymes", SCIENCE, AMERICAN ASSOCIATION FOR THE ADVANCEMENT OF SCIENCE, US, vol. 362, no. 6416, 16 November 2018 (2018-11-16), US , pages 839 - 842, XP055614750, ISSN: 0036-8075, DOI: 10.1126/science.aav4294

## Description

### Technical Field

The present disclosure relates to treatment of LCA10 disease using a CRISPR/Cas12f1 (Cas14a1) system. More specifically, the present disclosure relates to a composition for treatment of LCA10 disease, comprising a CRISPR/Cas12f1 (Cas14a1) system, and a treatment method using the same. In addition, the present disclosure relates to a composition for artificially manipulating the human CEP290 gene, the composition comprising a CRISPR/Cas12f1 (Cas14a1) system, and a method of manipulating the CEP290 gene using the same.

### Background Art

Leber congenital amaurosis type 10 (LCA10) is an autosomal recessive disease caused by a biallelic loss-of-function mutation in the CEP290 gene. The most common mutation in the CEP290 gene, which causes LCA10, is an adenine to guanine point mutation located within intron 26 of the CEP290 gene (called c.2991 + 1655A>G), and this mutation creates a new splice donor site so that a region for a 128 base pair cryptic exon is included in the messenger RNA (mRNA), thereby generating a premature stop codon. Currently, there is no approved treatment for LCA10. Gene replacement therapy may be a potential treatment. However, due to a large size (about 7.5 kb) of the normal CEP290 gene, there is a limitation in packaging of adeno-associated virus (AAV), which makes it difficult to develop a therapeutic agent. In order to overcome such limitation, treatment using a CRISPR/Cas9 system is receiving attention and is being developed.

WO2020/027982A1 relates to compositions and methods for treating CEP290-associated disease.

Maeder M. L. et al., (2019) Nature Medicine 25(2) relates to the development of a gene editing approach to restore vision loss in Leber congenital amaurosis type 10.

WO2020/223634A1 relates to programmable nucleases and methods of use.

### Disclosure

### Technical Problem

An object of the present disclosure is to provide a CRISPR/Cas12f1 (Cas14a1) system for treatment of LCA10 disease.

Another object of the present disclosure is to provide a composition for treatment of LCA10 disease, comprising a CRISPR/Cas12f1 (Cas14a1) system.

Yet another object of the present disclosure is to provide a method for treating LCA10 disease using a CRISPR/Cas12f1 (Cas14a1) system.

Still yet another object of the present disclosure is to provide a CRISPR/Cas12f1 (Cas14a1) system for artificially manipulating or modifying the CEP290 gene, and a method using the same.

### Technical Solution

The present invention provides a guide RNA for a CRISPR/Cas12f1 system, a DNA encoding the guide RNA, a guide RNA-Cas12f1 protein complex, a vector, a CRISPR/Cas12f1 composition, an *in vitro* or *ex vivo* method for editing CEP290 gene that comprises IVS26 mutation (c.2991 + 1655A>G), and a pharmaceutical composition for use in treating LCA10 disease, as defined in the appended claims.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis). Methods/uses for editing CEP290 gene referenced herein are in vitro or ex vivo.

The present disclosure provides a composition for genetic manipulation comprising a CRISPR/Cas12f1 system.

The CRISPR/Cas12f1 system may be a composition comprising:
a) at least one guide RNA or a nucleic acid encoding the same; and
b) a Cas12f1 protein or a nucleic acid encoding the same,

wherein the guide RNA has a scaffold region and a guide region,
the scaffold region is a region capable of interacting with the Cas12f1 protein and comprises a crRNA scaffold sequence and a tracrRNA scaffold sequence, with the scaffold region being located at the 5' end of the guide region, and
the guide region has a guide sequence that forms a complementary bond with a target sequence present in the CEP290 gene, and the guide sequence is at least one sequence selected from SEQ ID NOs: 51 to 100, wherein the complementary bond contains 0 to 5 mismatched bonds.

The crRNA scaffold sequence may be any one sequence selected from SEQ ID NOs: 101 to 109.

The tracrRNA scaffold sequence may be any one sequence selected from SEQ ID NOs: 110 to 121.

The scaffold region may further comprise a linker. Here, the scaffold region may be any one sequence selected from SEQ ID NOs: 122 to 126.

The guide sequence may be any one sequence selected from the group consisting of SEQ ID NOs: 60, 64, 66, 67, 79, 81, 92, 94, 96, and 100.

The composition may comprise two or more guide RNAs or nucleic acids encoding the same. Here, the two or more guide RNAs may comprise a first guide RNA and a second guide RNA. Here, the first guide RNA may comprise any one guide sequence selected from SEQ ID NOs: 51 to 74, and the second guide RNA may comprise any one guide sequence selected from SEQ ID NOs: 75 to 100. Here, the first guide RNA may comprise any one guide sequence selected from the group consisting of SEQ ID NOs: 60, 64, 66, and 67, and the second guide RNA may comprise any one guide sequence selected from the group consisting of SEQ ID NOs: 79, 81, 92, 94, 96, and 100.

The guide RNA may optionally further comprise a U-rich tail sequence at the 3' end. Here, the U-rich tail sequence may be 5'-(UₐN)_{d}Uₑ-3', 5'-UₐVUₐVUₑ-3', or 5'-UₐVUₐVUₐVUₑ-3'. Here, N may be A, C, G, or U. Each V may independently be A, C, or G. a may be an integer of 0 to 4. d may be an integer of 0 to 3. e may be an integer of 1 to 10.

The composition may comprise a nucleic acid encoding at least one guide RNA and a nucleic acid encoding the Cas12f1 protein in one vector. Here, the vector may further comprise a promoter for a nucleic acid encoding the one guide RNA and a promoter for a nucleic acid encoding the Cas12f1 protein. Here, in a case where the composition comprises at least two guide RNAs, the vector may comprise promoters for nucleic acids encoding the respective guide RNAs. Here, the promoter may be a U6 promoter, an H1 promoter, a 7SK promoter, a CMV promoter, an LTR promoter, an Ad MLP promoter, an HSV promoter, an SV40 promoter, a CBA promoter, or an RSV promoter. Here, the vector may be a plasmid, an mRNA (transcript), a PCR amplicon, or a viral vector. Here, the viral vector may be at least one viral vector selected from the group consisting of a retroviral (retrovirus) vector, a lentiviral (lentivirus) vector, an adenoviral (adenovirus vector), an adeno-associated viral vector (adeno-associated virus (AAV) vector), a vaccinia viral (vaccinia virus) vector, a poxviral (poxvirus) vector, and a herpes simplex viral (herpes simplex virus) vector.

The composition may be in a mixed form of a nucleic acid and a protein, that is, in a form of a CRISPR/Cas12f1 complex in which the guide RNA and the Cas12f1 protein are bound to each other.

### Advantageous Effects of Disclosure

The present disclosure relates to treatment of LCA10 disease using the CRISPR/Cas12f1 (Cas14a1) system. Through the techniques disclosed by the present specification, it is possible to provide a composition for treatment of LCA10 disease, comprising the CRISPR/Cas12f1 (Cas14a1) system, and a treatment method using the same. In addition, it is possible to provide a composition for artificially manipulating or modifying the CEP290 gene, the composition comprising the CRISPR/Cas12f1 (Cas14a1) system, and a manipulation or modification method using the same.

### Brief Description of Drawings

FIG. 1 illustrates a schematic diagram explaining a cryptic exon created by IVS26 mutation that is a mutation in the CEP290 gene which causes LCA10.
FIG. 2 illustrates a schematic diagram in which a target region for a CRISPR/Cas12f1 system is set and shown. The target region was set by classifying the upstream 1654bp portion as Forward region (F region) and the downstream 2000bp portion as Reverse region (R region) based on IVS26 mutation.
FIG. 3 illustrates results obtained by identifying deletion of mutant fragments caused by a CRISPR/Cas12f1 system using a combination of guide RNAs (two guide RNAs) that target the F region and the R region, respectively.
FIG. 4 illustrates results obtained by identifying deletion of fragments caused by a CRISPR/Cas 12f1 system using a combination of guide RNAs (a guide RNA having F14 as a guide sequence and a guide RNA having R22 as a guide sequence), which target the F region and the R region, respectively, in HEK-293T cells.
FIG. 5 illustrates results obtained by identifying deletion of fragments caused by a CRISPR/Cas 12f1 system using a combination of guide RNAs (a guide RNA having F14 as a guide sequence and a guide RNA having R22 as a guide sequence), which target the F region and the R region, respectively, in ARPE19-HPV cells.
FIG. 6 illustrates a schematic diagram of an experimental design for introducing a point mutation (c.2991+1655A>G) into intron 26 of the CEP290 locus using a CRISPR/Cas9 system and a single-stranded oligonucleotide (ssODN).
FIG. 7 illustrates results obtained by identifying, with PCR using the primer set in Table 20, whether a point mutation (c.2991 + 1655A>G) has been introduced in ARPE19/HPV16-LCA10 cells generated by transfecting ARPE19 cells with a CRISPR/Cas9 system expression vector and ssODN.

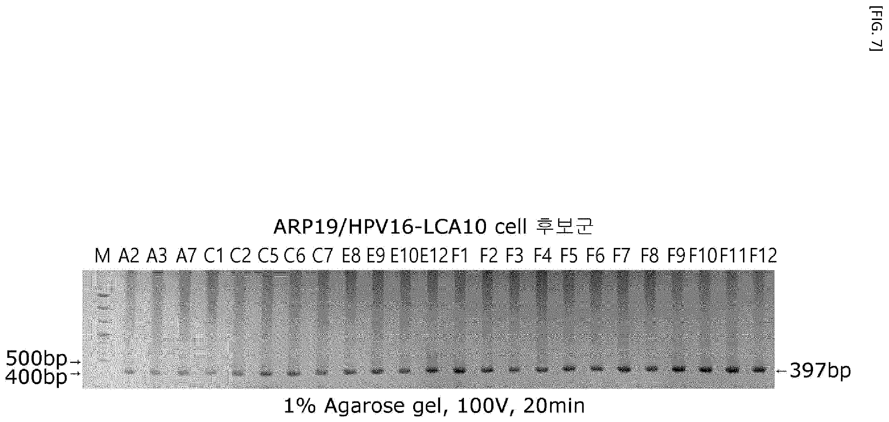

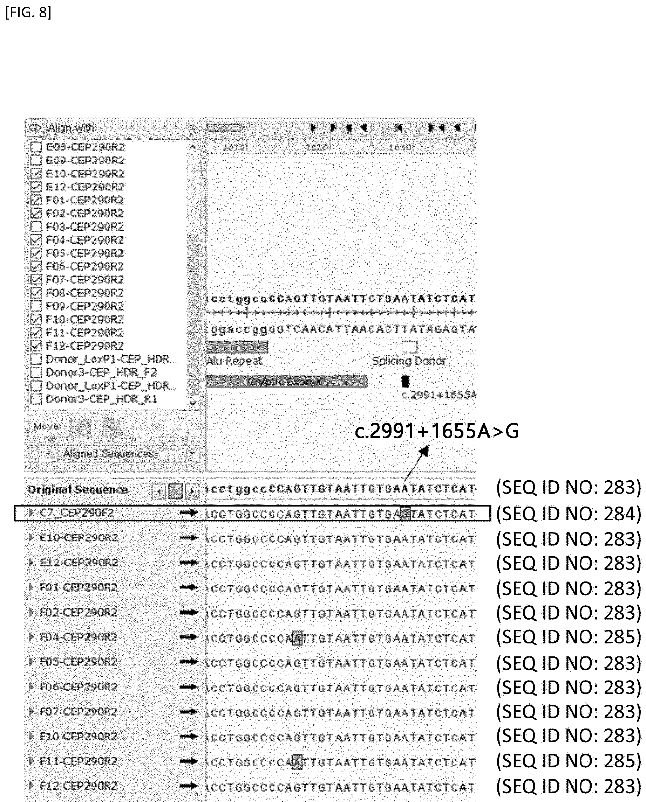

FIG. 8 illustrates results obtained by identifying, through sequencing analysis, the introduction of a point mutation (c.2991 + 1655A>G) in the ARPE19/HPV16-LCA10 cells identified in FIG. 7. Here, the wild-type sequence (original sequence) that does not contain the point mutation (c.2991+1655A>G) is 5'-CCTGGCCCCAGTTGTAATTGTGAATATCTCAT-3' (SEQ ID NO: 283), and the sequence, into which the point mutation (c.2991+1655A> G) has been introduced, is 5'-CCTGGCCCCAGTTGTAATTGTGAGTATCTCAT-3' (SEQ ID NO: 284). Another point mutation was identified in some of the results, and the sequence containing the other point mutation is 5'-CCTGGCCCCAATTGTAATTGTGAATATCTCAT-3' (SEQ ID NO: 285). Here, the underlined sequence in the above sequence is a point mutation.
FIG. 9 illustrates results obtained by extracting RNA from each of a clone of produced AC7 cells (hereinafter referred to as LCA10-AC7 cells) and cells of ARPE19/HPV16 (WT), and comparing wild-type (WT) mRNA expression levels of the CEP290 gene using qRT-PCR.
FIG. 10 illustrates results obtained by extracting RNA from each of a clone of produced LCA10-AC7 cells and cells of ARPE19/HPV16 (WT), and comparing expression levels of cryptic exon X, which has been generated by mutation (c.2991 + 1655A>G) of the CEP290 gene, using qRT-PCR.
FIG. 11 illustrates RT-PCR results, identifying that cryptic exon X has been generated by a point mutation (c.2991+1655A>G) in intron 26 of the CEP290 locus in LCA10-AC7 cells.

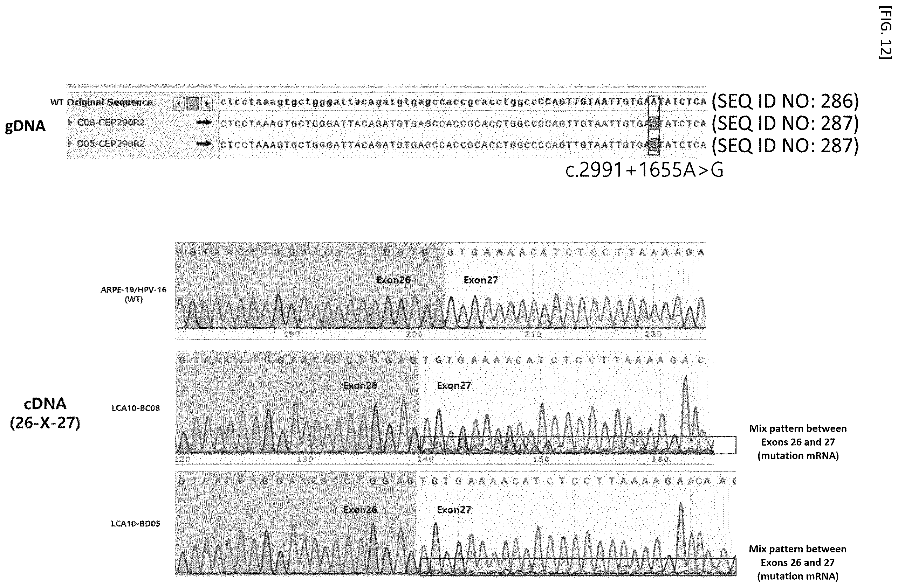

FIG. 12 illustrates sequencing results, which identify a point mutation (c.2991+1655A>G) in intron 26 of the CEP290 locus in genomic DNA extracted from LCA10-BC8 cells and LCA10-BD5 cells, and results obtained by extracting RNA from LCA10-BC8 cells and LCA10-BD5 cells and performing analysis. Here, the wild-type sequence (original sequence) that does not contain the point mutation (c.2991+1655A>G) is 5'-CTCCTAAAGTGCTGGGATTACAGATGTGAGCCACCGCACCTGGCCCCAGTT GTAATTGTGAATATCTCA-3' (SEQ ID NO: 286), and the sequence, into which the point mutation (c.2991+1655A> G) has been introduced, is 5'-CTCCTAAAGTGCTGGGATTACAGATGTGAGCCACCGCACCTGGCCCCAGTT GTAATTGTGAGTATCTCA-3' (SEQ ID NO: 287). Here, the underlined sequence in the above sequence is a point mutation. Here, the results obtained by extracting RNA and performing analysis show a pattern in which several sequences are mixed between exon 26 and exon 27, which indicates that a mutant mRNA is contained therein.
FIG. 13 illustrates results obtained by identifying, with RT-PCR, that cryptic exon X has been generated by a point mutation (c.2991+1655A>G) in intron 26 of the CEP290 locus in LCA10-AC7 cells, LCA10-BC8 cells, and LCA10-BD5 cells.
FIG. 14 illustrates results obtained by identifying an expression level of cryptic exon X in LCA10-BC8 cells and LCA10-BD5 cells.
FIG. 15 illustrates results obtained by identifying that expression of X-27 of the CEP290 gene is decreased by a guide RNA having F14 as a guide sequence and a guide RNA having R22 as a guide sequence, using produced LCA10-BC8 cells. Here, the comparator group is EDIT101.
FIG. 16 illustrates results obtained by identifying that expression of 26-X-27 of the CEP290 gene is decreased by a guide RNA having F14 as a guide sequence and a guide RNA having R22 as a guide sequence, using produced LCA10-BC8 cells. Here, the comparator group is EDIT101.
FIG. 17 schematically illustrates a method for producing 661W/LCA10 cell line.
FIG. 18 illustrates RT-PCR results, identifying expression of 26-X-27 (in which X means cryptic exon X) of 661W/LCA10 cell line, according to Example 6-2.
FIG. 19 illustrates RT-PCR results, identifying expression of X-27 (in which X means cryptic exon X) of 661W/LCA10 cell line, according to Example 6-2.
FIG. 20 illustrates a graph, showing an expression level of X-27 in each clone of 661W/LCA10 cell line, according to Example 6-2. Here, each label is named according to the following nomenclature: A to H, numbers of 1 to 12 (A to H: characters corresponding to rows of a 96-well plate; numbers of 1 to 12: numbers corresponding to columns in respective rows, for example, E08: clone at row E, column 8 in a 96-well plate).
FIG. 21 illustrates RT-PCR results, identifying deletion of a partial region of the CEP290 gene, caused by a LCA10 Cas12f1 therapeutic agent, in 661W/LCA10-E08 cell line, according to Example 7. Here, F14+R18 and F14+R22 refer to respective guide region combinations, and EDIT101 is a comparator group.
FIG. 22 illustrates q-PCR results, identifying deletion of a partial region of the CEP290 gene, according to Example 7. Here, F14+R18 and F14+R22 refer to respective guide region combinations, and EDIT101 is a control.
FIG. 23 illustrates results obtained by performing target screening of F-regions for a Cas12f1 variant, according to Example 8-1. Each label means a target sequence shown in [Table 1], and each row in the table below the graph represents an indel value obtained as a result of repeated experiments.
FIG. 24 illustrates results obtained by performing target screening of R-regions for a Cas12f1 variant, according to Example 8-1. Each label means a target sequence shown in [Table 2], and each row in the table below the graph represents an indel value obtained as a result of repeated experiments.
FIG. 25 illustrates indel efficiency for target sequences depending on guide RNA scaffolds for a Cas12f1 variant, according to Example 8-2. Each of F14, F18, and R22 refers to a target sequence shown in [Table 1] and [Table 2], ver4.0 refers to a guide RNA scaffold sequence of SEQ ID NO: 477, and ver4.1 refers to a guide RNA scaffold sequence of SEQ ID NO: 478.
FIG. 26 schematically illustrates a one-vector system that expresses a Cas12f1 variant and a guide RNA, according to Example 8-2. Specifically, this figure represents a one-vector system capable of expressing a Cas12f1 variant of SEQ ID NO: 288 and a guide RNA comprising a guide RNA scaffold sequence of SEQ ID NO: 477.
FIG. 27 schematically illustrates a one-vector system that expresses a Cas12f1 variant and a guide RNA, according to Example 8-2. Specifically, this figure represents a one-vector system capable of expressing a Cas12f1 variant of SEQ ID NO: 288 and a guide RNA comprising a guide RNA scaffold sequence of SEQ ID NO: 478.
FIG. 28 illustrates indel efficiency for target sequences depending on lengths of a guide sequence for a Cas12f1 variant, according to Example 8-3. F14-19mer to F14-25mer represent respective guide sequences shown in Tables 27 and 28, and ver4.0 means that a guide RNA scaffold sequence of SEQ ID NO: 277 was used. Each row in the table below the graph represents an indel value obtained as a result of repeated experiments.
FIG. 29 illustrates indel efficiency for target sequences depending on lengths of a guide sequence for a Cas12f1 variant, according to Example 8-3. R18-20mer to R18-25mer represent respective guide sequences shown in Tables 27 and 28, and ver4.0 means that a guide RNA scaffold sequence of SEQ ID NO: 277 was used. Each row in the table below the graph represents an indel value obtained as a result of repeated experiments.
FIG. 30 illustrates indel efficiency for target sequences depending on lengths of a guide sequence for a Cas12f1 variant, according to Example 8-3. R22-20mer to R22-25mer represent respective guide sequences shown in Tables 27 and 28, and ver4.0 means that a guide RNA scaffold sequence of SEQ ID NO: 277 was used. Each row in the table below the graph represents an indel value obtained as a result of repeated experiments.
FIG. 31 illustrates inhibitory effects on expression of X-27, caused by LCA10 Cas12f1 variant therapeutic agents, for ARPE19/LCA10-BC08 cell line, according to Example 9. Here, ARPE19/LCA10-BC08 (no treat) refers to a negative control that is not treated with the therapeutic agent; F14+R18 and F14+F22 mean that treatment was performed with the LCA10 Cas12f1 variant therapeutic agents in which respective target sequences shown in [Table 1] and [Table 2] are combined; and EDIT101 is a comparator group.
FIG. 32 illustrates inhibitory effects on expression of X-27, caused by LCA10 Cas12f1 variant therapeutic agents, for 661W/LCA10-E08 cell line, according to Example 9. Here, 661W/LCA10-E08 (no treat) refers to a negative control that is not treated with the therapeutic agent; F14+R18 and F14+F22 mean that treatment was performed with the LCA10 Cas12f1 variant therapeutic agents in which respective target sequences shown in [Table 1] and [Table 2] are combined; and EDIT101 is a comparator group.

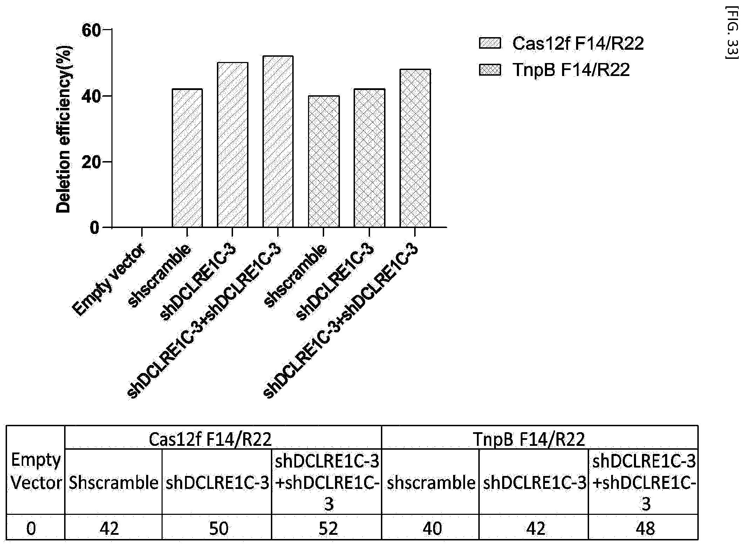

FIG. 33 illustrates effects of deleting the intron 26 region of CEP290 in a case where a LCA10 Cas12f1 therapeutic agent and a factor for decreasing activity of NHEJ are used in combination, according to Example 10. Here, Cas12f F14/R22 represents a LCA10 Cas12f1 therapeutic agent which comprises a Cas12f1 protein and guide RNAs targeting F14 and R22, respectively; and TnpB F14/R22 represents a LCA10 Cas12f1 variant therapeutic agent which comprises a Cas12f1 protein variant of SEQ ID NO: 288 and guide RNAs targeting F14 and R22, respectively. In addition, Empty vector is a negative control; shscramble is a positive control; shDCLRE1C-3 represents a result for a case where one nucleic acid encoding shRNA of SEQ ID NO: 473 is contained; and shDCLRE1C-3 + shDCLRE1C-3 represents a result for a case where two nucleic acids, each of which encodes shRNA of SEQ ID NO: 473, are contained.

### Mode Of Disclosure

### Definition of terms

Definitions of terms used in this specification are as follows.

### Nucleic acid, nucleotide, nucleoside, and base

"Nucleic acid" is a biomolecule (or biopolymer) composed of nucleotide units and is also called a polynucleotide. The nucleic acid comprises both DNA and RNA. "Nucleotide" is a unit composed of phosphoric acid, a pentose sugar, and a base (or nucleobase). In RNA (ribonucleic acid), the pentose sugar is ribose, and in DNA (deoxyribonucleic acid), the pentose sugar is deoxyribose. The nucleotide has one selected from adenine (A), guanine (G), cytosine (C), thymine (T) and uracil (U) as a nucleobase. Here, adenine, guanine, and cytosine exist both in RNA and DNA, thymine exists only in DNA, and uracil exists only in RNA. The nucleotide may also be said to be composed of phosphoric acid and a nucleoside. Here, "nucleoside" consists of a pentose sugar and a nucleobase. The nucleoside is classified into adenosine, thymidine, cytidine, guanosine, and uridine according to the type of nucleobase. Each nucleoside is abbreviated as U (uridine), A (adenosine), T (thymidine), C (cytidine) and G (guanosine). In addition, the nucleotide is abbreviated as U (uridine monophosphate), A (adenosine monophosphate), T (thymidine monophosphate), C (cytidine monophosphate) and G (guanosine monophosphate) according to the type of nucleoside. In addition, the terms include all meanings recognized by those skilled in the art, and may be appropriately interpreted according to the context.

In the present specification, bases, nucleosides, nucleotides, nucleic acids, RNA, and DNA are abbreviated as A, T, G, C, and U depending on the type of base. The above abbreviation may be appropriately interpreted depending on the context. For example, the sequence 5'-UUUUU-3' may be a sequence of five consecutive bases (uracil), a sequence of five consecutive nucleosides (uridine) and/or a sequence of five consecutive nucleotides (uridine monophosphate). In addition, in the case of a nucleic acid, RNA, and DNA, nucleotides constituting the nucleic acid, RNA, and DNA are abbreviated as uridine, adenosine, thymidine, cytidine and guanosine according to the type of nucleoside. The above abbreviation may be appropriately interpreted depending on the context. For example, RNA comprising a sequence of four consecutive uridines may be interpreted as RNA comprising four consecutive uridine monophosphate nucleotides.

### Target sequence, target strand, non-target strand

"Target sequence", which is a sequence present in a target nucleic acid or target gene, refers to a sequence recognized by a guide RNA of a CRISPR/Cas12f1 system (or CRISPR/Cas14a1 system) or a target sequence to be modified by a CRISPR/Cas12f1 system (or CRISPR/Cas14a1 system). Specifically, the target sequence refers to a sequence having complementarity to a guide sequence included in the guide RNA or a sequence complementarily binding to the guide sequence.

"Target strand" refers to a strand comprising a target sequence. When the target nucleic acid or target gene is single-stranded, the strand may be a target strand. Alternatively, when the target nucleic acid or target gene is double-stranded, one of the double-strand may be a target strand, and the other strand may be a strand complementary to the target strand. Here, the strand complementary to the target strand is referred to as a "non-target strand."

The non-target strand comprises a protospacer adjacent motif (PAM) sequence and a protospacer sequence. The PAM sequence is a sequence recognized by a Cas12f1 (or Cas14a1) protein of a CRISPR/Cas12f1 system (or CRISPR/Cas14a1 system). The protospacer sequence, which is located at the 5' end or the 3' end of the PAM sequence, is a sequence having complementarity to a target sequence or a sequence that forms a complementary bond with a target sequence. Correlation between the protospacer sequence and the target sequence is similar to correlation between the target sequence and the guide sequence. Due to these characteristics, in general, a protospacer sequence may be used to design a guide sequence. That is, when designing a guide sequence complementarily binding to a target sequence, the guide sequence may be designed as a nucleotide sequence having the same nucleotide sequence as the protospacer sequence. Here, the guide sequence is designed by replacing T with U in the nucleotide sequence of the protospacer sequence.

### Vector

"Vector", unless otherwise specified, refers collectively to any material capable of transporting a genetic material into a cell. For example, a vector may be a DNA molecule comprising a genetic material of interest, for example, a nucleic acid encoding an effector protein (Cas protein) of a CRISPR/Cas system, and/or a nucleic acid encoding a guide RNA; however, the vector is not limited thereto. The term includes all meanings recognized by those skilled in the art, and may be appropriately interpreted according to the context.

### Operably linked

The term "operably linked" means that a particular component is arranged with another component in a functional relationship, that is, a particular component is linked to another component so that the particular component can perform its intended function. For example, in a case where a promoter sequence is operably linked to a sequence encoding protein A, the promoter is linked to the sequence encoding the protein A so that the promoter transcribes and/or expresses the sequence encoding the protein A in a cell. In addition, the term includes all meanings recognized by those skilled in the art, and may be appropriately interpreted according to the context.

### Engineered

The term "engineered" is used to distinguish it from a material, a molecule, or the like whose configuration already exists in nature, and this means that the material, the molecule, or the like has undergone artificial modification. For example, the "engineered guide RNA" refers to a guide RNA obtained by applying artificial modification to the configuration of a naturally occurring guide RNA. In addition, the term includes all meanings recognized by those skilled in the art, and may be appropriately interpreted according to the context.

### Nuclear localization sequence or signal (NLS)

In a case where a substance outside the cell's nucleus is transported into the nucleus by nuclear transport, "nuclear localization sequence or signal (NLS)" refers to a peptide of a certain length or a sequence thereof, wherein the peptide is attached to a protein to be transported and acts as a type of "tag." As used herein, the term "NLS" includes all meanings recognized by those skilled in the art, and may be appropriately interpreted according to the context.

Unless defined otherwise, all technical and scientific terms used herein have the same meanings as commonly understood by those skilled in the art to which the present disclosure belongs. Although methods and materials similar or equivalent to those described herein may be used in practice or experimentation of the present disclosure, suitable methods and materials are described below.

Hereinafter, the present disclosure will be described.

### Overview - Treatment of LCA10 disease using CRISPR/Cas9

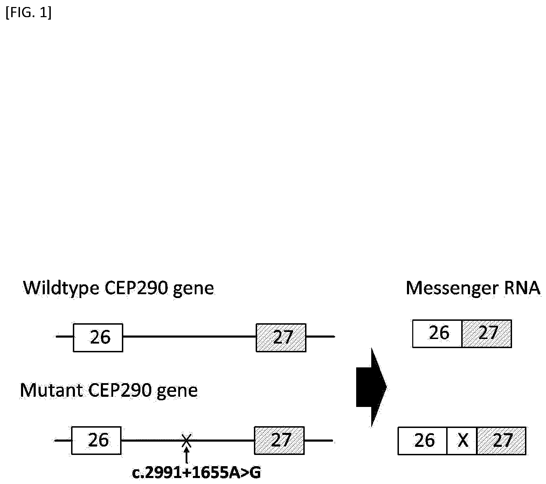

Leber congenital amaurosis type 10 (LCA10) is an autosomal recessive disease caused by a biallelic loss-of-function mutation in the CEP290 gene. Most patients are early infants, with clinically severe cone-rod dystrophy and decreased or lost vision. The protein encoded by the normal CEP290 gene is located in the photoreceptor-connecting cilium and is a necessary factor for phototransduction and outer segment regeneration. The most common mutation in the CEP290 gene, which causes LCA10, is IVS26 mutation. The IVS26 mutation is an adenine to guanine point mutation (c.2991 + 1655A>G) located within intron 26 between exon 26 and exon 27 of the CEP290 gene, and this mutation creates a new splice donor site so that a region for a 128 base pair cryptic exon is included in the messenger RNA (mRNA), thereby additionally generating cryptic exon X between exon 26 and exon 27 (FIG. 1). This aberrant splicing is more prominent in human photoreceptor cells than in other cell types. Currently, there is no approved treatment for LCA10. Although gene therapy is a potential therapeutic method, a large size (approximately 7.5 kb) of the normal CEP290 gene exceeds packaging capacity of adeno-associated virus (AAV). In order to overcome such limitation, treatment using a CRISPR/Cas9 system is receiving attention and is being developed. In particular, EDIT-101, a therapeutic agent using a CEP290-specific guide RNA and *Staphylococcus aureus* Cas9 (SaCas9), is being developed by Editas. EDIT-101 uses two guide RNAs with high specificity to the CEP290 locus which allow splicing defect in CEP290 to be removed through gene editing that deletes a mutated part. In this way, a pair of guide RNAs is used to induce inversion or elimination of IVS26 mutation, which leads to restoration of normal splicing and functional CEP290 expression (Morgan L. Maeder et al., Nature Medicine, 25, 229-233 (2019)).

Meanwhile, the present inventors have increased efficiency of the CRISPR/Cas12f1 system, which is a new CRISPR/Cas system, through previous studies, and named this system the CRISPR/Cas12f1-ge system. The CRISPR/Cas12f1 system is a new CRISPR/Cas system that was first reported in a previous study (Harrington et al., Science, 362, 839-842 (2018)), and it has been reported that despite the advantage of a significantly small size, the system has no or extremely low double-stranded DNA cleavage activity which limits its application to gene editing technology. In order to overcome such limitation, the present inventors researched, developed, and completed an engineered guide RNA that enhances double-stranded DNA (dsDNA) cleavage activity so that it can be used for gene editing (Korean Patent Application Nos. 10-2021-0051552, 10-2021-0050093, and 10-2021-0044152). The CRISPR/Cas12f1 system has a significantly smaller Cas protein size than the CRISPR/Cas9 system, which makes it possible to solve the difficulty of loading the CRISPR/Cas9 system on adeno-associated virus (AAV) due to the size of most previously studied Cas proteins and the difficulty of applying the CRISPR/Cas9 system as a gene therapy. In addition, the CRISPR/Cas12f1 system has a feature of inducing dsDNA cleavage outside a protospacer sequence. This feature means that even after the first trial of NHEJ-mediated indel mutation, the dsDNA cleavage-NHEJ process can be executed through additional trials until the protospacer sequence is significantly modified. These multiple cleavage and repair processes may provide more opportunities for reliable cleavage of a target sequence (and a protospacer sequence), and the CRISPR/Cas12f1 system with this feature may have excellent clinical utility in the field of gene therapy.

Based on previous approaches for treating LCA10 disease, the present inventors have introduced a new CRISPR/Cas12f1 system for treatment of LCA10 disease. Introduction of the CRISPR/Cas12f1 system has advantages over the existing CRISPR/Cas9 system, such as ease of loading it on adeno-associated virus (AAV) and reliable gene editing caused by multiple cleavage and repair processes. Accordingly, the present inventors have developed a therapeutic agent and a therapeutic method for LCA10 disease, using the CRISPR/Cas12f1 system having theses advantages.

Hereinafter, the therapeutic agent and the therapeutic method for LCA10 disease, using the CRISPR/Cas 12f1 system, will be described in detail.

### <CRISPR/Cas12f1 system for treatment of LCA10 disease>

In an aspect of the present disclosure, there is provided a CRISPR/Cas12f1 system for treatment of LCA10 disease. As described above, the LCA10 disease is a disease caused by IVS26 mutation of the CEP290 gene. In order to treat this LCA10 disease, a therapeutic strategy may be used which induces removal or inversion of all or part of the 128 base pair cryptic exon generated by the causative IVS26 mutation so that the normal CEP290 gene is expressed.

For this therapeutic strategy, in an aspect of the present disclosure, a CRISPR/Cas12f1 system is used. The CRISPR/Cas12f1 system disclosed herein includes both a wild-type CRISPR/Cas12f1 system and an artificially modified CRISPR/Cas12f1 system. Here, the artificially modified CRISPR/Cas12f1 system refers to a CRISPR/Cas12f1 system obtained by artificially modifying at least one of the elements, which constitute the CRISPR/Cas12f1 system, that is, a guide RNA and a Cas12f1 protein. For example, the artificially modified CRISPR/Cas12f1 system may comprise an artificially modified guide RNA and a Cas12f1 protein. The artificially modified CRISPR/Cas12f1 system includes CRISPR/Cas12f1-ge system. Thus, the CRISPR/Cas12f1 systems disclosed herein include both a wild-type CRISPR/Cas12f1 system and an artificially modified CRISPR/Cas12f1 system (for example, CRISPR/Cas12f1-ge system).

The CRISPR/Cas12f1 system is used to induce removal or inversion of all or part of the 128 base pair cryptic exon. The CRISPR/Cas12f1 system more effectively removes or inverts all or part of the causative cryptic exon through reliable gene editing caused by multiple cleavage and repair processes, thereby resulting in an increased therapeutic effect. In addition, the CRISPR/Cas12f1 system has a significantly smaller size than the existing CRISPR/Cas9 system, and thus can be more effectively used in application as a therapeutic agent, such as securing additional space (capacity) when inserted into a viral vector such as AAV.

More specifically, the CRISPR/Cas12f1 system for treating LCA10 disease comprises a guide RNA targeting the CEP290 gene and a Cas12f1 protein.

The guide RNA targets the intron 26 region in the CEP290 gene. Here, the intron 26 region contains IVS26 mutation, that is, a point mutation in which adenine located in intron 26 is altered to guanine (referred to as c.2991 + 1655A>G).

Hereinafter, each component and target of the CRISPR/Cas12f1 system for treatment of LCA10 disease are described in detail.

### 1. Target gene

LCA10 disease is known to be caused by a point mutation (c.2991 + 1655A>G) created in the intron 26 region of the CEP290 gene. This point mutation, that is, IVS26 mutation (c.2991 + 1655A>G) creates a new splice donor site in the intron 26 region, and the resulting aberrant splicing leads to formation of a 128 base pair cryptic exon within an mRNA during transcription of the CEP290 gene, thereby resulting in expression of abnormal CEP290 protein or inhibited expression of normal CEP290 protein. Therefore, for treatment of LCA10 disease, the CEP290 gene was selected as a target, that is, a target gene, of the CRISPR/Cas12f1 system. In particular, the CEP290 gene contains IVS26 mutation (c.2991 + 1655A>G). As used herein, the "CEP290 gene" refers to CEP290 gene containing IVS26 mutation (c.2991 + 1655A>G). Here, the CEP290 gene containing IVS26 mutation (c.2991 + 1655A>G) is also referred to as "abnormal CEP290 gene", "CEP290 gene variant," or "CEP290 gene (IVS26)", and the terms are used interchangeably herein. In addition, the CEP290 gene, which does not contain IVS26 mutation (c.2991 + 1655A>G), or the CEP gene, which normally expresses CEP290 protein, is referred to as "wild-type CEP290 gene," "normal CEP290 gene," or "functional CEP290 gene," and the terms are used interchangeably herein.

### 1-1) Target region

For treatment of LCA10 disease, the CRISPR/Cas12f1 system targets the CEP290 gene. More specifically, the CRISPR/Cas12f1 system targets a region in the CEP290 gene. The region in the CEP290 gene is a target region for the CRISPR/Cas12f1 system, and the target region comprises a target sequence that binds complementarily to a guide RNA constituting the CRISPR/Cas12f1 system.

The region, that is, the target region, in the CEP290 gene is the intron 26 region in the CEP290 gene. Here, the intron 26 region contains IVS26 mutation (c.2991 + 1655A>G). Here, the intron 26 region may be divided into two regions based on the IVS26 mutation (c.2991 + 1655A>G). That is, the intron 26 region may be divided into an upstream region and a downstream region based on the IVS26 mutation (c.2991 + 1655A>G). Here, the upstream region may be a region between the 3' end of exon 26 of the CEP290 gene and the IVS26 mutation (c.2991 + 1655A>G). Here, the downstream region may be a region between the IVS26 mutation (c.2991 + 1655A>G) and the 5' end of exon 27 of the CEP290 gene.

In an embodiment, the target region may be a partial region of the intron 26 region containing the IVS26 mutation (c.2991 + 1655A>G).

In another embodiment, the target region may be an upstream region based on the IVS26 mutation (c.2991 + 1655A>G) in the intron 26 region. Alternatively, the target region may be a region between the 3' end of exon 26 of the CEP290 gene and the IVS26 mutation (c.2991 + 1655A>G).

In yet another embodiment, the target region may be a downstream region based on the IVS26 mutation (c.2991 + 1655A>G) in the intron 26 region. Alternatively, the target region may be a region between the IVS26 mutation (c.2991 + 1655A>G) and the 5' end of exon 27 of the CEP290 gene.

The target region is double-stranded DNA and is composed of a target strand and a non-target strand. Here, the target strand comprises a target sequence and is a strand to which the guide RNA constituting the CRISPR/Cas12f1 system binds. The non-target strand is a strand complementary to the target strand and comprises a protospacer adjacent motif (PAM) sequence and a protospacer sequence. Here, the protospacer sequence is a sequence having complementarity to the target sequence or a sequence that forms a complementary bond with the target sequence. The target sequence, the target strand, the non-target strand, and the protospacer sequence, as described above, are as previously described in the section "Definition of terms."

### i) Target sequence

The target sequence is a partial sequence present in the target region and is a sequence that binds complementarily to the guide RNA constituting the CRISPR/Cas12f1 system. In addition, the target sequence is as previously described in the section "Definition of terms,", and has the same characteristics and relationships as the target sequence, the target strand, the non-target strand, and the protospacer sequence as described in the above section.

The target sequence may be a sequence of 15 to 40 nucleotides. For example, the target sequence may be a sequence of 15 to 20, 15 to 25, 15 to 30, 15 to 35, or 15 to 40 nucleotides. Alternatively, the target sequence may be a sequence of 20 to 25, 20 to 30, 20 to 35, or 20 to 40 nucleotides. Alternatively, the target sequence may be a sequence of 25 to 30, 25 to 35, or 25 to 40 nucleotides. Alternatively, the target sequence may be a sequence of 30 to 35 or 30 to 40 nucleotides. Alternatively, the target sequence may be a sequence of 35 to 40 nucleotides. In another example, the target sequence may be a sequence of 15, 16, 17, 18, 19, 20, 21, 22, 23, 24, 25, 26, 27, 28, 29, 30, 31, 32, 33, 34, 35, 36, 37, 38, 39, or 40 nucleotides.

In an embodiment, the target sequence may be a sequence of 15 to 40 nucleotides present in a partial region of the intron 26 region containing the IVS26 mutation (c.2991 + 1655A>G).

In another embodiment, the target sequence may be a sequence of 15 to 40 nucleotides present in an upstream region based on the IVS26 mutation (c.2991 + 1655A>G) in the intron 26 region. Alternatively, the target sequence may be a sequence of 15 to 40 nucleotides present in a region between the 3' end of exon 26 of the CEP290 gene and the IVS26 mutation (c.2991 + 1655A>G). In an embodiment, the target sequences are summarized in Table 1. In Table 1, based on the c.2991+1655A>G gene mutation, the upstream 1654bp portion was classified as a forward region (F region), and the target sequences are summarized based on protospacer sequences present in the F region. In order to easily distinguish the respective sequences, the sequences present in the F region were classified as F and numbered, and are summarized in such a manner in Table 1.

**[Table 1] Target sequences**

| Name | Target sequence | SEQ ID NO |
|---|---|---|
| Target-F01 | 5'-TAAATAGGCATAATTTTCTA-3' | 1 |
| Target-F02 | 5'-TTGCTTTCTGCTGCTTTTGC-3' | 2 |
| Target-F03 | 5'-GTAGCTTTCAGGATTCCTAC-3' | 3 |
| Target-F04 | 5'-GGAGCTTGTTCTGTCCTCAG-3' | 4 |
| Target-F05 | 5'-GTTTAACGTTATCATTTTCC-3' | 5 |
| Target-F06 | 5'-GCTCATAGAGACACATTCAG-3' | 6 |
| Target-F07 | 5'-TTTCTGATGAGGAAGATGAA-3' | 7 |
| Target-F08 | 5'-GATCTTAGATAAGAATAATC-3' | 8 |
| Target-F09 | 5'-TTACTTCTAAATAATATTGA-3' | 9 |
| Target-F10 | 5'-TGGACCATGGATGCACTCTG-3' | 10 |
| Target-F11 | 5'-GCTACATCCATTCCAAGGAA-3' | 11 |
| Target-F12 | 5'-TTTTCTCTTAGATGTCTGGT-3' | 12 |
| Target-F13 | 5'-TGTAGAATTTTAATGTAGAA-3' | 13 |
| Target-F14 | 5'-TCTTACCCCTGTACCCAGAA-3' | 14 |
| Target-F15 | 5'-CTTGCATGATTTAGCTGAAT-3' | 15 |
| Target-F16 | 5'-CAGAAGTCATTCAGCCACTA-3' | 16 |
| Target-F17 | 5'-GTGTGTGTGTTATGTGGGAA-3' | 17 |
| Target-F18 | 5'-TTATTCATTCAGTTTAGTTA-3' | 18 |
| Target-F19 | 5'-GGGATTTGGCAGATTTTAAT-3' | 19 |
| Target-F20 | 5'-CTGCCAAATCCCCCCAAACA-3' | 20 |
| Target-F21 | 5'-CACAAGGTTCAAGAATCACA-3' | 21 |
| Target-F22 | 5'-CATCATTTTTTATTGTAGAA-3' | 22 |
| Target-F23 | 5'-GAATGTGTCTCTATGAGCCAG-3' | 23 |
| Target-F24 | 5'-TAAGATCTAATTCTATTAGCT-3' | 24 |

In another embodiment, the target sequence may be a sequence of 15 to 40 nucleotides present in a downstream region based on the IVS26 mutation (c.2991 + 1655A>G) in the intron 26 region. Alternatively, the target sequence may be a sequence of 15 to 40 nucleotides present in a region between the IVS26 mutation (c.2991 + 1655A>G) and the 5' end of exon 27 of the CEP290 gene. In an embodiment, the target sequences are summarized in Table 2. In Table 2, based on the c.2991+1655A>G gene mutation, the downstream 2000bp portion was classified as a reverse region (R region), and the target sequences are summarized based on protospacer sequences present in the R region. In order to easily distinguish the respective sequences, the sequences present in the R region were classified as R and numbered, and are summarized in such a manner in Table 2.

**[Table 2] Target sequences**

| Name | Target sequence (20nt) | SEQ ID NO |
|---|---|---|
| Target-R01 | 5'-TCTCATGAACCTTTACCTCT-3' | 25 |
| Target-R02 | 5'-TATTAGCTTGAACTCTGTGC-3' | 26 |
| Target-R03 | 5'-CATTAAGGAAGACAGATATG-3' | 27 |
| Target-R04 | 5'-CAGGAGTGACTTTGTTCCAT-3' | 28 |
| Target-R05 | 5'-GCTACCGGTTACCTGAAGGG-3' | 29 |
| Target-R06 | 5'-TGCCACAAGAATGATCATTC-3' | 30 |
| Target-R07 | 5'-TCCCATGTGACTCCCCGCCT-3' | 31 |
| Target-R08 | 5'-TATTGTTGCTTTTTGAGAGG-3' | 32 |
| Target-R09 | 5'-GTGAATTTCTATTCCTGTTT-3' | 33 |
| Target-R10 | 5'-GCATACTTTTTTTAATGGAA-3' | 34 |
| Target-R11 | 5'-CTCAACACACAGAAACAAAT-3' | 35 |
| Target-R12 | 5'-GAATAGATAATAAGGAAATA-3' | 36 |
| Target-R13 | 5'-TTTCTTTTTTCTTCTACTAT-3' | 37 |
| Target-R14 | 5'-CCTGTGTTTCAAGGGGATCT-3' | 38 |
| Target-R15 | 5'-CCCTTGAAACACAGGAATTG-3' | 39 |
| Target-R16 | 5'-TAAGCAATGGATTTCAGTGA-3' | 40 |
| Target-R17 | 5'-CAATGGATTTCAGTGATAAA-3' | 41 |
| Target-R18 | 5'-TATTTTCCAGGTACTCCACT-3' | 42 |
| Target-R19 | 5'-TACAAACAGGGTAATGAGAC-3' | 43 |
| Target-R20 | 5'-TCCTGTGTATTCATAGTATA-3' | 44 |
| Target-R21 | 5'-GATAATCCTATTGTTTGTTA-3' | 45 |
| Target-R22 | 5'-CACTCTCTCCCTAGTAACTC-3' | 46 |
| Target-R23 | 5'-GAAACTTCACTGGTCTCCTT-3' | 47 |
| Target-R24 | 5'-CTGGCGGATTACCTGAGATC-3' | 48 |
| Target-R25 | 5'-CTATAATTATGTACTTTACT-3' | 49 |
| Target-R26 | 5'-CATGTTGGCCGGGCTGGTCT-3' | 50 |

### 2. Guide RNA

The CRISPR/Cas12f1 system for treatment of LCA10 disease comprises a guide RNA. The guide RNA comprises a trans-activating CRISPR RNA (tracrRNA) and a CRISPR RNA (crRNA). Here, the crRNA comprises a guide sequence capable of complementarily binding to the target sequence. The crRNA comprises a scaffold sequence that binds to and/or interacts with the tracrRNA and/or the Cas12f1 protein. Here, the tracrRNA has a scaffold sequence that binds to and/or interacts with the crRNA and/or the Cas12f1 protein. Thus, the guide RNA comprises a guide region and a scaffold region. Here, the guide region has a guide sequence, and the scaffold region comprises a scaffold sequence of the crRNA (hereinafter referred to as crRNA scaffold sequence) and a scaffold sequence of the tracrRNA (hereinafter referred to as tracrRNA scaffold sequence).

The guide RNA may optionally further comprise a U-rich tail sequence at the 3' end. Here, the U-rich tail sequence is a sequence rich in uridine (U), and the U-rich tail sequence is known to play a role in increasing editing efficiency of a target gene (target nucleic acid) which is achieved with an engineered guide RNA (or engineered CRISPR/Cas12f1 (Cas14a1) system) (PCT/KR2020/014961).

Hereinafter, the respective components of the guide RNA are described in detail.

### 2-1) Guide region

The guide RNA comprises a guide region. The guide region serves to allow the guide RNA to specifically bind to a target gene. Here, the guide region has a guide sequence. The guide sequence is an RNA sequence that recognizes, binds to, or targets a target gene. More specifically, the guide sequence is an RNA sequence that binds complementarily to a target sequence, an RNA sequence capable of forming a complementary bond with a target sequence, or an RNA sequence having complementarity to a target sequence. Alternatively, the guide sequence is an RNA sequence identical to, similar to or corresponding to a protospacer sequence. Here, the protospacer sequence has a systematic relationship with a target sequence, and the description therefor is as described in "Target sequence, target strand, non-target strand" in the definition of terms section. The guide sequence is a sequence that changes depending on a target sequence, and the guide sequence varies depending on the target sequence. In addition, the guide sequence is an RNA sequence, and comprises uridine (U) capable of forming a complementary bond with adenosine (A) present in a target sequence of a target gene. Alternatively, for thymidine (T) present in a protospacer sequence of a target gene, the guide sequence comprises uridine (U) instead of thymidine (T). In addition, the guide sequence is also referred to as a spacer sequence; hereinafter, the guide sequence and the spacer sequence are used interchangeably.

The guide sequence is located at the 3' end of the crRNA. Alternatively, the guide sequence is located at the 3' end of the crRNA scaffold sequence.

The guide sequence is covalently linked to the 3' end of the crRNA scaffold sequence.

The guide sequence may be a sequence of 15 to 40 nucleotides.

In an embodiment, the guide sequence may be a sequence of 15 to 20, 15 to 25, 15 to 30, 15 to 35, or 15 to 40 nucleotides. Alternatively, the guide sequence may be a sequence of 20 to 25, 20 to 30, 20 to 35, or 20 to 40 nucleotides. Alternatively, the guide sequence may be a sequence of 25 to 30, 25 to 35, or 25 to 40 nucleotides. Alternatively, the guide sequence may be a sequence of 30 to 35 or 30 to 40 nucleotides. Alternatively, the guide sequence may be a sequence of 35 to 40 nucleotides.

In another embodiment, the guide sequence may be a sequence of 15, 16, 17, 18, 19, 20, 21, 22, 23, 24, 25, 26, 27, 28, 29, 30, 31, 32, 33, 34, 35, 36, 37, 38, 39, or 40 nucleotides.

The guide sequence may be a sequence that forms a complementary bond with a target sequence. Here, the complementary bond may optionally comprise at least one mismatched bond. For example, the guide sequence is a sequence that forms a complementary bond with a target sequence, wherein the complementary bond may comprise 0 to 5 mismatched bonds, or the complementary bond may comprise 0 to 5 mismatches.

The guide sequence may be a sequence complementary to a target sequence. Here, the complementary sequence may comprise a sequence of 0 to 5 mismatched nucleotides with respect to the target sequence. Alternatively, the guide sequence may be a nucleotide sequence that is at least 70% complementary to a target sequence. Here, in a case where the target sequence is DNA, for adenosine (A) present in the target sequence, the guide sequence may comprise uridine (U) capable of forming a complementary bond with the adenosine (A).

In an embodiment, the guide sequence may be a sequence that is at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95%, or at least 75% to 100% to the target sequence. Alternatively, the guide sequence may be a sequence that is at least 80% to 85%, at least 80% to 90%, at least 80% to 95%, at least 80% to 100%, at least 85% to 90%, at least 85% to 95%, or at least 85% to 100% complementary to the target sequence. Alternatively, the guide sequence may be a sequence that is at least 90% to 95%, at least 90% to 100%, or at least 95% to 100% complementary to the target sequence. Alternatively, the guide sequence may be a sequence that is at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% complementary to the target sequence.

The guide sequence may be a sequence that is identical or similar to a protospacer sequence. Alternatively, the guide sequence may be a sequence having sequence identity or similarity to the protospacer sequence. Here, the sequence identity or similarity may be at least 70%. Here, for thymidine (T) present in the protospacer sequence, the guide sequence may comprise uridine (U) instead of thymidine (T).

In an embodiment, the guide sequence may be a sequence that is at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95%, or at least 75% to 100% identical or similar to the protospacer sequence. Alternatively, the guide sequence may be a sequence that is at least 80% to 85%, at least 80% to 90%, at least 80% to 95%, at least 80% to 100%, at least 85% to 90%, at least 85% to 95%, or at least 85% to 100% identical or similar to the protospacer sequence. Alternatively, the guide sequence may be a sequence that is at least 90% to 95%, at least 90% to 100%, or at least 95% to 100% identical or similar to the protospacer sequence. Alternatively, the guide sequence may be a sequence that is at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% identical or similar to the protospacer sequence.

In another embodiment, the guide sequence may be a sequence having sequence identity or similarity of at least 70% to 75%, at least 70% to 80%, at least 70% to 85%, at least 70% to 90%, at least 70% to 95%, 70% to 100%, at least 75% to 80%, at least 75% to 85%, at least 75% to 90%, at least 75% to 95%, or at least 75% to 100% to the protospacer sequence. Alternatively, the guide sequence may be a sequence having sequence identity or similarity of at least 80% to 85%, at least 80% to 90%, at least 80% to 95%, at least 80% to 100%, at least 85% to 90%, at least 85% to 95%, or at least 85% to 100% to the protospacer sequence. Alternatively, the guide sequence may be a sequence having sequence identity or similarity of at least 90% to 95%, at least 90% to 100%, or at least 95% to 100% to the protospacer sequence. Alternatively, the guide sequence may be a sequence having sequence identity or similarity of at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% to the protospacer sequence.

In an embodiment, the guide sequence may be a sequence of 15 to 40 nucleotides that forms a complementary bond with a target sequence present in a partial region of the intron 26 region containing the IVS26 mutation (c.2991 + 1655A>G) of the CEP290 gene.

In an embodiment, the guide sequence may be a sequence of 15 to 40 nucleotides that forms a complementary bond with the target sequence present in an upstream region based on the IVS26 mutation (c.2991 + 1655A>G) in the intron 26 region of the CEP290 gene. Alternatively, the guide sequence may be a sequence of 15 to 40 nucleotides that forms a complementary bond with the target sequence present in a region between the 3' end of exon 26 of the CEP290 gene and the IVS26 mutation (c.2991 + 1655A>G). In an embodiment, the guide sequence may be a sequence that forms a complementary bond with any one sequence selected from the target sequences listed in Table 1. Here, the guide sequences are summarized in Table 3. In Table 3, the guide sequences are summarized which bind complementarily to target sequences present in a forward region (F region) that is an upstream 1654bp portion based on the c.2991+1655A>G gene mutation. In order to easily distinguish the respective sequences, the guide sequences targeting the F region were classified as F and numbered, and are summarized in such a manner in Table 3.

**[Table 3] Guide sequences**

| Name | Guide sequence (20nt) (5' to 3') | SEQ ID NO |
|---|---|---|
| Guide-F01 | UAGAAAAUUAUGCCUAUUUA | 51 |
| Guide-F02 | GCAAAAGCAGCAGAAAGCAA | 52 |
| Guide-F03 | GUAGGAAUCCUGAAAGCUAC | 53 |
| Guide-F04 | CUGAGGACAGAACAAGCUCC | 54 |
| Guide-F05 | GGAAAAUGAUAACGUUAAAC | 55 |
| Guide-F06 | CUGAAUGUGUCUCUAUGAGC | 56 |
| Guide-F07 | UUCAUCUUCCUCAUCAGAAA | 57 |
| Guide-F08 | GAUUAUUCUUAUCUAAGAUC | 58 |
| Guide-F09 | UCAAUAUUAUUUAGAAGUAA | 59 |
| Guide-F10 | CAGAGUGCAUCCAUGGUCCA | 60 |
| Guide-F11 | UUCCUUGGAAUGGAUGUAGC | 61 |
| Guide-F12 | ACCAGACAUCUAAGAGAAAA | 62 |
| Guide-F13 | UUCUACAUUAAAAUUCUACA | 63 |
| Guide-F14 | UUCUGGGUACAGGGGUAAGA | 64 |
| Guide-F15 | AUUCAGCUAAAUCAUGCAAG | 65 |
| Guide-F16 | UAGUGGCUGAAUGACUUCUG | 66 |
| Guide-F17 | UUCCCACAUAACACACACAC | 67 |
| Guide-F18 | UAACUAAACUGAAUGAAUAA | 68 |
| Guide-F19 | AUUAAAAUCUGCCAAAUCCC | 69 |
| Guide-F20 | UGUUUGGGGGGAUUUGGCAG | 70 |
| Guide-F21 | UGUGAUUCUUGAACCUUGUG | 71 |
| Guide-F22 | UUCUACAAUAAAAAAUGAUG | 72 |
| Guide-F23 | CUGGCUCAUAGAGACACAUUC | 73 |
| Guide-F24 | AGCUAAUAGAAUUAGAUCUUA | 74 |

In another embodiment, the guide sequence may be a sequence of 15 to 40 nucleotides that forms a complementary bond with the target sequence present in a downstream region based on the IVS26 mutation (c.2991 + 1655A>G) in the intron 26 region of the CEP290 gene. Alternatively, the guide sequence may be a sequence of 15 to 40 nucleotides that forms a complementary bond with the target sequence present in a region between the IVS26 mutation (c.2991 + 1655A>G) and the 5' end of exon 27 of the CEP290 gene. In an embodiment, the guide sequence may be a sequence that forms a complementary bond with any one sequence selected from the target sequences listed in Table 2. Here, the guide sequences are summarized in Table 4. In Table 4, the guide sequences are summarized which bind complementarily to target sequences present in a reverse region (R region) that is a downstream 2000 bp portion based on the c.2991+1655A>G gene mutation. In order to easily distinguish the respective sequences, the guide sequences targeting the R region were classified as R and numbered, and are summarized in such a manner in Table 4.

**[Table 4] Guide sequences**

| Name | Guide sequence (20nt) (5' to 3') | SEQ ID NO |
|---|---|---|
| Guide-R01 | AGAGGUAAAGGUUCAUGAGA | 75 |
| Guide-R02 | GCACAGAGUUCAAGCUAAUA | 76 |
| Guide-R03 | CAUAUCUGUCUUCCUUAAUG | 77 |
| Guide-R04 | AUGGAACAAAGUCACUCCUG | 78 |
| Guide-R05 | CCCUUCAGGUAACCGGUAGC | 79 |
| Guide-R06 | GAAUGAUCAUUCUUGUGGCA | 80 |
| Guide-R07 | AGGCGGGGAGUCACAUGGGA | 81 |
| Guide-R08 | CCUCUCAAAAAGCAACAAUA | 82 |
| Guide-R09 | AAACAGGAAUAGAAAUUCAC | 83 |
| Guide-R10 | UUCCAUUAAAAAAAGUAUGC | 84 |
| Guide-R11 | AUUUGUUUCUGUGUGUUGAG | 85 |
| Guide-R12 | UAUUUCCUUAUUAUCUAUUC | 86 |
| Guide-R13 | AUAGUAGAAGAAAAAAGAAA | 87 |
| Guide-R14 | AGAUCCCCUUGAAACACAGG | 88 |
| Guide-R15 | CAAUUCCUGUGUUUCAAGGG | 89 |
| Guide-R16 | UCACUGAAAUCCAUUGCUUA | 90 |
| Guide-R17 | UUUAUCACUGAAAUCCAUUG | 91 |
| Guide-R18 | AGUGGAGUACCUGGAAAAUA | 92 |
| Guide-R19 | GUCUCAUUACCCUGUUUGUA | 93 |
| Guide-R20 | UAUACUAUGAAUACACAGGA | 94 |
| Guide-R21 | UAACAAACAAUAGGAUUAUC | 95 |
| Guide-R22 | GAGUUACUAGGGAGAGAGUG | 96 |
| Guide-R23 | AAGGAGACCAGUGAAGUUUC | 97 |
| Guide-R24 | GAUCUCAGGUAAUCCGCCAG | 98 |
| Guide-R25 | AGUAAAGUACAUAAUUAUAG | 99 |
| Guide-R26 | AGACCAGCCCGGCCAACAUG | 100 |

### 2-2) Scaffold region

The guide RNA comprises a scaffold region. The scaffold region serves to interact with a Cas12f1 protein and form a CRISPR/Cas12f1 complex. Here, the scaffold region comprises a crRNA scaffold sequence and a tracrRNA scaffold sequence. The scaffold region is located at the 5' end of the guide region.

The scaffold region may have a dual scaffold sequence or a single scaffold sequence. Here, the dual scaffold sequence is composed of two molecules and may comprise a crRNA scaffold sequence and a tracrRNA scaffold sequence as separate molecules. Here, the guide RNA is a dual guide RNA and may be composed of two molecules. That is, in the dual guide RNA, crRNA and tracrRNA may each independently exist. Here, the single scaffold sequence is composed of one molecule and may comprise a crRNA scaffold sequence, a linker, and a tracrRNA scaffold sequence. The single scaffold sequence may be a single RNA molecule in which a crRNA scaffold sequence, a linker, and a tracrRNA scaffold sequence are linked to each other, and may be a sequence of 5'-[tracrRNA scaffold sequence]-[linker]-[crRNA scaffold sequence]-3'. Here, the guide RNA is a single guide RNA, and may be composed of one RNA molecule and have a sequence of 5'-[tracrRNA]-[linker]-[crRNA]-3' in which the tracrRNA, the linker, and the crRNA are linked to each other.

Hereinafter, the crRNA scaffold sequence, the tracrRNA scaffold sequence, and the linker are described in detail.

### i) crRNA scaffold sequence

The scaffold region comprises a crRNA scaffold sequence. The crRNA scaffold sequence is a partial sequence present in the crRNA that binds to and/or interacts with the tracrRNA and/or the Cas12f1 protein.

The crRNA scaffold sequence may be a wild-type crRNA scaffold sequence or an engineered crRNA scaffold sequence. Here, the engineered crRNA scaffold sequence may be an engineered form of the wild-type crRNA scaffold sequence in which artificial modification (substitution, deletion, or insertion) is made at a partial nucleotide sequence in the wild-type crRNA scaffold sequence, or in which modification is made to have a shorter length than the wild-type crRNA scaffold sequence.

The crRNA scaffold sequence may be located at the 5' end of the guide sequence. Alternatively, the crRNA scaffold sequence may be located at the 5' end of the crRNA.

The crRNA scaffold sequence may covalently bind to the 5' end of the guide sequence.

In an embodiment, the crRNA scaffold sequence may be a wild-type crRNA scaffold sequence. Here, the wild-type crRNA scaffold sequence may be 5'-GUUGCAGAACCCGAAUAGACGAAUGAAGGAAUGCAAC-3' (SEQ ID NO: 101).

In another embodiment, the crRNA scaffold sequence may be an engineered crRNA scaffold sequence. Here, the engineered crRNA scaffold sequence may be an engineered form of SEQ ID NO: 101 in which a partial nucleotide sequence in SEQ ID NO: 101 is subjected to artificial substitution, deletion, or insertion. Alternatively, the engineered crRNA scaffold sequence may be an engineered form of SEQ ID NO: 101 in which modification is made to have a shorter length than SEQ ID NO: 101.

In an embodiment, the engineered crRNA scaffold sequence is a partial sequence of SEQ ID NO: 101, and this sequence may be a sequence of SEQ ID NO: 101 that does not contain a partial sequence of the 5' end or 3' end of SEQ ID NO: 101. For example, the engineered crRNA scaffold sequence may be 5'-UGCAGAACCCGAAUAGACGAAUGAAGGAAUGCAAC-3' (SEQ ID NO: 102), 5'-CAGAACCCGAAUAGACGAAUGAAGGAAUGCAAC-3' (SEQ ID NO: 103), 5'-GAAUAGACGAAUGAAGGAAUGCAAC-3' (SEQ ID NO: 104), 5'-ACGAAUGAAGGAAUGCAAC-3' (SEQ ID NO: 105), or 5'-GGAAUGCAAC-3' (SEQ ID NO: 106). These examples are provided for illustrative purposes only and are not intended to limit the disclosure.

In another embodiment, the engineered crRNA scaffold sequence is an engineered form of SEQ ID NO: 101 in which an artificial modification is made at a partial nucleotide sequence in SEQ ID NO: 101, and this sequence may be an engineered form of SEQ ID NO: 101 in which substitution, deletion, and/or addition are made at one or more nucleotides in SEQ ID NO: 101. For example, the engineered crRNA scaffold sequence may be 5'-GUUGCAGAACCCGAAUAGAGCAAUGAAGGAAUGCAAC-3' (SEQ ID NO: 107), 5'-GAAUAGAGCAAUGAAGGAAUGCAAC-3' (SEQ ID NO: 108), or 5'-AGCAAUGAAGGAAUGCAAC-3' (SEQ ID NO: 109). These examples are provided for illustrative purposes only and are not intended to limit the disclosure.

### ii) tracrRNA scaffold sequence

The scaffold region comprises a tracrRNA scaffold sequence. The tracrRNA scaffold sequence is all or part of the tracrRNA that binds to and/or interacts with the crRNA and/or the Cas12f1 protein.

The tracrRNA scaffold sequence may be a wild-type tracrRNA scaffold sequence or an engineered tracrRNA scaffold sequence. Here, the engineered tracrRNA scaffold sequence may be an engineered form of the wild-type tracrRNA scaffold sequence in which artificial modification (substitution, deletion, or insertion) is made at a partial nucleotide sequence in the wild-type tracrRNA scaffold sequence, or in which modification is made to have a shorter length than the wild-type tracrRNA scaffold sequence.

In an embodiment, the tracrRNA scaffold sequence may be a wild-type tracrRNA scaffold sequence. Here, the wild-type tracrRNA scaffold sequence may be 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCU CUCCAAUUCUGCACAA-3' (SEQ ID NO: 110).

In another embodiment, the tracrRNA scaffold sequence may be an engineered tracrRNA scaffold sequence. Here, the engineered tracrRNA scaffold sequence may be an engineered form of SEQ ID NO: 110 in which artificial modification (substitution, deletion, or insertion) is made at a partial nucleotide sequence in SEQ ID NO: 110. Alternatively, the engineered tracrRNA scaffold sequence may be an engineered form of SEQ ID NO: 110 in which modification is made to have a shorter length than SEQ ID NO: 110.

In an embodiment, the engineered tracrRNA scaffold sequence is a partial sequence of SEQ ID NO: 110, and this sequence may be a sequence of SEQ ID NO: 110 that does not contain a partial sequence of the 5' end or 3' end of SEQ ID NO: 110. For example, the engineered tracrRNA scaffold sequence may be 5'-ACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAA GUAACCCUCGAAACAAAUUCAUUUUUCCUCUCCAAUUCUGCACAA-3' (SEQ ID NO: 111), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUU-3' (SEQ ID NO: 112), 5'-ACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAA GUAACCCUCGAAACAAAUUCAUUU-3' (SEQ ID NO: 113), 5'-ACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAA GUAACCCUCGAAACAAA-3' (SEQ ID NO: 114), 5'-ACCGCUUCACCUUAGGAGUGAAGGUGGGCUGCUUGCAUCAGCCUAAUG UCGAGAAGUGCUUUCUUCGGAAAGUAACCCUCGAAACAAAUUCAUUU-3' (SEQ ID NO: 115),, or 5'-ACCGCUUCACUUAGAGUGAAGGUGGGCUGCUUGCAUCAGCCUAAUGUC GAGAAGUGCUUUCUUCGGAAAGUAACCCUCGAAACAAA-3' (SEQ ID NO: 116). These examples are provided for illustrative purposes only and are not intended to limit the disclosure.

In another embodiment, the engineered tracrRNA scaffold sequence is an engineered form of SEQ ID NO: 110 in which an artificial modification is made at a partial nucleotide sequence in SEQ ID NO: 110, and this sequence may be an engineered form of SEQ ID NO: 110 in which substitution, deletion, and/or addition are made at one or more nucleotides in SEQ ID NO: 110. For example, the engineered tracrRNA scaffold sequence may be 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAGUGCUCCUC UCCAAUUCUGCACAA-3' (SEQ ID NO: 117), 5'-ACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAA GUAACCCUCGAAACAAAUUCAGUGCUCCUCUCCAAUUCUGCACAA-3' (SEQ ID NO: 118), 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCA GUGCUCCUCUCCAAUUCUGCACAA-3' (SEQ ID NO: 119), 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCA GUGCUCCUCUC-3' (SEQ ID NO: 120), or 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCA GUGCU-3' (SEQ ID NO: 121). These examples are provided for illustrative purposes only and are not intended to limit the disclosure.

### iii) Linker

In a case where the scaffold region is a single scaffold sequence, the scaffold region further comprises a linker. The linker is a sequence that serves to link the tracrRNA scaffold sequence to the crRNA scaffold sequence. That is, the scaffold region is a single RNA molecule in which tracrRNA scaffold sequence and the crRNA scaffold sequence are connected to each other via a linker.

The linker may be a sequence that does not affect functions of the tracrRNA scaffold sequence and/or the crRNA scaffold sequence. Alternatively, the linker may be a sequence that does not form an RNA duplex with the tracrRNA scaffold sequence and/or the crRNA scaffold sequence.

The linker may be a sequence of 1 to 30 nucleotides.

In an embodiment, the linker may be a sequence of 1 to 5, 5 to 10, 10 to 15, 15 to 20, 20 to 25, or 25 to 30 nucleotides.

In another embodiment, the linker may be a sequence of 1 to 30, 5 to 30, 10 to 30, 15 to 30, 20 to 30, or 25 to 30 nucleotides.

In yet another embodiment, the linker may be a sequence of 1, 2, 3, 4, 5, 6, 7, 8, 9, 10, 11, 12, 13, 14, 15, 16 17, 18, 19, 20, 21, 22, 23, 24, 25, 26, 27, 28, 29, or 30 nucleotides. For example, the linker may be a sequence of 5'-GAAA-3', but is not limited thereto.

### iv) Examples of scaffold region

Based on the previous description, examples of the scaffold region will be described. Specific descriptions of the components included in the examples below are the same as those described above for the corresponding components. The examples are for illustrative purposes only, and the scope of the present disclosure is not limited thereto.

In an embodiment, the scaffold region may comprise a wild-type crRNA scaffold sequence and a wild-type tracrRNA scaffold sequence. The scaffold region may have a dual scaffold sequence. Here, the dual scaffold sequence may comprise the wild-type crRNA scaffold sequence and the wild-type tracrRNA scaffold sequence as separate RNA molecules. Alternatively, the scaffold region may have a single scaffold sequence. Here, the scaffold region may further comprise a linker, and the wild-type crRNA scaffold sequence and the wild-type tracrRNA scaffold sequence may be connected to each other via the linker.

In an embodiment, the scaffold region may comprise SEQ ID NOS: 101 and 110. Here, the scaffold region may optionally further comprise a linker. As an example, the scaffold region may comprise SEQ ID NO: 110, a linker, and SEQ ID NO: 101 sequentially in a 5' to 3' direction. For example, the scaffold region may have 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCU CUCCAAUUCUGCACAAGAAAGUUGCAGAACCCGAAUAGACGAAUGAAG GAAUGCAAC-3' (SEQ ID NO: 122).

In another embodiment, the scaffold region may comprise a wild-type crRNA scaffold sequence and an engineered tracrRNA scaffold sequence. The scaffold region may have a dual scaffold sequence. Here, the dual scaffold sequence may comprise the wild-type crRNA scaffold sequence and the engineered tracrRNA scaffold sequence as separate RNA molecules. Alternatively, the scaffold region may have a single scaffold sequence. Here, the scaffold region may further comprise a linker, and the wild-type crRNA scaffold sequence and the engineered tracrRNA scaffold sequence may be connected to each other via the linker.

In an embodiment, the scaffold region may comprise i) a sequence of SEQ ID NO: 101, and ii) a sequence of SEQ ID NO: 110, in each of which substitution and/or deletion of one or more nucleotides are made, and/or insertion of one or more other nucleotides is made. Here, the scaffold region may optionally further comprise a linker. For example, the scaffold region may have SEQ ID NO: 101 and SEQ ID NO: 117. Alternatively, the scaffold region may comprise SEQ ID NO: 117, a linker, and SEQ ID NO: 101 sequentially in a 5' to 3' direction. For example, the scaffold region may have 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAGUGCUCCUC UCCAAUUCUGCACAAGAAAGUUGCAGAACCCGAAUGAAGGAAUGCAAC-3' (SEQ ID NO: 123).

In another embodiment, the scaffold region may comprise an engineered crRNA scaffold sequence and an engineered tracrRNA scaffold sequence. The scaffold region may have a dual scaffold sequence. Here, the dual scaffold sequence may comprise the engineered crRNA scaffold sequence and the engineered tracrRNA scaffold sequence as separate RNA molecules. Alternatively, the scaffold region may have a single scaffold sequence. Here, the scaffold region may further comprise a linker, and the engineered crRNA scaffold sequence and the engineered tracrRNA scaffold sequence may be connected to each other via the linker.

In an embodiment, the scaffold region may comprise i) a sequence of SEQ ID NO: 101 in which substitution and/or deletion of one or more nucleotides are made, and/or insertion of one or more other nucleotides is made, and ii) a sequence of SEQ ID NO: 110 in which substitution and/or deletion of one or more nucleotides are made, and/or insertion of one or more other nucleotides is made. Here, the scaffold region may optionally further comprise a linker. For example, the scaffold region may have SEQ ID NO: 106 and SEQ ID NO: 116. Alternatively, the scaffold region may comprise SEQ ID NO: 116, a linker, and SEQ ID NO: 106 sequentially in a 5' to 3' direction. For example, the scaffold region may have 5'-ACCGCUUCACUUAGAGUGAAGGUGGGCUGCUUGCAUCAGCCUAAUGUC GAGAAGUGCUUUCUUCGGAAAGUAACCCUCGAAACAAAGAAAGGAAUG CAAC-3' (SEQ ID NO: 124). As another example, the scaffold region may have SEQ ID NO: 106 and SEQ ID NO: 114. Alternatively, the scaffold region may comprise SEQ ID NO: 114, a linker, and SEQ ID NO: 106 sequentially in a 5' to 3' direction. For example, the scaffold region may have 5'-ACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAA GUAACCCUCGAAACAAAGAAAGGAAUGCAAC-3' (SEQ ID NO: 125). As yet another example, the scaffold region may have SEQ ID NO: 107 and SEQ ID NO: 118. Alternatively, the scaffold region may comprise SEQ ID NO: 118, a linker, and SEQ ID NO: 107 sequentially in a 5' to 3' direction. For example, the scaffold region may have 5'-ACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAA GUAACCCUCGAAACAAAUUCAGUGCUCCUCUCCAAUUCUGCACAAGAAA GUUGCAGAACCCGAAUAGAGCAAUGAAGGAAUGCAAC-3' (SEQ ID NO: 126).

### 2-3) U-rich tail sequence

The guide RNA may optionally further comprise a U-rich tail sequence. The U-rich tail sequence is a uridine (U)-rich sequence added to the 3' end of the guide RNA.

The U-rich tail sequence may be 5'-(UₐN)_{d}Uₑ-3', 5'-UₐVUₐVUₑ-3', or 5'-UₐVUₐVUₐVUₑ-3'. Here, N may be A, C, G or U, and each V may be independently A, C or G. Here, a may be an integer of 0 to 4, d may be an integer of 0 to 3, and e may be an integer of 1 to 10.

In an embodiment, the U-rich tail sequence may be 5'-(UₐU)_{d}Uₑ-3', that is, Uₓ. Here, x may be an integer of 0 to 22. For example, the U-rich tail sequence may be U, UU, UUU, UUUU, UUUUU, UUUUUU, UUUUUUU, UUUUUUUU or UUUUUUUUU.

In another embodiment, the U-rich tail sequence may be 5'-(UₐA)_{d}Uₑ-3', 5'-(UₐG)_{d}Uₑ-3', or 5'-(UₐC)_{d}Uₑ-3'. For example, the U-rich tail sequence may be UUUUAUUUU, UUUAUUUUU, UUUUGUUUU, UGUGUGUU, UUUUCUUUU, or UUCUUCUUU.

In yet another embodiment, the U-rich tail sequence may be 5'-UₐAUₐAUₑ-3', 5'-UₐAUₐCUₑ-3', 5'-UₐAUₐGUₑ-3', 5'-UₐCUₐAUₑ-3', 5'-UₐCUₐCUₑ-3', 5'-UₐCUₐGUₑ-3', 5'-UₐGUₐAUₑ-3', 5'-UₐGUₐCUₑ-3', or 5'-UₐGUₐGUₑ-3'. For example, the U-rich tail sequence may be UUAUUUAUU, UUUCUAUUUU (SEQ ID NO: 127), UGUCU, UUAUGUUUUU (SEQ ID NO: 128), or UCUUUUGUU.

In still yet another embodiment, the U-rich tail sequence may be 5'-UₐAUₐAUₐAUₑ-3', 5'-UₐAUₐAUₐCUₑ-3', 5'-UₐAUₐAUₐGUₑ-3', 5'-UₐAUₐCUₐAUₑ-3', 5'-UₐAUₐCUₐCUₑ-3', 5'-UₐAUₐCUₐGUₑ-3', 5'-UₐAUₐGUₐAUₑ-3', 5'-UₐAUₐGUₐCUₑ-3', 5'-UₐAUₐGUₐGUₑ-3', 5'-UₐCUₐAUₐAUₑ-3', 5'-UₐCUₐAUₐCUₑ-3', 5'-UₐCUₐAUₐGUₑ-3', 5'-UₐCUₐCUₐAUₑ-3', 5'-UₐCUₐCUₐCUₑ-3', 5'-UₐCUₐCUₐGUₑ-3', 5'-UₐCUₐGUₐAUₑ-3', 5'-UₐCUₐGUₐCUₑ-3', 5'-UₐCUₐGUₐGUₑ-3', 5'-UₐGUₐAUₐAUₑ-3', 5'-UₐGUₐAUₐCUₑ-3', 5'-UₐGUₐAUₐGUₑ-3', 5'-UₐGUₐCUₐAUₑ-3', 5'-UₐGUₐCUₐCUₑ-3', 5'-UₐGUₐCUₐGUₑ-3', 5'-UₐGUₐGUₐAUₑ-3', 5'-UₐGUₐGUₐCUₑ-3', or 5'-UₐGUₐGUₐGUₑ-3'. For example, the U-rich tail sequence may be UUAUUCUG, UUCUCUUCU, or UGUUAUAUU.

### 2-4) Examples of guide RNA

Based on the previous description, examples of the guide RNA will be described. Specific descriptions of the components included in the examples below are the same as those described above for the corresponding components. The examples are for illustrative purposes only, and the scope of the present disclosure is not limited thereto.

In an embodiment, the guide RNA may comprise a guide sequence, a wild-type crRNA scaffold sequence, and a wild-type tracrRNA scaffold sequence. Alternatively, the guide RNA may comprise a guide sequence, a wild-type crRNA scaffold sequence, a linker, and a wild-type tracrRNA scaffold sequence. The guide RNA may optionally further comprise a U-rich tail sequence.

In an embodiment, the guide RNA may comprise a wild-type tracrRNA scaffold sequence, a wild-type crRNA scaffold sequence, and a guide sequence. Here, the guide sequence may be covalently linked to the 3' end of the wild-type crRNA scaffold sequence. Here, the wild-type tracrRNA scaffold sequence and the wild-type crRNA scaffold sequence may be each independently an RNA molecule. Here, the guide RNA may be a dual guide RNA. The guide RNA may optionally further comprise a U-rich tail sequence. Here, the U-rich tail sequence may be covalently linked to the 3' end of the guide sequence. As an example, the guide RNA may comprise SEQ ID NO: 110 and 5'-[SEQ ID NO: 101]-[guide sequence]-3'. Here, the guide sequence may be any one sequence selected from the sequences described in Table 3 or Table 4. As another example, the guide RNA may comprise SEQ ID NO: 110 and 5'-[SEQ ID NO: 101]-[guide sequence]-[U-rich-tail sequence]-3'. Here, the guide sequence may be any one sequence selected from the sequences described in Table 3 or Table 4.

In another embodiment, the guide RNA may comprise a wild-type tracrRNA scaffold sequence, a linker, a wild-type crRNA scaffold sequence, and a guide sequence. Here, the wild-type tracrRNA scaffold sequence, the linker, the wild-type crRNA scaffold sequence, and the guide sequence may be located sequentially in a 5' to 3' direction (5'-[wild-type tracrRNA scaffold sequence]-[linker]-[wild-type crRNA scaffold sequence]-[guide sequence]-3'). The guide RNA may be a single guide RNA. The guide RNA may optionally further comprise a U-rich tail sequence. Here, the U-rich tail sequence may be covalently linked to the 3' end of the guide sequence. As an example, the guide RNA may comprise 5'-[SEQ ID NO: 110]-[linker]-[SEQ ID NO: 101]-[guide sequence]-3'. Here, the guide sequence may be any one sequence selected from the sequences described in Table 3 or Table 4. In another example, the guide RNA may comprise 5'-[SEQ ID NO: 110]-[linker]-[SEQ ID NO: 101]-[guide sequence]-[U-rich tail sequence]-3'. Here, the guide sequence may be any one sequence selected from the sequences described in Table 3 or Table 4.

In another embodiment, the guide RNA may comprise a guide sequence, an engineered crRNA scaffold sequence, and an engineered tracrRNA scaffold sequence. Alternatively, the guide RNA may comprise a guide sequence, an engineered crRNA scaffold sequence, a linker, and an engineered tracrRNA scaffold sequence. The guide RNA may optionally further comprise a U-rich tail sequence.

In an embodiment, the guide RNA may comprise an engineered tracrRNA scaffold sequence, an engineered crRNA scaffold sequence, and a guide sequence. Here, the guide sequence may be covalently linked to the 3' end of the engineered crRNA scaffold sequence. Here, the engineered tracrRNA scaffold sequence and the engineered crRNA scaffold sequence may be each independently an RNA molecule. Here, the guide RNA may be a dual guide RNA. The guide RNA may optionally further comprise a U-rich tail sequence. Here, the U-rich tail sequence may be covalently linked to the 3' end of the guide sequence. As an example, the guide RNA may comprise SEQ ID NO: 116 and 5'-[SEQ ID NO: 106]-[guide sequence]-3'. Alternatively, the guide RNA may comprise SEQ ID NO: 116 and 5'-[SEQ ID NO: 106]-[guide sequence]-[U-rich tail sequence]-3'. Here, the guide sequence may be any one sequence selected from the sequences described in Table 3 or Table 4. As another example, the guide RNA may comprise SEQ ID NO: 114 and 5'-[SEQ ID NO: 106]-[guide sequence]-3'. Alternatively, the guide RNA may comprise SEQ ID NO: 114 and 5'-[SEQ ID NO: 106]-[guide sequence]-[U-rich tail sequence]-3'. Here, the guide sequence may be any one sequence selected from the sequences described in Table 3 or Table 4. As yet another example, the guide RNA may comprise SEQ ID NO: 118 and 5'-[SEQ ID NO: 107]-[guide sequence]-3'. Alternatively, the guide RNA may comprise SEQ ID NO: 118 and 5'-[SEQ ID NO: 107]-[guide sequence]-[U-rich tail sequence]-3'. Here, the guide sequence may be any one sequence selected from the sequences described in Table 3 or Table 4.

In another embodiment, the guide RNA may comprise an engineered tracrRNA scaffold sequence, a linker, an engineered crRNA scaffold sequence, and a guide sequence. Here, the engineered tracrRNA scaffold sequence, the linker, the engineered crRNA scaffold sequence, and the guide sequence may be located sequentially in a 5' to 3' direction (5'-[engineered tracrRNA scaffold sequence]-[linker]-[engineered crRNA scaffold sequence]-[guide sequence]-3'). The guide RNA may be a single guide RNA. The guide RNA may optionally further comprise a U-rich tail sequence. Here, the U-rich tail sequence may be covalently linked to the 3' end of the guide sequence. As an example, the guide RNA may comprise 5'-[SEQ ID NO: 116]-[linker]-[SEQ ID NO: 106]-[guide sequence]-3'. Alternatively, the guide RNA may comprise 5'-[SEQ ID NO: 116]-[linker]-[SEQ ID NO: 106]-[guide sequence]-[U-rich tail sequence]-3'. Here, the guide sequence may be any one sequence selected from the sequences described in Table 3 or Table 4. As another example, the guide RNA may comprise 5'-[SEQ ID NO: 114]-[linker]-[SEQ ID NO: 106]-[guide sequence]-3'. Alternatively, the guide RNA may comprise 5'-[SEQ ID NO: 114]-[linker]-[SEQ ID NO: 106]-[guide sequence]-[U-rich tail sequence]-3'. Here, the guide sequence may be any one sequence selected from the sequences described in Table 3 or Table 4. As yet another example, the guide RNA may comprise 5'-[SEQ ID NO: 118]-[linker]-[SEQ ID NO: 107]-[guide sequence]-3'. Alternatively, the guide RNA may comprise 5'-[SEQ ID NO: 118]-[linker]-[SEQ ID NO: 107]-[guide sequence]-[U-rich tail sequence]-3'. Here, the guide sequence may be any one sequence selected from the sequences described in Table 3 or Table 4.

### 3. Cas12f1 (Cas14a1) protein

The CRISPR/Cas12f1 system for treatment of LCA10 disease comprises a Cas12f1 (Cas14a1) protein. The Cas12f1 protein, which is a major protein component of the CRISPR/Cas12f1 (Cas14a1) system, is one of the effector proteins named Cas14 in a previous study (Harrington et al., Science, 362, 839-842 (2018)) and is also called a Cas14a1 protein. In the present specification, the Cas12f1 protein is used interchangeably with the Cas14a1 protein or Cas12f1 (Cas14a1) protein. The Cas12f1 protein disclosed herein may be a wildtype Cas12f1 protein (wildtype Cas14a1 protein) existing in nature. Alternatively, the Cas12f1 protein may be a variant of the wildtype Cas12f1 protein, wherein the variant is referred to as "Cas12f1 variant" or "Cas14a1 variant." The Cas 12f1 variant may be a variant having the same function as the wildtype Cas12f1 protein, a variant of which some or all functions are modified as compared with the wildtype Cas12f1 protein, and/or a variant to which an additional function is added as compared with the wildtype Cas12f1 protein. The meaning of the Cas12f1 protein may be appropriately interpreted according to the context, and is interpreted in the broadest sense except for a particular case. Hereinafter, the Cas12f1 protein will be described in detail.

### 3-1) Wildtype Cas12f1 (Cas14a1) protein

The Cas12f1 protein may be a wild-type Cas12f1 protein. Here, the Cas12f1 protein is capable of cleaving a double-strand or single-strand of a target nucleic acid or target gene. The Cas12f1 protein is capable of recognizing a protospacer adjacent motif (PAM) sequence present in a target nucleic acid or target gene. Here, the PAM sequence is a unique sequence determined depending on the Cas14a1 protein. The PAM sequence for the Cas12f1 protein may be a T-rich sequence. The PAM sequence for the Cas12f1 protein may be 5'-TTTR-3'. Here, R may be A or G. For example, the PAM sequence may be 5'-TTTA-3' or 5'-TTTG-3'.

In an embodiment, the Cas12f1 protein may be derived from the Cas14 family (Harrington et al., Science 362, 839-842 (2018); US 2020/0172886 A1).

In another embodiment, the Cas12f1 protein may be a Cas14a1 protein derived from an uncultured archaeon (Harrington et al., Science 362, 839-842 (2018); US 2020/0172886 A1). For example, the Cas14a1 protein may be the amino acid sequence of SEQ ID NO: 129.

**[Table 5] Amino acid sequence of Cas14a1 protein**

| Name | Amino acid sequence |
|---|---|
| Cas12f1 protein | mAKNTITKTLKLRIVRPYNSAEVEKIVADEKNNREKI ALEKNKDKVKEACSKHLKVAAYCTTQVERNACLFC KARKLDDKFYQKLRGQFPDAVFWQEISEIFRQLQK QAAEIYNQSLIELYYEIFIKGKGIANASSVEHYLSDVC YTRAAELFKNAAIASGLRSKIKSNFRLKELKNMKSGL PTTKSDNFPIPLVKQKGGQYTGFEISNHNSDFIIKIPF GRWQVKKEIDKYRPWEKFDFEQVQKSPKPISLLLST QRRKRNKGWSKDEGTEAEIKKVMNGDYQTSYIEVK RGSKIGEKSAWMLNLSIDVPKIDKGVDPSIIGGIDVG VKSPLVCAINNAFSRYSISDNDLFHFNKKMFARRRIL LKKNRHKRAGHGAKNKLKPITILTEKSERFRKKLIER WACEIADFFIKNKVGTVQMENLESMKRKEDSYFNIR LRGFWPYAEMQNKIEFKLKQYGIEIRKVAPNNTSKT CSKCGHLNNYFNFEYRKKNKFPHFKCEKCNFKENA DYNAALNISNPKLKSTKEEP (SEQ ID NO: 129) |

### 3-2) Cas12f1 (Cas14a1) variant - Cas12f1 (Cas14a1) mutant

The Cas12f1 protein may be a Cas12f1 variant. The Cas12f1 variant may be a variant of a wildtype Cas12f1 protein in which at least one amino acid in the amino acid sequence of the wildtype Cas12f1 protein is modified. Here, the modification may be deletion and/or substitution. Alternatively, the Cas12f1 variant may be a variant of a wildtype Cas12f1 protein in which at least one amino acid sequence is added to both ends of the amino acid sequence of the wild-type Cas12f1 protein and/or within the amino acid sequence thereof. Here, the modification may be insertion. Here, the Cas12f1 variant is referred to as "Cas12f1 mutant" or "Cas14a1 mutant."

In an embodiment, the Cas12f1 mutant may be obtained by deleting at least one amino acid in an amino acid sequence of a wildtype Cas12f1 protein. For example, the Cas12f1 mutant may be obtained by deleting at least one amino acid in a RuvC domain included in a wildtype Cas12f1 protein. Alternatively, the Cas12f1 mutant may be obtained by deleting at least one amino acid in a domain that recognizes a PAM included in a wildtype Cas12f1 protein. Alternatively, the Cas12f1 mutant may be obtained by deleting at least one amino acid in the amino acid sequence of SEQ ID NO: 129.

In another embodiment, the Cas12f1 mutant may be obtained by substituting at least one amino acid in an amino acid sequence of a wildtype Cas12f1 protein with other amino acid(s). Here, the substitution may be such that one amino acid is substituted with one other amino acid. Alternatively, the substitution may be such that one amino acid is substituted with a plurality of other amino acids. Alternatively, the substitution may be such that a plurality of amino acids are substituted with one other amino acid. Alternatively, the substitution may be such that a plurality of amino acids are substituted with a plurality of other amino acids wherein the number of amino acids to be substituted and the number of substituting amino acids may be the same or different from each other. For example, the Cas12f1 mutant may be obtained by substituting at least one amino acid in a RuvC domain included in a wildtype Cas12f1 protein with other amino acid(s). Alternatively, the Cas12f1 mutant may be obtained by substituting at least one amino acid in a domain, which recognizes a PAM included in a wildtype Cas12f1 protein, with other amino acid(s). Alternatively, the Cas12f1 mutant may be obtained by substituting at least one amino acid in the amino acid sequence of SEQ ID NO: 129 with other amino acid(s).

The Cas12f1 mutant may be a variant having the same function as a wildtype Cas12f1 protein or a variant of which some or all functions are modified as compared with a wildtype Cas12f1 protein. For example, the Cas12f1 mutant may be a variant in which modification is made to cleave only one strand in a double-strand of a target nucleic acid. Alternatively, the Cas12f1 mutant may be a variant in which modification is made to recognize a PAM sequence other than 5'-TTTA-3' or 5'-TTTG-3'.

The inventors of the present application made research and have found that among the mutants in which amino acids are added to the N-terminus and/or C-terminus of the amino acid sequence of the wild-type Cas12f1 protein, there is a Cas12f1 mutant having the same function as the Cas12f1 protein. Furthermore, a Cas12f1 mutant having the same function as the wild-type Cas12f1 protein was invented by adding an amino acid sequence of a certain length to the N-terminus of the wild-type Cas12f1 protein. The Cas12f1 variant is disclosed in detail in Korean Patent Application No. 10-2021-0181875.

In an embodiment, the Cas12f1 variant may be a variant of a wild-type Cas12f1 protein in which 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids are added to the N-terminus and/or C-terminus of the wild-type Cas 12f1 protein.

In an embodiment, the Cas12f1 variant may be a variant of a wild-type Cas12f1 protein in which the number of amino acids within the above-mentioned two numerical ranges is added to the N-terminus and/or C-terminus of the wild-type Cas12f1 protein. For example, the Cas12f1 variant may be a variant of a wild-type Cas12f1 protein in which 26 to 28 amino acids are added to the N-terminus of the wild-type Cas12f1 protein.

In an embodiment, the Cas12f1 variant may be a protein represented by an amino acid sequence selected from SEQ ID NOS: 288 to 291. Among them, the protein represented by SEQ ID NO: 288 is also known as transposon-associated transposase B (TnpB) derived from Candidatus Woesearchaeota archaeon, and the terms such as Cas12f1 protein, Cas12f1 variant, Cas12f1 functional analog, TnpB, and TnpB functional analog may be used interchangeably in the present specification.

### 3-3) Cas12f1 (Cas14a1) variant - Cas12f1 (Cas14a1) fusion protein

The Cas12f1 protein may be a Cas12f1 variant. The Cas12f1 variant may be a variant obtained by adding a domain, peptide or protein having an additional function to a wildtype Cas12f1 protein or a Cas12f1 mutant. Here, the variant, to which the domain, peptide or protein having an additional function is added, is referred to as "Cas12f1 fusion protein" or "Cas14a1 fusion protein." The domain, peptide or protein having an additional function may be added to the N-terminus and/or C-terminus of a wildtype Cas12f1 protein or a Cas12f1 mutant, and/or within the amino acid sequence thereof. The domain, peptide or protein having an additional function may be a domain, peptide or protein having the same or different function as a wildtype Cas12f1 protein. For example, the domain, peptide or protein having an additional function may be a domain, peptide or protein having methylase activity, demethylase activity, transcription activation activity, transcription repression activity, transcription release factor activity, histone modification activity, RNA cleavage activity or nucleic acid binding activity, or may be a tag or reporter protein used for isolation and purification of a protein (including a peptide), but is not limited thereto. Alternatively, the domain, peptide or protein having an additional function may be reverse transcriptase or deaminase.

### 3-4) Cas12f1 (Cas14a1) variant - others

The Cas12f1 protein may be a Cas12f1 variant. The Cas12f1 variant may be a variant of a wildtype Cas12f1 protein, a Cas12f1 mutant, or a Cas12f1 fusion protein, in which a nuclear localization sequence (NLS) or a nuclear export sequence (NES) is optionally further included. The NLS and NES are signal peptides for positioning the Cas12f1 protein in or outside a cell. These terms include all meanings recognized by those skilled in the art, and may be appropriately interpreted according to the context. For example, the NLS may be, but is not limited to, an NLS sequence derived from the NLS of an SV40 virus large T-antigen, having the amino acid sequence PKKKRKV (SEQ ID NO: 130); the NLS from a nucleoplasmin (for example, the nucleoplasmin bipartite NLS having the sequence KRPAATKKAGQAKKKK (SEQ ID NO: 131)); the c-myc NLS having the amino acid sequence PAAKRVKLD (SEQ ID NO: 132) or RQRRNELKRSP (SEQ ID NO: 133); the hRNPA1 M9 NLS having the sequence NQSSNFGPMKGGNFGGRSSGPYGGGGQYFAKPRNQGGY (SEQ ID NO: 134); the sequence RMRIZFKNKGKDTAELRRRRVEVSVELRKAKKDEQILKRRNV (SEQ ID NO: 135) of an IBB domain from importin alpha; the sequences VSRKRPRP (SEQ ID NO: 136) and PPKKARED (SEQ ID NO: 137) of myoma T protein; the sequence PQPKKKPL (SEQ ID NO: 138) of human p53; the sequence SALIKKKKKMAP (SEQ ID NO: 139) of mouse c-abl IV; the sequences DRLRR (SEQ ID NO: 140) and PKQKKRK (SEQ ID NO: 141) of influenza virus NS1; the sequence RKLKKKIKKL (SEQ ID NO: 142) of hepatitis virus delta antigen; the sequence REKKKFLKRR (SEQ ID NO: 143) of mouse Mx1 protein; the sequence KRKGDEVDGVDEVAKKKSKK (SEQ ID NO: 144) of human poly (ADP-ribose) polymerase; or the sequence RKCLQAGMNLEARKTKK (SEQ ID NO: 145) of steroid hormone receptor (human) glucocorticoid.

In addition, the Cas12f1 variant may be a variant of a wildtype Cas12f1 protein, a Cas12f1 mutant, or a Cas12f1 fusion protein, in which a tag is optionally further included. The tag is a functional domain, peptide or protein for isolation and purification and/or tracking of the Cas12f1 protein, and the tag includes, but is not limited to, a tag protein such as a histidine (His) tag, a V5 tag, a FLAG tag, an influenza hemagglutinin (HA) tag, a Myc tag, a VSV-G tag, and a thioredoxin (Trx) tag; a fluorescent protein such as green fluorescent protein (GFP), yellow fluorescent protein (YFP), cyan fluorescent protein (CFP), blue fluorescent protein (BFP), HcRED, and DsRed; and a reporter protein (enzyme) such as glutathione-S-transferase (GST), horseradish peroxidase (HRP), chloramphenicol acetyltransferase (CAT) beta-galactosidase, beta-glucuronidase, and luciferase.

### 4. Guide RNA-Cas12f1(Cas14a1) protein(s) complex (CRISPR/Cas12f1 complex or CRISPR complex)

The CRISPR/Cas12f1 system for treatment of LCA10 disease may operate in a form of a guide RNA-Cas12f1 (Cas14a1) protein(s) complex (CRISPR/Cas12f1 complex or CRISPR complex). The CRISPR complex comprises a guide RNA and a Cas12f1 protein. Here, the CRISPR complex comprises a guide RNA and two Cas12f1 proteins (Satoru N. Takeda et al., Molecular Cell, 81, 1-13, (2021)). The CRISPR complex may be a ribonucleoprotein (RNP) formed by interaction of a guide RNA with a Cas12f1 protein. Here, the CRISPR complex may be a ribonucleoprotein (RNP) formed by interaction of a guide RNA with one Cas12f1 protein or an RNP formed by interaction of a guide RNA with two Cas12f1 proteins (Satoru N. Takeda et al., Molecular Cell, 81, 1-13, (2021)). The CRISPR complex is referred to as " guide RNA-Cas12f1 (Cas14a1) protein complex", "CRISPR/Cas12f1 complex," or "CRISPR/Cas14a1 complex", and the terms are used interchangeably herein. Specific descriptions of the components included in the examples below are the same as those described above for the corresponding components. The examples are for illustrative purposes only, and the scope of the present disclosure is not limited thereto.

In an embodiment, the CRISPR complex may be an RNP formed by combination of a wildtype Cas12f1 protein and a guide RNA. Here, the CRISPR complex may comprise two wildtype Cas12f1 proteins and a guide RNA. Here, the wild-type Cas12f1 protein may have the amino acid sequence of SEQ ID NO: 129. Here, the guide RNA may be one of the examples described in the section "2-4) Examples of guide RNA" described above.

In another embodiment, the CRISPR complex may be an RNP formed by combination of a Cas12f1 variant and a guide RNA. Here, the CRISPR complex may comprise two Cas12f1 variants and a guide RNA. Here, the Cas12f1 variant may be a Cas12f1 mutant or a Cas12f1 fusion protein. Here, the guide RNA may be one of the examples described in the section "2-4) Examples of guide RNA" described above.

In yet another embodiment, the CRISPR complex may be an RNP formed by combination of a wild-type Cas12f1 protein, a Cas12f1 mutant, and a guide RNA. Here, the wild-type Cas12f1 protein may have the amino acid sequence of SEQ ID NO: 129. Here, the Cas12f1 variant may be a Cas12f1 mutant or a Cas12f1 fusion protein. Here, the guide RNA may be one of the examples described in the section "2-4) Examples of guide RNA" described above.

### <Composition for treatment of LCA10 disease>

In an aspect disclosed herein, there is provided a composition for treatment of LCA10 disease. The composition comprises a CRISPR/Cas12f1 system. The CRISPR/Cas12f1 system is as described in the section "<CRISPR/Cas12f1 system for treatment of LCA10 disease>" described above, and has the same characteristics and structure as each configuration described in the section. The CRISPR/Cas12f1 system is used to induce deletion or inversion of all or part of the 128 base pair cryptic exon. Therefore, the composition effectively deletes or inverts all or part of the cryptic exon that causes LCA10 disease, and thus may be used as an effective therapeutic agent for LCA10 disease.

More specifically, the composition may comprise:
at least one guide RNA targeting the CEP290 gene or a nucleic acid encoding the same; and
a Cas12f1 protein or a nucleic acid encoding the same.

Here, the at least one guide RNA targeting the CEP290 gene may complementarily bind to a target sequence present in the CEP290 gene.

Here, the nucleic acid encoding the guide RNA may be DNA encoding the guide RNA to transcribe the guide RNA.

Here, the nucleic acid encoding the Cas12f1 protein may be a nucleic acid encoding a wild-type Cas12f1 protein or a nucleic acid encoding a Cas12f1 variant. The nucleic acid encoding the Cas12f1 protein may be codon-optimized according to a target into which the Cas12f1 protein is to be introduced. "Codon optimization" refers to a process of modifying a native nucleic acid sequence for enhanced expression in a cell of interest by replacing at least one codon in the native sequence with a codon, which is used more frequently or most frequently in a gene of a target cell, while maintaining its native amino acid sequence. Different species have specific biases for specific codons of specific amino acids, and codon bias (differences in codon usage between organisms) is often correlated with translation efficiency of an mRNA, which is considered to be dependent on the nature of codons being translated and availability of specific tRNA molecules. Predominance of tRNA selected in a cell generally reflects the most frequently used codon in peptide synthesis. Thus, genes may be tailored for optimal gene expression in a given organism based on codon optimization. In an embodiment, the nucleic acid encoding the Cas12f1 protein may be a nucleic acid encoding a human codon-optimized Cas12f1 protein. For example, the nucleic acid encoding the human codon-optimized Cas12f1 protein may be SEQ ID NO: 146.

**[Table 6] Human codon-optimized nucleic acid encoding Cas12f1 protein**

| Name | Sequence |
|---|---|
| Human codon-optimized Cas12f1 encoding DNA | ATGGCCAAGAACACAATTACAAAGACACTGAAGCTGAGGATCGTGAGACCATACAACAGCGC TGAGGTCGAGAAGATTGTGGCTGATGAAAAGAACAACAGGGAAAAGATCGCCCTCGAGAAGA ACAAGGATAAGGTGAAGGAGGCCTGCTCTAAGCACCTGAAAGTGGCCGCCTACTGCACCACAC AGGTGGAGAGGAACGCCTGTCTGTTTTGTAAAGCTCGGAAGCTGGATGATAAGTTTTACCAGA AGCTGCGGGGCCAGTTCCCCGATGCCGTCTTTTGGCAGGAGATTAGCGAGATCTTCAGACAGC TGCAGAAGCAGGCCGCCGAGATCTACAACCAGAGCCTGATCGAGCTCTACTACGAGATCTTCA TCAAGGGCAAGGGCATTGCCAACGCCTCCTCCGTGGAGCACTACCTGAGCGACGTGTGCTACA CAAGAGCCGCCGAGCTCTTTAAGAACGCCGCTATCGCTTCCGGGCTGAGGAGCAAGATTAAGA GTAACTTCCGGCTCAAGGAGCTGAAGAACATGAAGAGCGGCCTGCCCACTACAAAGAGCGACA ACTTCCCAATTCCACTGGTGAAGCAGAAGGGGGGCCAGTACACAGGGTTCGAGATTTCCAACC ACAACAGCGACTTTATTATTAAGATCCCCTTTGGCAGGTGGCAGGTCAAGAAGGAGATTGACA AGTACAGGCCCTGGGAGAAGTTTGATTTCGAGCAGGTGCAGAAGAGCCCCAAGCCTATTTCCC TGCTGCTGTCCACACAGCGGCGGAAGAGGAACAAGGGGTGGTCTAAGGATGAGGGGACCGAG GCCGAGATTAAGAAAGTGATGAACGGCGACTACCAGACAAGCTACATCGAGGTCAAGCGGGG CAGTAAGATTGGCGAGAAGAGCGCCTGGATGCTGAACCTGAGCATTGACGTGCCAAAGATTG ATAAGGGCGTGGATCCCAGCATCATCGGAGGGATCGATGTGGGGGTCAAGAGCCCCCTCGTGT GCGCCATCAACAACGCCTTCAGCAGGTACAGCATCTCCGATAACGACCTGTTCCACTTTAACAA GAAGATGTTCGCCCGGCGGAGGATTTTGCTCAAGAAGAACCGGCACAAGCGGGCCGGACACG GGGCCAAGAACAAGCTCAAGCCCATCACTATCCTGACCGAGAAGAGCGAGAGGTTCAGGAAG AAGCTCATCGAGAGATGGGCCTGCGAGATCGCCGATTTCTTTATTAAGAACAAGGTCGGAACA GTGCAGATGGAGAACCTCGAGAGCATGAAGAGGAAGGAGGATTCCTACTTCAACATTCGGCT GAGGGGGTTCTGGCCCTACGCTGAGATGCAGAACAAGATTGAGTTTAAGCTGAAGCAGTACG GGATTGAGATCCGGAAGGTGGCCCCCAACAACACCAGCAAGACCTGCAGCAAGTGCGGGCACC TCAACAACTACTTCAACTTCGAGTACCGGAAGAAGAACAAGTTCCCACACTTCAAGTGCGAGA AGTGCAACTTTAAGGAGAACGCCGATTACAACGCCGCCCTGAACATCAGCAACCCTAAGCTGA AGAGCACTAAGGAGGAGCCC (SEQ ID NO: 146) |

Here, the composition may optionally further comprise at least one guide RNA or a nucleic acid encoding the same. Here, the composition may comprise a plurality of guide RNAs, and each of the plurality of guide RNAs may recognize a different target sequence. Here, the composition optionally further comprises at least one Cas12f1 protein or a nucleic acid encoding the same. Here, the composition may comprise two or more Cas12f1 proteins having different amino acid sequences. Here, the two or more Cas12f1 proteins having different amino acid sequences may be a wild-type Cas12f1 protein and a Cas14a1 variant.

The guide RNA is as described in the section "2. Guide RNA" described above, and has the same characteristics and structure as each configuration described in the section. In addition, the Cas12f1 protein is as described in the section "3. Cas12f1 (Cas14a1) protein" described above, and has the same characteristics and structure as each configuration described in the section.

The composition may be in a form of a nucleic acid. Alternatively, the composition may be in a form of a mixed form of a nucleic acid and a protein. Hereinafter, the form of the composition will be described in detail.

### 1. Form of nucleic acid

The composition may be in a form of a nucleic acid. The nucleic acid may be in a form of DNA or RNA, or in a mixed form thereof. The nucleic acid may be in a form of a vector. Here, the vector may be a viral vector or a non-viral vector.

In addition, a shape of the nucleic acid may be circular or linear.

In addition, a shape of the nucleic acid may be double-stranded or single-stranded.

### 1-1) Viral vector

The composition may be in a form of a viral vector. For example, the viral vector may be one or more viral vectors selected from the group consisting of a retroviral (retrovirus) vector, a lentiviral (lentivirus) vector, an adenoviral (adenovirus) vector, an adeno-associated viral (adeno-associated virus; AAV) vector, a vaccinia viral (vaccinia virus) vector, a poxviral (poxvirus) vector, and a herpes simplex viral (herpes simplex virus) vector.

In an embodiment, the composition may be in a form of a viral vector. That is, the composition may comprise a nucleic acid encoding a guide RNA and a nucleic acid encoding a Cas12f1 protein in a form of a viral vector. Here, the composition may comprise a first viral vector comprising a nucleic acid encoding a guide RNA and a second viral vector comprising a nucleic acid encoding a Cas 12f1 protein. Alternatively, the composition may comprise a viral vector comprising both a nucleic acid encoding a guide RNA and a nucleic acid encoding a Cas12f1 protein. In an embodiment, the composition may comprise an adeno-associated viral vector comprising both a nucleic acid encoding a guide RNA and a nucleic acid encoding a Cas 12f1 protein.

In another embodiment, in a case where the composition comprises nucleic acids encoding a plurality of guide RNAs and a nucleic acid encoding a Cas12f1 protein, the composition may comprise a first viral vector comprising all of the nucleic acids encoding the plurality of guide RNAs and a second viral vector comprising the nucleic acid encoding the Cas12f1 protein. Alternatively, the composition may be in a form of a single viral vector comprising all of nucleic acids encoding a plurality of guide RNAs and a nucleic acid encoding a Cas12f1 protein. Alternatively, the composition may comprise a plurality of (as much as the number of guide RNAs) viral vectors, each of which comprises a nucleic acid encoding one guide RNA and a nucleic acid encoding a Cas12f1 protein.

In another embodiment, in a case where the composition comprises a nucleic acid encoding a guide RNA and nucleic acids encoding two or more Cas12f1 proteins (for example, a first Cas12f1 protein and a second Cas12f1 protein), the composition may comprise a first viral vector comprising a nucleic acid encoding a guide RNA, a second viral vector comprising a nucleic acid encoding a first Cas12f1 protein, and a third viral vector comprising a nucleic acid encoding a second Cas12f1 protein. Alternatively, the composition may be in a form of a single viral vector comprising both a nucleic acid encoding a guide RNA and nucleic acids encoding two or more Cas12f1 proteins (for example, a first Cas12f1 protein and a second Cas12f1 protein). Alternatively, the composition may comprise a first viral vector comprising a nucleic acid encoding a guide RNA and a nucleic acid encoding a first Cas12f1 protein, and a second viral vector comprising a nucleic acid encoding a second Cas12f1 protein.

### 1-2) Non-viral vector

The composition may be in a form of a non-viral vector. Here, the guide RNA in the composition may be in a form of RNA or a nucleic acid encoding the same.

The non-viral vector may be a plasmid, a phage, a naked DNA, a DNA complex, an mRNA (transcript) or a PCR amplicon, but is not limited thereto. For example, the plasmid may be selected from the group consisting of: pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19. For example, the phage may be selected from the group consisting of λgt4λB, λ-Charon, λΔz1, and M13.

In an embodiment, the composition may be in a form of a non-viral vector. That is, the composition may comprise a nucleic acid encoding a guide RNA and a nucleic acid encoding a Cas12f1 protein in a form of a non-viral vector. Here, the composition may comprise a first non-viral vector comprising a nucleic acid encoding a guide RNA and a second non-viral vector comprising a nucleic acid encoding a Cas12f1 protein. Alternatively, the composition may comprise a non-viral vector comprising both a nucleic acid encoding a guide RNA and a nucleic acid encoding a Cas12f1 protein. In an embodiment, the composition may comprise a plasmid comprising both a nucleic acid encoding a guide RNA and a nucleic acid encoding a Cas12f1 protein. In another embodiment, the composition may comprise a PCR amplicon comprising a nucleic acid encoding a guide RNA and an mRNA encoding a Cas12f1 protein. In another embodiment, the composition may comprise a PCR amplicon comprising a nucleic acid encoding a guide RNA and a naked DNA comprising a nucleic acid encoding a Cas12f1 protein.

In another embodiment, the composition may be in a form of a non-viral vector. That is, the composition may comprise nucleic acids encoding a guide RNA and a Cas12f1 protein in a form of a non-viral vector. In an embodiment, the composition may comprise a guide RNA, and an mRNA encoding a Cas12f1 protein. In another embodiment, the composition may comprise a guide RNA, and a plasmid comprising a nucleic acid encoding a Cas12f1 protein.

### 1-3) Mixing of viral and non-viral vectors

The composition may be in a mixed form of a viral vector and a non-viral vector. The description of the viral vector and the non-viral vector is as described above. Here, the guide RNA in the composition may be in a form of RNA or a nucleic acid encoding the same.

In an embodiment, the composition may comprise a viral vector comprising a nucleic acid encoding a guide RNA and a non-viral vector comprising a nucleic acid encoding a Cas12f1 protein. In an embodiment, the composition may comprise an adeno-associated viral vector comprising a nucleic acid encoding a guide RNA and an mRNA encoding a Cas12f1 protein. In another embodiment, the composition may comprise a lentiviral vector comprising a nucleic acid encoding a guide RNA and a plasmid comprising a nucleic acid encoding a Cas12f1 protein.

In another embodiment, the composition may comprise a non-viral vector comprising a nucleic acid encoding a guide RNA and a viral vector comprising a nucleic acid encoding a Cas12f1 protein. In an embodiment, the composition may comprise a PCR amplicon comprising a nucleic acid encoding a guide RNA and an adeno-associated viral vector comprising a nucleic acid encoding a Cas12f1 protein. In another embodiment, the composition may comprise a plasmid comprising a nucleic acid encoding a guide RNA and a lentiviral vector comprising a nucleic acid encoding a Cas12f1 protein.

In yet another embodiment, the composition may comprise a guide RNA, and a viral vector comprising a nucleic acid encoding a Cas12f1 protein. In an embodiment, the composition may comprise a guide RNA, and an adeno-associated viral vector comprising a nucleic acid encoding a Cas12f1 protein.

In another embodiment, the composition may comprise a guide RNA, and a non-viral vector comprising a nucleic acid encoding a Cas12f1 protein. In an embodiment, the composition may comprise a guide RNA, and an mRNA encoding a Cas12f1 protein. In another embodiment, the composition may comprise a guide RNA, and a plasmid comprising a nucleic acid encoding a Cas12f1 protein.

### 1-4) Others - additional component of vector

The vectors described above may optionally further comprise a regulatory/control element, a promoter and/or an additionally expressed element.

### Regulating/control element

The vector may optionally comprise a regulatory/control element. Here, the regulatory/control element may be operably linked to a sequence encoding each component included in the vector (that is, a nucleic acid encoding a guide RNA and/or a nucleic acid encoding a Cas12f1 protein). The regulatory/control element may be, but is not limited to, an enhancer, an intron, a termination signal, a polyadenylation signal, a Kozak consensus sequence, an internal ribosome entry site (IRES), a splice acceptor, a 2A sequence and/or an origin of replication. Here, the origin of replication may be, but is not limited to, an f1 origin of replication, an SV40 origin of replication, a pMB1 origin of replication, an adeno origin of replication, an AAV origin of replication, and/or a BBV origin of replication.

### Promoter

The vector may optionally comprise a promoter. Here, the promoter may be operably linked to a sequence encoding each component included in the vector (that is, a nucleic acid encoding a guide RNA and/or a nucleic acid encoding a Cas12f1 protein). The promoter is not limited as long as it is capable of properly expressing a sequence encoding each component included in the vector (that is, a nucleic acid encoding a guide RNA and/or a nucleic acid encoding a Cas12f1 protein). In an embodiment, the promoter sequence may be a promoter that promotes transcription of an RNA polymerase (for example, Pol I, Pol II, or Pol III). For example, the promoter may be, but is not limited to, any one selected from: an SV40 early promoter, a mouse mammary tumor virus long terminal repeat (LTR) promoter, an adenovirus major late promoter (Ad MLP), a herpes simplex virus (HSV) promoter, a cytomegalovirus (CMV) promoter such as CMV immediate early promoter region (CMVIE), a chicken b-actin (CBA) promoter, a rous sarcoma virus (RSV) promoter, a human U6 small nuclear promoter (U6) (Miyagishi et al., Nature Biotechnology 20, 497 - 500 (2002)), an enhanced U6 promoter (for example, Xia et al., Nucleic Acids Res.2003 Sep 1:31(17)), a human H1 promoter (H1), and a 7SK promoter.

### Additionally expressed element

The vector may optionally comprise an additionally expressed element. The vector may comprise a nucleic acid sequence encoding an additionally expressed element to be expressed as necessary by those skilled in the art in addition to a nucleic acid encoding a guide RNA and/or a nucleic acid encoding a Cas12f1 protein. For example, the additionally expressed element may be one of the tags described in the section "Tag" in definition of terms as described above, but is not limited thereto. For example, the additionally expressed element may be a herbicide resistance gene such as glyphosate, glufosinate ammonium, or phosphinothricin; or an antibiotic resistance gene such as ampicillin, kanamycin, G418, bleomycin, hygromycin, or chloramphenicol, but is not limited thereto.

### 2. Mixed form of nucleic acid and protein

The composition may be in a mixed form of a nucleic acid and a protein. Here, the nucleic acid is as described above in the section "1) Form of nucleic acid." Here, the composition comprises a Cas12f1 protein. Here, the composition may comprise two Cas12f1 proteins, and the two Cas12f1 proteins may be Cas12f1 proteins having the same amino acid sequence or Cas12f1 proteins having different amino acid sequences.

In an embodiment, the composition may comprise a viral vector comprising a nucleic acid encoding a guide RNA and a Cas12f1 protein. In an embodiment, the composition may comprise an adeno-associated viral vector comprising a nucleic acid encoding a guide RNA and a Cas12f1 protein. In another embodiment, the composition may comprise an adeno-associated viral vector comprising nucleic acids encoding a plurality of guide RNAs and a Cas12f1 protein.

In another embodiment, the composition may comprise a non-viral vector comprising a nucleic acid encoding a guide RNA and a Cas12f1 protein. In an embodiment, the composition may comprise a plasmid comprising a nucleic acid encoding a guide RNA and a Cas12f1 protein. In another embodiment, the composition may comprise a PCR amplicon comprising a nucleic acid encoding a guide RNA and a Cas12f1 protein. In yet another embodiment, the composition may comprise a PCR amplicon comprising nucleic acids encoding a plurality of guide RNAs and a Cas 12f1 protein.

In yet another embodiment, the composition may comprise a guide RNA and a Cas12f1 protein. Here, the composition may be in a form of an RNP which is in a form of a CRISPR/Cas12f1 complex. In a case where a composition comprises a plurality of guide RNAs and Cas12f1 proteins, the composition may comprise a plurality of CRISPR/Cas12f1 complexes, and the plurality of CRISPR/Cas12f1 complexes each may recognize a different target sequence.

### 3. Examples (1) of composition

In an embodiment, the composition may comprise:
two or more guide RNAs targeting the CEP290 gene or nucleic acids encoding the same; and
a Cas12f1 protein or a nucleic acid encoding the same.

Here, the guide RNA may complementarily bind to a target sequence present in the CEP290 gene.

The two or more guide RNAs may comprise a first guide RNA and a second guide RNA. Here, the nucleic acids encoding the two or more guide RNAs may comprise a nucleic acid encoding the first guide RNA and a nucleic acid encoding the second guide RNA.

Here, the first guide RNA and the second guide RNA may target different target sequences present in the CEP gene.

Here, the first guide RNA may target an upstream region based on the IVS26 mutation (c.2991 + 1655A>G) in the intron 26 region of the CEP gene. Alternatively, the first guide RNA may target a region between the 3' end of exon 26 of the CEP290 gene and the IVS26 mutation (c.2991 + 1655A>G).

Here, the second guide RNA may target a downstream region based on the IVS26 mutation (c.2991 + 1655A>G) in the intron 26 region of the CEP gene. Alternatively, the second guide RNA may target a region between the IVS26 mutation (c.2991 + 1655A>G) and the 5' end of exon 27 of the CEP290 gene.

Here, the first guide RNA may comprise a first guide region and a first scaffold region. Here, the first guide region may have a first guide sequence. Here, the first guide sequence may be a sequence of 15 to 40 nucleotides capable of forming a complementary bond with a target sequence present in an upstream region based on the IVS26 mutation (c.2991 + 1655A>G) in the intron 26 region of the CEP290 gene. Alternatively, the first guide sequence may be a sequence of 15 to 40 nucleotides capable of forming a complementary bond with a target sequence present in a region between the 3' end of exon 26 of the CEP290 gene and the IVS26 mutation (c.2991 + 1655A>G). Here, the complementary bond may optionally contain 0 to 5 mismatched bonds. As an example, the first guide sequence may be any one sequence selected from the guide sequences listed in Table 3. Here, the first scaffold region may comprise a crRNA scaffold sequence and a tracrRNA scaffold sequence. The first scaffold region may optionally further comprise a linker, and the linker may be located between the tracrRNA scaffold sequence and the crRNA scaffold sequence. Here, the crRNA scaffold sequence may be a wild-type crRNA scaffold sequence or an engineered crRNA scaffold sequence. As an example, the wild-type crRNA scaffold sequence may be SEQ ID NO: 101. As another example, the engineered crRNA scaffold sequence is an artificially modified sequence of SEQ ID NO: 101, and may be any one selected from the examples described in the section "i) crRNA scaffold sequence" described above. Here, the tracrRNA scaffold sequence may be a wild-type tracrRNA scaffold sequence or an engineered tracrRNA scaffold sequence. As an example, the wild-type tracrRNA scaffold sequence may be SEQ ID NO: 110. As another example, the engineered tracrRNA scaffold sequence is an artificially modified sequence of SEQ ID NO: 110, and may be any one selected from the examples described in the section "ii) tracrRNA scaffold sequence" described above. As an example, the first scaffold region may be any one selected from the examples described in the section "iv) Examples of scaffold region" described above.

Here, the first guide RNA may optionally further comprise a U-rich tail sequence. The U-rich tail sequence may be located at the 3' end of the first guide region. The U-rich tail sequence may be 5'-(UₐN)_{d}Uₑ-3', 5'-UₐVUₐVUₑ-3', or 5'-UₐVUₐVUₐVUₑ-3'. Here, N may be A, C, G or U, and each V may be independently A, C or G. Here, a may be an integer of 0 to 4, d may be an integer of 0 to 3, and e may be an integer of 1 to 10. As an example, the U-rich tail sequence may be any one selected from the examples described in the section "2-3) U-rich tail sequence" described above.

Here, the second guide RNA may comprise a second guide region and a second scaffold region. Here, the second guide region may have a second guide sequence. Here, the second guide sequence may be a sequence of 15 to 40 nucleotides capable of forming a complementary bond with a target sequence present in a downstream region based on the IVS26 mutation (c.2991 + 1655A>G) in the intron 26 region of the CEP290 gene. Alternatively, the second guide sequence may be a sequence of 15 to 40 nucleotides capable of forming a complementary bond with a target sequence present in a region between the IVS26 mutation (c.2991 + 1655A>G) and the 5' end of exon 27 of the CEP290 gene. Here, the complementary bond may optionally contain 0 to 5 mismatched bonds. As an example, the second guide sequence may be any one sequence selected from the guide sequences listed in Table 4. Here, the second scaffold region may comprise a crRNA scaffold sequence and a tracrRNA scaffold sequence. The second scaffold region may optionally further comprise a linker, and the linker may be located between the tracrRNA scaffold sequence and the crRNA scaffold sequence. Here, the crRNA scaffold sequence may be a wild-type crRNA scaffold sequence or an engineered crRNA scaffold sequence. As an example, the wild-type crRNA scaffold sequence may be SEQ ID NO: 101. As another example, the engineered crRNA scaffold sequence is an artificially modified sequence of SEQ ID NO: 101, and may be any one selected from the examples described in the section "i) crRNA scaffold sequence" described above. Here, the tracrRNA scaffold sequence may be a wild-type tracrRNA scaffold sequence or an engineered tracrRNA scaffold sequence. As an example, the wild-type tracrRNA scaffold sequence may be SEQ ID NO: 110. As another example, the engineered tracrRNA scaffold sequence is an artificially modified sequence of SEQ ID NO: 110, and may be any one selected from the examples described in the section "ii) tracrRNA scaffold sequence" described above. As an example, the second scaffold region may be any one selected from the examples described in the section "iv) Examples of scaffold region" described above.

Here, the second guide RNA may optionally further comprise a U-rich tail sequence. The U-rich tail sequence may be located at the 3' end of the second guide region. The U-rich tail sequence may be 5'-(UₐN)_{d}Uₑ-3', 5'-UₐVUₐVUₑ-3', or 5'-UₐVUₐVUₐVUₑ-3'. Here, N may be A, C, G or U, and each V may be independently A, C or G. Here, a may be an integer of 0 to 4, d may be an integer of 0 to 3, and e may be an integer of 1 to 10. As an example, the U-rich tail sequence may be any one selected from the examples described in the section "2-3) U-rich tail sequence" described above.

The Cas12f1 protein may be a wild-type Cas12f1 protein or a Cas12f1 variant. Here, the Cas12f1 protein is as described in the section "3. Cas12f1 (Cas14a1) protein" described above, and has the same characteristics and structure as each configuration described in the section.

The composition may optionally further comprise at least one guide RNA or a nucleic acid encoding the same. Here, the further included guide RNA may recognize a target sequence different from those recognized by the first guide RNA and the second guide RNA.

The composition may optionally further comprise at least one Cas12f1 (Cas14a1) protein or a nucleic acid encoding the same. Here, the composition may comprise two or more Cas12f1 (Cas14a1) proteins having different amino acid sequences. Here, the two or more Cas12f1 (Cas14a1) proteins having different amino acid sequences may be a wild-type Cas12f1 (Cas14a1) protein and a Cas12f1 variant.

The composition may be in a form of a nucleic acid. Here, the composition may comprise nucleic acids encoding two or more guide RNAs targeting the CEP290 gene and a nucleic acid encoding a Cas12f1 protein in one vector. Alternatively, the composition may comprise nucleic acids encoding two or more guide RNAs targeting the CEP290 gene and a nucleic acid encoding a Cas12f1 protein in separate vectors. Here, the vector may be a plasmid, an mRNA (transcript), a PCR amplicon, or a viral vector. Here, the viral vector may be at least one viral vector selected from the group consisting of a retroviral (retrovirus) vector, a lentiviral (lentivirus) vector, an adenoviral (adenovirus vector), an adeno-associated viral vector (adeno-associated virus (AAV) vector), a vaccinia viral (vaccinia virus) vector, a poxviral (poxvirus) vector, and a herpes simplex viral (herpes simplex virus) vector. Here, the vector may further comprise a promoter for a nucleic acid encoding the guide RNA and a promoter for a nucleic acid encoding the Cas12f1 protein. Here, the promoter may be a U6 promoter, an H1 promoter, a 7SK promoter, a CMV promoter, a CBA promoter, an LTR promoter, an Ad MLP promoter, an HSV promoter, an SV40 promoter, or an RSV promoter. As an example, the composition may comprise a first vector comprising a nucleic acid encoding the first guide RNA and a nucleic acid encoding the second guide RNA, and a second vector comprising a nucleic acid encoding the Cas12f1 protein. Here, each of the first vector and the second vector may be a viral vector. Here, the viral vector may be an adeno-associated virus (AAV) vector. Here, the first vector may comprise a promoter for each of the nucleic acid encoding the first guide RNA and the nucleic acid encoding the second guide RNA, and each promoter may be a U6 promoter, an H1 promoter, or a 7SK promoter. Here, the second vector may comprise a promoter for the nucleic acid encoding the Cas12f1 protein, and the promoter may be a CMV promoter, a CBA promoter, an LTR promoter, an Ad MLP promoter, an HSV promoter, an SV40 promoter, or an RSV promoter. As another example, the composition may comprise a vector that contains a nucleic acid encoding the first guide RNA, a nucleic acid encoding the second guide RNA, and a nucleic acid encoding the Cas12f1 protein. Here, the vector may be a viral vector. Here, the viral vector may be an adeno-associated virus (AAV) vector. Here, the vector may comprise a promoter for each of the nucleic acid encoding the first guide RNA, the nucleic acid encoding the second guide RNA, and the nucleic acid encoding the Cas12f1 protein. Here, each promoter may be a U6 promoter, an H1 promoter, a 7SK promoter, a CMV promoter, a CBA promoter, an LTR promoter, an Ad MLP promoter, an HSV promoter, an SV40 promoter, or an RSV promoter.

The composition may be in a mixed form of a nucleic acid and a protein. Here, the composition may comprise a CRISPR/Cas12f1 complex in which the guide RNA and the Cas12f1 protein are bound to each other. As an example, the composition may comprise a first CRISPR/Cas12f1 complex in which the first guide RNA and the Cas12f1 protein are bound to each other, and a second CRISPR/Cas12f1 complex in which the second guide RNA and the Cas12f1 protein are bound to each other.

### 4. Examples (2) of composition

In an embodiment, the composition may comprise:
at least one guide RNA targeting the CEP290 gene or a nucleic acid encoding the same; and
a Cas12f1 protein or a nucleic acid encoding the same.

Here, the guide RNA may complementarily bind to a target sequence present in the CEP290 gene.

Here, the guide RNA may target a partial region of the intron 26 region containing the IVS26 mutation (c.2991 + 1655A>G) of the CEP gene.

Here, the guide RNA may comprise a guide region and a scaffold region. Here, the guide region may have a guide sequence. Here, the guide sequence may be a sequence of 15 to 40 nucleotides capable of forming a complementary bond with a target sequence present in a partial region of the intron 26 region containing the IVS26 mutation (c.2991 + 1655A>G) of the CEP gene. Here, the complementary bond may optionally contain 0 to 5 mismatched bonds. As an example, the guide sequence may be any one sequence selected from the guide sequences described in Tables 3 and 4. Here, the scaffold region may comprise a crRNA scaffold sequence and a tracrRNA scaffold sequence. The scaffold region may optionally further comprise a linker, and the linker may be located between the tracrRNA scaffold sequence and the crRNA scaffold sequence. Here, the crRNA scaffold sequence may be a wild-type crRNA scaffold sequence or an engineered crRNA scaffold sequence. As an example, the wild-type crRNA scaffold sequence may be SEQ ID NO: 101. As another example, the engineered crRNA scaffold sequence is an artificially modified sequence of SEQ ID NO: 101, and may be any one selected from the examples described in the section "i) crRNA scaffold sequence" described above. Here, the tracrRNA scaffold sequence may be a wild-type tracrRNA scaffold sequence or an engineered tracrRNA scaffold sequence. As an example, the wild-type tracrRNA scaffold sequence may be SEQ ID NO: 110. As another example, the engineered tracrRNA scaffold sequence is an artificially modified sequence of SEQ ID NO: 110, and may be any one selected from the examples described in the section "ii) tracrRNA scaffold sequence" described above. As an example, the first scaffold region may be any one selected from the examples described in the section "iv) Examples of scaffold region" described above.

Here, the guide RNA may optionally further comprise a U-rich tail sequence. The U-rich tail sequence may be located at the 3' end of the guide region. The U-rich tail sequence may be 5'-(UₐN)_{d}Uₑ-3', 5'-UₐVUₐVUₑ-3', or 5'-UₐVUₐVUₐVUₑ-3'. Here, N may be A, C, G or U, and each V may be independently A, C or G. Here, a may be an integer of 0 to 4, d may be an integer of 0 to 3, and e may be an integer of 1 to 10. As an example, the U-rich tail sequence may be any one selected from the examples described in the section "2-3) U-rich tail sequence" described above.

The Cas12f1 protein may be a wild-type Cas12f1 protein or a Cas12f1 variant. Here, the Cas12f1 protein is as described in the section "3. Cas12f1 (Cas14a1) protein" described above, and has the same characteristics and structure as each configuration described in the section.

The composition may optionally further comprise at least one guide RNA or a nucleic acid encoding the same. Here, the further included guide RNA may recognize a target sequence different from that recognized by the guide RNA.

The composition may optionally further comprise at least one Cas12f1 (Cas14a1) protein or a nucleic acid encoding the same. Here, the composition may comprise two or more Cas12f1 (Cas14a1) proteins having different amino acid sequences. Here, the two or more Cas12f1 (Cas14a1) proteins having different amino acid sequences may be a wild-type Cas12f1 (Cas14a1) protein and a Cas12f1 variant.

The composition may be in a form of a nucleic acid. Here, the composition may comprise a nucleic acid encoding at least one guide RNA targeting the CEP290 gene and a nucleic acid encoding a Cas12f1 protein in one vector. Alternatively, the composition may comprise a nucleic acid encoding at least one guide RNA targeting the CEP290 gene and a nucleic acid encoding a Cas12f1 protein in separate vectors. Here, the vector may be a plasmid, an mRNA (transcript), a PCR amplicon, or a viral vector. Here, the viral vector may be at least one viral vector selected from the group consisting of a retroviral (retrovirus) vector, a lentiviral (lentivirus) vector, an adenoviral (adenovirus vector), an adeno-associated viral vector (adeno-associated virus (AAV) vector), a vaccinia viral (vaccinia virus) vector, a poxviral (poxvirus) vector, and a herpes simplex viral (herpes simplex virus) vector. Here, the vector may further comprise a promoter for a nucleic acid encoding the guide RNA and a promoter for a nucleic acid encoding the Cas12f1 protein. Here, the promoter may be a U6 promoter, an H1 promoter, a 7SK promoter, a CMV promoter, a CBA promoter, an LTR promoter, an Ad MLP promoter, an HSV promoter, an SV40 promoter, or an RSV promoter.

The composition may be in a mixed form of a nucleic acid and a protein. Here, the composition may comprise a CRISPR/Cas12f1 complex in which the guide RNA and the Cas12f1 protein are bound to each other.

### <Method for treating LCA10 disease>

In an aspect disclosed by the present specification, there is provided a method for treating LCA10 disease. The method for treating LCA10 disease uses a CRISPR/Cas12f1 system. More specifically, the method relates to a method of introducing (or delivering) a composition comprising the CRISPR/Cas12f1 (Cas14a1) system to a subject and/or a target cell, thereby modifying the CEP290 gene that is a target gene present in the subject and/or target cell. Here, the subject is an animal or some tissue thereof, and the animal may be a human or a non-human animal. Here, the target cells may be non-human animal cells or human cells. The method may be performed in vitro, ex vivo or in vivo. Here, the in vivo may be in a human body or in a non-human animal body.

The method may be a method that comprises introducing (or delivering, applying, injecting, or administering) the CRISPR/Cas12f1 (Cas14a1) system into a subject and/or a target cell. The CRISPR/Cas12f1 system is as described in the section "<CRISPR/Cas12f1 system for treatment of LCA10 disease>" described above, and has the same characteristics and structure as each configuration described in the section.

The method induces deletion or inversion of a partial nucleic acid sequence in the intron 26 region of the CEP290 gene using the CRISPR/Cas12f1 system. Here, the partial nucleic acid sequence contains the IVS26 mutation (c.2991 + 1655A>G). This allows removal of all or part of the 128 base pair cryptic exon that is transcribed by the CEP290 gene. Therefore, the method effectively removes all or part of the cryptic exon that causes LCA10 disease, and thus may be used as an effective treatment method for LCA10 disease.

Hereinafter, embodiments of the method will be described in detail.

### 1. Examples of method

### 1-1) Example (1) of method

In an embodiment, the method may be a method of artificially modifying the CEP290 gene in a cell. Here, the method may comprise treating a cell with a composition or introducing a composition into a cell.

Here, the composition may comprise:
at least one guide RNA targeting the CEP290 gene or a nucleic acid encoding the same; and
a Cas12f1 protein or a nucleic acid encoding the same.

Alternatively, the composition may comprise:
two or more guide RNAs targeting the CEP290 gene or nucleic acids encoding the same; and
a Cas12f1 protein or a nucleic acid encoding the same. Here, each of the two or more guide RNAs may target a different target sequence present in the CEP290 gene.

The composition is as described in the section "<Composition for treatment of LCA10 disease>" described above, and has the same characteristics and structure as each configuration described in the section. In addition, the guide RNA is as described in the section "2. Guide RNA" of <CRISPR/Cas12f1 system for treatment of LCA10 disease> described above, and has the same characteristics and structure as each configuration described in the section. In addition, the Cas12f1 protein is as described in the section "3. Cas12f1 (Cas14a1) protein" in <CRISPR/Cas12f1 system for treatment of LCA10 disease>, and has the same characteristics and structure as each configuration described in the section.

The composition may be a viral vector or a non-viral vector, or in a mixed form thereof. Alternatively, the composition may be in a mixed form of a nucleic acid and a protein. The viral vector, the non-viral vector, or the mixed form thereof is as described in the section "1. Form of nucleic acid" in <Composition for treatment of LCA10 disease> described above, and has the same characteristics and structure as each configuration described in the section. In addition, the mixed form of a nucleic acid and a protein is as described in the section "2. Mixed form of nucleic acid and protein" in <Composition for treatment of LCA10 disease> described above, and has the same characteristics and structure as each configuration described in the section.

In an embodiment, the composition may be in a form of a viral vector comprising nucleic acids encoding two or more guide RNAs and a nucleic acid encoding a Cas12f1 protein. Here, the viral vector may be one or more selected from the group consisting of a retroviral vector, a lentiviral vector, an adenoviral vector, an adeno-associated viral vector, a vaccinia virus vector, a poxvirus vector, and a herpes simplex virus vector. Here, the composition may comprise one viral vector comprising both the nucleic acids encoding two or more guide RNAs and the nucleic acid encoding the Cas12f1 protein. Alternatively, the composition may comprise two viral vectors which respectively comprise the nucleic acids encoding two or more guide RNAs and the nucleic acid encoding the Cas12f1 protein.

In another embodiment, the composition may be in a form of a non-viral vector comprising a nucleic acid encoding a guide RNA and a nucleic acid encoding a Cas12f1 protein. Here, the non-viral vector may be a plasmid. Here, the composition may comprise one plasmid comprising both the nucleic acid encoding the guide RNA and the nucleic acid encoding the Cas12f1 protein. Alternatively, the composition may comprise two plasmids which respectively comprise the nucleic acid encoding the guide RNA and the nucleic acid encoding the Cas12f1 protein.

In yet another embodiment, the composition may comprise a PCR amplicon comprising the nucleic acid encoding the guide RNA and an mRNA encoding the Cas12f1 protein.

In still yet another embodiment, the composition may comprise a PCR amplicon comprising the nucleic acid encoding the guide RNA and the Cas12f1 protein. Alternatively, the composition may comprise a CRISPR/Cas12f1 (Cas14a1) complex in which the guide RNA and the Cas12f1 protein are bound to each other.

The cell may be a cell containing the CEP290 gene. The cell may be a non-human animal cell or human cell.

The cells may be cells obtained from a subject with LCA10 disease. Here, the subject may be a human or a non-human animal. Here, the non-human animal may be a non-human animal susceptible to LCA10 disease.

Treating the cell with the composition or introducing the composition into the cell may be performed to introduce the CRISPR/Cas12f1 (Cas14a1) system into the cell. Alternatively, treating the cell with the composition or introducing the composition into the cell may be performed to bring the CRISPR/Cas12f1 (Cas14a1) system in contact with the CEP290 gene present in the cell. Treating the cell with the composition may be performed using electroporation, gene gun, sonoporation, magnetofection, nanoparticles, and/or transient cell compression or squeezing. Alternatively, treating the eukaryotic cell with the composition may be performed using cationic liposome, lithium acetate-DMSO, lipid-mediated transfection, calcium phosphate precipitation, lipofection, polyethyleneimine (PEI)-mediated transfection, DEAE-dextran-mediated transfection, and/or nanoparticle-mediated nucleic acid delivery (see Panyam et al., Adv Drug Deliv Rev. 2012 Sep 13. pii: S0169-409X(12)00283-9. doi: 10.1016/j.addr.2012.09.023), but the treating method is not limited thereto.

As a result of treatment of the cell with the composition or introduction of the composition into the cell, deletion or inversion of a partial nucleic acid sequence in the intron 26 region of the CEP290 gene may be induced. Here, the partial nucleic acid sequence may contain the IVS26 mutation (c.2991 + 1655A>G). This allows removal or inversion of all or part of the 128 base pair cryptic exon that is transcribed by the CEP290 gene. Alternatively, as a result of treatment of the cell with the composition or introduction of the composition into the cell, modification caused by non-homologous end joining (NHEJ) may occur in a partial nucleic acid sequence in the intron 26 region of the CEP290 gene. Here, the partial nucleic acid sequence may contain the IVS26 mutation (c.2991 + 1655A>G). Here, the modification caused by NHEJ may be deletion, insertion, or indel of one or more nucleotides.

### 2-2) Example (2) of method

In another embodiment, the method is a method for treating LCA10 disease, and may comprise introducing or administering a composition to a subject.

Here, the composition may comprise:
at least one guide RNA targeting the CEP290 gene or a nucleic acid encoding the same; and
a Cas12f1 protein or a nucleic acid encoding the same.

Alternatively, the composition may comprise:
two or more guide RNAs targeting the CEP290 gene or nucleic acids encoding the same; and
a Cas12f1 protein or a nucleic acid encoding the same. Here, each of the two or more guide RNAs may target a different target sequence present in the CEP gene.

The composition is as described in the section "<Composition for treatment of LCA10 disease>" described above, and has the same characteristics and structure as each configuration described in the section. In addition, the guide RNA is as described in the section "2. Guide RNA" of <CRISPR/Cas12f1 system for treatment of LCA10 disease> described above, and has the same characteristics and structure as each configuration described in the section. In addition, the Cas12f1 protein is as described in the section "3. Cas12f1 (Cas14a1) protein" in <CRISPR/Cas12f1 system for treatment of LCA10 disease>, and has the same characteristics and structure as each configuration described in the section.

The composition may be a viral vector or a non-viral vector, or in a mixed form thereof. Alternatively, the composition may be in a mixed form of a nucleic acid and a protein. The viral vector, the non-viral vector, or the mixed form thereof is as described in the section "1. Form of nucleic acid" in <Composition for treatment of LCA10 disease> described above, and has the same characteristics and structure as each configuration described in the section. In addition, the mixed form of a nucleic acid and a protein is as described in the section "2. Mixed form of nucleic acid and protein" in <Composition for treatment of LCA10 disease> described above, and has the same characteristics and structure as each configuration described in the section.

In an embodiment, the composition may be in a form of a viral vector comprising nucleic acids encoding two or more guide RNAs and a nucleic acid encoding a Cas12f1 protein. Here, the viral vector may be one or more selected from the group consisting of a retroviral vector, a lentiviral vector, an adenoviral vector, an adeno-associated viral vector, a vaccinia virus vector, a poxvirus vector, and a herpes simplex virus vector. Here, the composition may comprise one viral vector comprising both the nucleic acids encoding two or more guide RNAs and the nucleic acid encoding the Cas12f1 protein. Alternatively, the composition may comprise two viral vectors which respectively comprise the nucleic acids encoding two or more guide RNAs and the nucleic acid encoding the Cas12f1 protein.

In another embodiment, the composition may be in a form of a non-viral vector comprising a nucleic acid encoding a guide RNA and a nucleic acid encoding a Cas12f1 protein. Here, the non-viral vector may be a plasmid. Here, the composition may comprise one plasmid comprising both the nucleic acid encoding the guide RNA and the nucleic acid encoding the Cas12f1 protein. Alternatively, the composition may comprise two plasmids which respectively comprise the nucleic acid encoding the guide RNA and the nucleic acid encoding the Cas12f1 protein.

In yet another embodiment, the composition may comprise a PCR amplicon comprising a nucleic acid encoding a guide RNA and an mRNA encoding a Cas12f1 protein.

In still yet another embodiment, the composition may comprise a PCR amplicon comprising a nucleic acid encoding a guide RNA and a Cas12f1 protein. Alternatively, the composition may comprise a CRISPR/Cas12f1 (Cas14a1) complex in which the guide RNA and the Cas12f1 protein are bound to each other.

The subject may be a human or non-human animal with LCA10 disease. Alternatively, the subject may be a human or non-human animal having the CEP290 gene containing a cryptic exon.

Introducing or administering the composition to the subject may be performed to introduce the CRISPR/Cas12f1 (Cas14a1) system into one or more cells present in the subject. Alternatively, introducing or administering the composition to the subject may be performed to bring the CRISPR/Cas12f1 (Cas14a1) system in contact with the CEP290 gene present in one or more cells present in the subject. Introducing or administering the composition to the subject may be performed using microinjection, cationic liposome, lithium acetate-DMSO, lipid-mediated transfection, calcium phosphate precipitation, lipofection, polyethyleneimine (PEI)-mediated transfection, DEAE-dextran-mediated transfection, and/or nanoparticle-mediated nucleic acid delivery (see Panyam et al., Adv Drug Deliv Rev. 2012 Sep 13. pii: S0169-409X(12)00283-9. doi: 10.1016/j.addr.2012.09.023), but the treating method is not limited thereto. Alternatively, administering the composition to the subject may be performed by injection, transfusion, implantation, or transplantation. Here, administering the composition to the subject may be performed by a route of administration selected from subretinal, subcutaneous, intradermal, intraocular, intravitreal, intratumoral, intranodal, intramedullary, intramuscular, intravenous, intralymphatic, or intraperitoneal.

A single dose of the composition (a pharmaceutically effective amount to obtain a predetermined desired effect) may be appropriately prescribed by taking into account the age, health and body weight of the subject to be administered, the type of concurrent treatment, if any, the treatment frequency, characteristics of a desired effect, and the like.

As a result of introducing or administering the composition to the subject, removal or inversion of a partial nucleic acid sequence in the intron 26 region of the CEP290 gene may be induced. Here, the partial nucleic acid sequence may contain the IVS26 mutation (c.2991 + 1655A>G). This allows removal or inversion of all or part of the 128 base pair cryptic exon that is transcribed by the CEP290 gene. Alternatively, as a result of introducing or administering the composition to the subject, modification caused by non-homologous end joining (NHEJ) may occur in a partial nucleic acid sequence in the intron 26 region of the CEP290 gene in one or more cells present in the subject. Here, the partial nucleic acid sequence may contain the IVS26 mutation (c.2991 + 1655A>G). Here, the modification caused by NHEJ may be deletion, insertion, or indel of one or more nucleotides.

Therefore, the method effectively deletes or inverts all or part of the cryptic exon that causes LCA10 disease, and thus may be used as an effective treatment method for LCA10 disease.

### 2. Therapeutic effect - modification of target gene

The composition and the method disclosed herein allow the CEP290 gene to be modified. More specifically, the CEP290 gene may be modified by the CRISPR/Cas12f1 system. Here, the modification may comprise both i) cleavage or damage of the CEP290 gene caused by the CRISPR/Cas12f1 (Cas14a1) system and ii) repair or recovery of the cleaved (or damaged) CEP290 gene. Here, the modification may be inversion of a partial nucleic acid sequence in intron 26 present in the CEP290 gene, which may result in inversion of all or part of the cryptic exon that is transcribed and generated by the CEP290 gene. Alternatively, the modification may be deletion of a partial nucleic acid sequence in intron 26 present in the CEP290 gene, which may result in deletion of a partial nucleic acid sequence in the cryptic exon that is transcribed and generated by the CEP290 gene. Alternatively, the modification may be insertion of an additional nucleic acid sequence into the intron 26 present in the CEP290 gene, which may result in insertion of an additional nucleic acid sequence into the cryptic exon that is transcribed and generated by the CEP290 gene. Alternatively, the modification may be insertion and deletion (indel) in which a partial nucleic acid sequence in intron 26 present in the CEP290 gene is deleted and an additional sequence is inserted thereinto, which may result in deletion of a partial sequence in the cryptic exon and insertion of an additional sequence thereinto, the cryptic exon being transcribed and generated by the CEP290 gene. Alternatively, the modification may be substitution of at least one nucleic acid sequence present in intron 26 of the CEP290 gene, which may result in modification of a sequence of mRNA transcribed by the CEP290 gene. Here, the partial nucleic acid sequence in intron 26 may contain the IVS26 mutation (c.2991 + 1655A>G). Here, at least one nucleic acid sequence present in intron 26 may contain the IVS26 mutation (c.2991 + 1655A>G) sequence. Hereinafter, the modification will be described in detail.

### 2-1) Deletion

As a result of performing the method provided herein, the modification may be removal (or deletion) of all or part of the nucleic acid sequence of the intron 26 present in the CEP290 gene. The modification may mean deleting a partial nucleotide sequence in intron 26 present in the CEP290 gene. In an embodiment, the method comprises introducing, into a cell containing the CEP290 gene or a subject having the cell, a Cas12f1 protein or a nucleic acid encoding the same, a first engineered guide RNA or a nucleic acid encoding the same, and a second engineered guide RNA or a nucleic acid encoding the same. This results in removal of a specific sequence in intron 26 present in the CEP290 gene. Here, the specific sequence may be a nucleic acid sequence containing the IVS26 mutation (c.2991 + 1655A>G).

### 2-2) Inversion

As a result of performing the method provided herein, the modification may be inversion of all or part of intron 26 present in the CEP290 gene. The inversion may be a change in the direction of transcription of all or a partial region of intron 26 present in the CEP290 gene. That is, the inversion may mean that all or a partial region of intron 26 in the CEP290 gene is recombined and reversed, indicating that all or a partial region of intron 26 is reversed from one end to the other. In an embodiment, the method comprises introducing, into a cell containing the CEP290 gene or a subject having the cell, a Cas12f1 protein or a nucleic acid encoding the same, a first engineered guide RNA or a nucleic acid encoding the same, and a second engineered guide RNA or a nucleic acid encoding the same. This results in inversion of all or a partial region of intron 26 present in the CEP290 gene. Here, the partial region may be a region containing the IVS26 mutation (c.2991 + 1655A>G).

### 2-3) Insertion and deletion (or indel)

As a result of performing the method provided herein, deletion and insertion (indel) may occur in intron 26 of the CEP290 gene. The indel may be caused by non-homologous end joining (NHEJ). In general, NHEJ is a method of repairing or recovering a break (for example, cleavage) in a double-strand in DNA in which two compatible ends formed by the break repeatedly contact each other. Repair of a damaged gene or nucleic acid using NHEJ results in partial insertion and/or deletion (indel) of a nucleic acid sequence at the NHEJ repair site. This insertion and/or deletion alters its reading frame and produces a frameshifted transcript mRNA, which, in turn, undergoes nonsense-mediated decay or fails to synthesize a normal protein, thereby losing its original function. Therefore, when indel occurs in intron 26 of the CEP290 gene, the cryptic exon, which is transcribed and generated by the CEP290 gene, may be inactivated. In an embodiment, as a result of performing the above method, deletion and/or addition of one or more bases may occur in intron 26 of the CEP290 gene.

### 2-4) Substitution or base editing

As a result of performing the method provided herein, base editing may occur in the intron 26 region containing the point mutation (c.2991 + 1655A>G) of the CEP290 gene. Unlike indel in which deletion or addition of any nucleotide occurs in a target gene or target nucleic acid, the base editing means altering one or more specific bases in a nucleic acid as intended. In other words, the base editing causes a pre-intended point mutation at a specific position in a target gene or target nucleic acid. In an embodiment, as a result of performing the method, the point mutation (c.2991 + 1655A>G) in the CEP290 gene may undergo substitution with A again.

### 2-5) Insertion

As a result of performing the method provided herein, knockin may occur in the CEP290 gene. The knockin means insertion of an additional nucleic acid sequence into the CEP290 gene. To cause the knockin, a donor comprising the additional nucleic acid sequence is further required in addition to a CRISPR/Cas12f1 (Cas14a1) system. When the CRISPR/Cas12f1 (Cas14a1) system cleaves the CEP290 gene in a cell, repair of the cleaved CEP290 gene occurs. Here, the repair may be performed using homology directed repairing (HDR), wherein the donor participates in the repair process so that the additional nucleic acid sequence is inserted into the CEP290 gene. In an embodiment, the method may further comprise delivering a donor into a cell. Here, the donor comprises an additional nucleic acid of interest, and induces insertion of the additional nucleic acid into the CEP290 gene. Here, when the donor is delivered into the cell, one of the above-described treating methods for the CRISPR/Cas12f1 (Cas14a1) system may be used.

### 2-6) Phenotype by modification of target gene

Modification of the CEP290 gene may result in effects of knockout, knockdown, or knockin. The "knockout" means inactivation of the CEP290 gene, and the "inactivation of the CEP290 gene" means a state in which transcription and/or translation of the CEP290 gene does not occur. The knockout may suppress transcription and translation of a gene, which causes a disease or has an abnormal function, thereby preventing expression of a protein. The "knockdown" means decreasing transcription and/or translation of the CEP290 gene, or decreasing expression of the CEP290 protein. The knockdown may regulate expression of a gene or protein to be overexpressed, thereby preventing occurrence of or treating a disease. The "knockin" means inserting a specific nucleic acid or gene into the CEP290 gene. Here, the "specific nucleic acid or gene" means a gene or nucleic acid to be inserted or expressed. The knockin may be used to treat a disease by correcting a mutant gene, which causes a disease, or inserting a normal gene so that expression of the normal gene is induced.

In an embodiment, when the CEP290 gene is modified by the method, the cell may have suppressed or decreased transcription and/or translation of the CEP290 gene containing the IVS26 mutation (c.2991 + 1655A>G). Alternatively, the cell may have increased transcription and/or translation of the normal CEP290 gene.

In another embodiment, when the CEP290 gene is modified by the method, the subject may have suppressed or decreased transcription and/or translation of the CEP290 gene containing the IVS26 mutation (c.2991 + 1655A>G). Alternatively, the subject may have increased transcription and/or translation of the normal CEP290 gene.

### <Factor for decreasing non-homologous end joining DNA repair pathway (NHEJ) activity >

The composition for treatment of LCA10 disease provided herein may further comprise a factor capable of decreasing non-homologous end joining DNA repair pathway (NHEJ) activity. In addition, the method for treating LCA10 disease provided herein may be performed using the CRISPR/Cas12f1 system together with the factor for decreasing NHEJ activity.

The factor for decreasing NHEJ activity acts in a cell and serves to decrease non-homologous end-joining activity for cleavage of double-stranded DNA.

The non-homologous end-joining activity may be, for example, canonical NHEJ pathway (Kasparek & Humphrey (2011) Seminars in Cell & Dev. Biol. 22:886-897,), or alternative NHEJ pathway. Specifically, the NHEJ activity may be canonical NHEJ pathway caused by expression or activity of MRE11, RAD50, NBS1, DNA-PK, CtIP, Ku70, Ku80, Artemis (DCLRE1C), ligase IV (Lig4), PNKP, XRCC4, XLF (XRCC4-like factor), ATM (ATM serine/threonine kinase), CHK1/CHK2, CLF (CURLY LEAF), and/or Pol Mu (POLM), or alternative NHEJ pathway caused by expression or activity of XRCC1, PARP (for example, PARP1), Lig1, and/or Lig3.

The factor for decreasing NHEJ activity may be, for example, a small molecule or inhibitory nucleic acid such as a short interfering nucleic acid (for example, short interfering RNA (siRNA), double-stranded RNA (dsRNA), micro-RNA (miRNA), and short hairpin RNA (shRNA) specific for gene transcript), or an antisense oligonucleotide. The factor for decreasing NHEJ activity functions to decrease or inhibit expression and/or activity of factors involved in the aforementioned canonical NHEJ pathway and/or alternative NHEJ pathway.

In a case where this factor for decreasing NHEJ activity is used together with the composition for treatment of LCA10 disease, intracellular non-homologous end joining activity may be decreased to increase efficiency of the composition for treatment of LCA10 disease for modifying the CEP290 gene.

### Examples

### Experiment methods

### 1. Extraction of gDNA

gDNA Prep was performed using the Genomic DNA Prep Kit (GCBL200, Nanohelix). For cells transfected in a 24-well plate, removal of media was performed. The cells were removed from the bottom with 200 µl of trypsin and placed in a 1.5 ml tube. Centrifugation was performed at 300 × g for 5 minutes and the supernatant was removed. To the resultant were added 300 µl of NGD1 buffer and 2 µl of RNase A (50 mg/ml), and vortexing was performed for 1 minute. 8 µl of Proteinase K (10 mg/ml) was added thereto, and then reaction was allowed to occur at 60°C for 10 minutes. Cooling was performed on ice for 5 minutes. 300 µl of NPS buffer was added thereto and the resultant was mixed well. Then, reaction was allowed to occur on ice for 5 minutes and centrifugation was performed at 12,000 rpm for 5 minutes. Columns were prepared depending on the number of samples. 100 µl of MaxBinder Solution was added thereto, and centrifugation was performed at 12,000 rpm for 30 seconds. The entire supernatant was collected, placed in the column, and centrifuged at 12,000 rpm for 1 minute. Then, the filtered solution was discarded. 500 µl of 80% EtOH was added to the column, centrifugation was performed at 10,000 rpm for 30 seconds, and the filtered solution was discarded. After repeating the procedure twice, centrifugation was performed at 13,000 rpm for 3 minutes. The column was placed in a new 1.5 ml tube, and 30 µl of EB solution was dropped in the center. Then, reaction was allowed to occur for 1 minute, and then centrifugation was performed at 12,000 rpm for 2 minutes. The eluted gDNA was quantified and stored at 4°C.

### 2. PCR & gel purification

This experiment was conducted using the GEL & PCR Purification System (GP104-200, Biofact). To the resulting PCT product was added UB buffer equivalent to 3 times the PCR product's volume, and mixed well. To the mixture was added isopropanol equivalent to 2 times the PCR product's volume, and mixed well. For the gels, the gel of the corresponding band was cut and weighed. Then, UB buffer equivalent to three times the gel's weight was added thereto, and reaction was allowed to occur at 65°C for 10 minutes so that the gel was dissolved. Then, isopropanol was added thereto in an amount equivalent to 1 time the gel's volume and mixed well. Columns were prepared and 200 µl of HelpB buffer was added to each column. Then, centrifugation was performed at 13,000 rpm for 30 seconds, and then the filtered solution was discarded. The reaction solution was placed in the column, centrifugation was performed at 7,000 rpm for 1 minute, and then the filtered solution was discarded. To the resultant was added 750 µl of 80% EtOH and centrifugation was performed at 13,000 rpm for 30 seconds. Then, the filtered solution was discarded. After repeating the procedure twice, centrifugation was performed at 13,000 rpm for 3 minutes. After the centrifugation was completed, the column was placed in a 1.5 ml tube, 30 µl of EB buffer was dropped in the center, and reaction was allowed to occur at room temperature for 1 minute. Centrifugation was performed at 13,000 rpm for 1 minute. The DNA collected in the 1.5 ml tube was quantified, and then stored at 4°C.

### 3. Collection of plasmid vector

Vectors transformed into DH5a were used for transfection or produced for Sanger sequencing. The Plasmid Mini prep kit (PM105-200, Biofact) was used and the procedure was performed according to the manufacturer's manual. The vector-transformed DH5a culture was placed in a 1.5 ml tube, and then centrifugation was performed at 13,000 rpm for 5 minutes. After the centrifugation, the supernatant was discarded, and the pellet was sufficiently loosened by vortexing. To the resultant was added 350 µl of B1 buffer, and then the tube was shaken to allow sufficient reaction. 350 µl of A1 buffer containing RNase A was added thereto and the tube was inverted until the blue color disappeared. Then, centrifugation was performed at 13,000 rpm for 5 minutes. Columns were prepared, and 200 µl of HelpB buffer was added to each column. Then, centrifugation was performed at 13,000 rpm for 30 seconds, and the filtered solution was discarded. 750 µl of the supernatant was added to the column, centrifugation was performed at 7,000 rpm for 1 minute, and the filtered solution was discarded. To the resultant was added 750 µl of 80% EtOH and centrifugation was performed at 13,000 rpm for 30 seconds. Then, the filtered solution was discarded. After repeating the procedure twice, centrifugation was performed at 13,000 rpm for 3 minutes. After the centrifugation was completed, the column was placed in a 1.5 ml tube, 30 µl of EB buffer was dropped in the center, and reaction was allowed to occur at room temperature for 1 minute. Centrifugation was performed at 13,000 rpm for 1 minute. The plasmid vector collected in the 1.5 ml tube was quantified, and then stored at -20°C.

### 4. Production of cassette DNA

To identify indel efficiency of targets that are spacers of Cas14a1, a cassette containing a U6 promoter and a scaffold sequence, a guide sequence, and a U-rich tail sequence (T₄AT₆) was amplified by PCR and used. The procedure was performed in the following manner.

### 1) Selection of spacer and order of oligo

For spacers, 20-mer sequences located after TTTR, which is PAM, were selected, and spacers ending in T were excluded. To decrease off-target, the spacer was designed in CRISPR RGEN TOOL in such a way that 2 or fewer mismatches were sorted. Reverse complement sequences containing a direct repeat and a U-rich sequence were ordered for use as R primers.

### 2) PCR

PCR was performed with the following composition and conditions.

**[Table 7] PCR-related information**

| Reagent | | PCR conditions | |
|---|---|---|---|
| 2X pfu PCR Master mix | 205 *µ*ℓ | Pre-denaturation | 95°C, 5 min |
| hU6 F primer (10 P) | 2.05 *µ*ℓ | Denaturation | 95°C 30 sec |
| Target oligo (10 P) | 2.05 *µ*ℓ | Annealing | 60°C, 30 sec |
| Template | 1 *µ*ℓ (200 ng) | Extension | 72°C, 2 min |
| DW | 199.9 *µ*ℓ | D-E cycle | 30 cycles |
| Total | 410 *µ*ℓ | Final extension | 72°C, 3 min |
| Divide by 50 µl and proceed in 8 PCR tubes | | Storage | 4°C, ∞ |

400 µl of the mixture was placed in PCR tubes at 50 µl each, and one sample was amplified using 8 tubes.

### 3) Gel analysis

Amplification sizes were checked by electrophoresis on 1% agarose gel.

### 4) PCR purification & quantification

### 5. Cell culture

HEK-293T cells and ARPE-19/HPV-16 cells used in the experiment were cultured with the following composition and conditions: HEK-293T cell media - DMEM, 10% FBS, 1% penicillin-streptomycin; and AREP-19/HPV-16 cell media - DEME/F12, 10% FBS, 1% penicillin-streptomycin. Frozen cells were rapidly thawed at 37°C, and then placed and dissolved well in 5 ml of pre-warmed cell media. Then, centrifugation was performed at 1,500 rpm for 3 minutes. After the centrifugation, the supernatant with remaining freezing liquid was rapidly removed, and the pellet was loosened well with the cell media. Then, the resultant was aliquoted into two 90 mm dishes each containing 10 ml of media and culture was performed. The next day, the cell media was replaced with new media and culture was performed continuously. When the confluency reached 80%, subcultures were performed at respective ratios (performed at a ratio of 1/15 for HEK-293T, and at a ratio of 1/4 for AREP-19/HPV-16).

### 6. Transfection (HEK-293T, ARPE19-HPV)

One day before transfection, the cells (80% confluency) were removed from the bottom of 100 mm dishes by treatment with trypsin. The removed cells were put into 50 ml of pre-warmed media (HEK-293T - DMEM, 10% FBS, 1% p/s, ARPE19-HPV - DMEM/F12, 10% FBS, 1% p/s) and loosened slowly with a pipette. 24-well plates were prepared depending on the number of samples and repetitions, and 500 µl of media in which the cells were loosened once was added to each well (1/100 dilution, 0.5 ml/50 ml). Then, incubation was performed overnight in a CO₂ incubator at 37°C until transfection. Transfection was performed when the confluency reached about 70 to 80%. Out of 500 µl of media per well, 200 µl of media was removed and placed in the incubator. 1.5 ml tubes were prepared depending on the number of samples, and 200 µl of Opti-MEM was added to each tube. A corresponding amount of DNA was added to the tube containing Opti-MEM and vortexing was performed for 5 seconds (DNA - Cas14a1 1.5 µg + gRNA 0.5 µg). Then, FuGENE HD was added at a ratio of DNA:Reagent = 1:3 (6 µl of FuGENE HD was added when DNA was 2 µg). Reaction was allowed to occur at room temperature for 20 minutes. The 24-well plate was taken out of the incubator, and 200 µl of the reaction solution of No. 8 was allowed to flow through the wall of the well. The plate was sufficiently shaken in an S shape, and incubation was performed in a CO₂ incubator at 37°C up to 72 hours. After 72 hours, the cells were harvested, and gDNA was extracted therefrom.

### 7. NGS sample PCR

Preparation of NGS samples for identifying indel efficiency of targets was done through PCR product purification after conducting 1st to 3rd PCRs.

### 1) Composition and conditions for PCR (first PCR)

F: CEP290-F-F#10, LCA10-2nd-9-R (1323 bp), R : CEP290-R-F#1, CEP290-R-R#8 (1879 bp)

**[Table 8] PCR-related information**

| Reagent | | PCR conditions | |
|---|---|---|---|
| KAPA HiFi 2X PCR mix | 5 *µℓ* | Pre-denaturation | 95°C, 3 min |
| Forward primer (10 P) | 0.5 *µℓ* | Denaturation | 98°C, 20 sec |
| Reverse primer (10 P) | 0.5 *µℓ* | Annealing | 60°C, 15 sec |
| Template (gDNA) | 1*µℓ* | Extension | 72°C, 2 min |
| DW | 3 *µℓ* | D-E cycle | 30 cycles |
| Total | 10 *µℓ* | Final extension | 72°C, 3 min |
| | | Storage | 4°C, ∞ |

### 2) Composition and conditions for PCR (second PCR)

**[Table 9] PCR-related primer information**

| Primer set | Forward primer | Reverse primer | Protospacer sequence | | | |
|---|---|---|---|---|---|---|
| S1 | LCA10-2nd-1-F | LCA10-2nd-1-R | F21 | | | |
| S2 | LCA10-2nd-2-F | LCA10-2nd-2-R | F17 | F18 | F19 | F20 |
| S3 | LCA10-2nd-3-F | CEP-F-R#11-miseq-R | F12 | F15 | F16 | |
| S4 | LCA10-2nd-4-F | LCA10-2nd-4-R | F11 | F14 | | |
| S5 | CEP-F-F#9-miseq-F | CEP-F-R#8-miseq-R | F10 | F13 | F22 | |
| S6 | LCA10-2nd-6-F | LCA10-2nd-6-R | F04 | F07 | F08 | |
| S7 | CEP-F-F#11-miseq-F | CEP-F-R#10-miseq-R | F03 | F05 | F23 | F24 |
| S8 | CEP-F-F#3-miseq-F | CEP-F-R#3-miseq-R | F01 | F02 | F09 | |
| S9 | LCA10-2nd-9-F | CEP-F-R#5-miseq-R | F05 | | | |
| S10 | LCA10-2nd-10-F | LCA10-2nd-10-R | R05 | R06 | R07 | |
| S11 | LCA10-2nd-11-F | LCA10-2nd-11-R | R04 | R09 | R10 | |
| S12 | CEP-R-F#5-miseq-F | CEP-R-R#4-miseq-R | R03 | R11 | | |
| S13 | LCA10-2nd-13-F | LCA10-2nd-13-R | R01 | R02 | R08 | |
| S14 | LCA10-2nd-14-F | LCA10-2nd-14-R | R12 | R13 | | |
| S15 | LCA10-2nd-15-F | LCA10-2nd-15-R | R24 | R26 | | |
| S16 | LCA10-2nd-16-F | LCA10-2nd-16-R | R22 | R23 | | |
| S17 | LCA10-2nd-17-F | LCA10-2nd-17-R | R21 | | | |
| S18 | LCA10-2nd-18-F | LCA10-2nd-18-R | R20 | R25 | | |
| S19 | LCA10-2nd-19-F | LCA10-2nd-19-R | R19 | | | |
| S20 | LCA10-2nd-20-F | LCA10-2nd-20-R | R16 | R17 | R18 | |
| S21 | LCA10-2nd-21-F | LCA10-2nd-21-R | R14 | R15 | | |

**[Table 10] PCR-related information**

| Reagent | | PCR conditions | |
|---|---|---|---|
| KAPA HiFi 2X PCR mix | 5 *µℓ* | Pre-denaturation | 95°C, 3 min |
| Forward primer (10 P) | 0.5 *µℓ* | Denaturation | 98°C, 20 sec |
| Reverse primer (10 P) | 0.5 *µℓ* | Annealing | 60°C, 15 sec |
| Template (PCR product) | 0.5 *µℓ* | Extension | 72°C, 30 sec |
| DW | 3.5 µℓ | D-E cycle | 30 cycles |
| Total | 10 *µℓ* | Final extension | 72°C, 3 min |
| | | Storage | 4°C, ∞ |

The samples RO1, R02, R08, and S13 NT were annealed at 57°C, and the samples R20, R25, S18 NT, R19, and S19 NT were annealed at 53°C.

### 3) Composition and conditions for PCR (third PCR)

**[Table 11] PCR-related information**

| Reagent | | PCR conditions | |
|---|---|---|---|
| KAPA HiFi 2X PCR mix | 5 *µ*ℓ | Pre-denaturation | 95°C, 3 min |
| Forward primer (10 P) | 0.5 *µ*ℓ | Denaturation | 98°C, 20 sec |
| Reverse primer (10 P) | 0.5 µ*ℓ* | Annealing | 60°C, 15 sec |
| Template (PCR product) | 0.5 *µ*ℓ | Extension | 72°C, 30 sec |
| DW | 3.5 *µℓ* | D-E cycle | 30 cycles |
| Total | 10 *µℓ* | Final extension | 72°C, 3 min |
| | | Storage | 47°C, ∞ |

The samples R01, R02, R08, S13 NT, R20, R25, S18 NT, R19, and S19 NT were annealed at 57°C, and the samples F10, F13, F22, S5 NT, R12, R13, S14 NT, R24, R26, S15 NT, R16, R17, R18, and S20NT were annealed at 63°C.

### 4) Summary of PCR primers

**[Table 12] PCR-related primer information**

| Use | Target region | No. | Name | Direction | Sequence | Length (nt) |
|---|---|---|---|---|---|---|
| For 1st PCR | F region | 1 | CEP290-F-F#10 | F | TGAACTGACTGCTAAGTACAGGGACA (SEQ ID NO: 147) | 26 |
| | | 2 | LCA10-2^{nd}-9-R | R | AAGCGTTAAGCCGAGATCGT (SEQ ID NO: 148) | 20 |
| | R region | 3 | CEP290-R-F#1 | F | TGTGGCAGCCATTATTCCTGTCTCTA (SEQ ID NO: 149) | 26 |
| | | 4 | CEP290-R-R#8 | R | TCTGCCTTGACTAGCTGGTTAGGTAA (SEQ ID NO: 150) | 26 |

**[Table 13] PCR-related primer information**

| Use | Target region | No. | Name | Direction | Sequence | Length (nt) |
|---|---|---|---|---|---|---|
| | | 1 | LCA10-2nd-1-F | F | CACTCTTTCCCTACACGACGCTCTTCCGATCTGAACTGACTGCTAAGTACAGGGA C (SEQ ID NO: 151) | 56 |
| | | 2 | LCA10-2nd-1-R | R | GTGACTGGAGTTCAGACGTGTGCTCTTCCGATCTGCAGGCAAACTTTAATTAAAA TCTGCC (SEQ ID NO: 152) | 61 |
| | | 3 | LCA10-2nd-2-F | F | CACTCTTTCCCTACACGACGCTCTTCCGATCTGGAACACCTGGAGGTAAGTTTG (SEQ ID NO: 153) | 54 |
| | | 4 | LCA10-2nd-2-R | R | GTGACTGGAGTTCAGACGTGTGCTCTTCCGATCTCATTCAGAAGTCATTCAGCCA CT (SEQ ID NO: 154) | 57 |
| | | 5 | LCA10-2nd-3-F | F | CACTCTTTCCCTACACGACGCTCTTCCGATCTTTGTTCCCACATAACACACACA (SEQ ID NO: 155) | 54 |
| | | 6 | CEP-F-R#11-miseq-R | R | GTGACTGGAGTTCAGACGTGTGCTCTTCCGATCTTTCTGGGTACAGGGGTAAGAG AAAGG (SEQ ID NO: 156) | 60 |
| | | 7 | LCA10-2nd-4-F | F | CACTCTTTCCCTACACGACGCTCTTCCGATCTTGTAATCAAAGGAGGGAAGCA (SEQ ID NO: 157) | 53 |
| | | 8 | LCA10-2nd-4-R | R | GTGACTGGAGTTCAGACGTGTGCTCTTCCGATCTGATGAGAATTTACAGAGTGCA TCC (SEQ ID NO: 158) | 58 |
| for 2nd PCR | F region | 9 | CEP-F-F#9-miseq-F | F | CACTCTTTCCCTACACGACGCTCTTCCGATCTACCCAGAACAAACTATTCTCCCA TGG (SEQ ID NO: 159) | 58 |
| | | 10 | CEP-F-R#8-miseq-R | R | GTGACTGGAGTTCAGACGTGTGCTCTTCCGATCTGGGTCTGGTCCATATTCACAG ATTGT (SEQ ID NO: 160) | 60 |
| | | 11 | LCA10-2nd-6-F | F | CACTCTTTCCCTACACGACGCTCTTCCGATCTTGGACCAGACCCTTTGTAGTT (SEQ ID NO: 161) | 53 |
| | | 12 | LCA10-2nd-6-R | R | GTGACTGGAGTTCAGACGTGTGCTCTTCCGATCTTGTCTCTATGAGCCAGCAAAA G (SEQ ID NO: 162) | 56 |
| | | 13 | CEP-F-F#11-miseq-F | F | CACTCTTTCCCTACACGACGCTCTTCCGATCTTCATCTTCCTCATCAGAAATAGA GGCTT (SEQ ID NO: 163) | 60 |
| | | 14 | CEP-F-R#10-miseq-R | R | GTGACTGGAGTTCAGACGTGTGCTCTTCCGATCTGCAAAAGCAGCAGAAAGCAAA CTGAT (SEQ ID NO: 164) | 60 |
| | | 15 | CEP-F-F#3-miseq-F | F | CACTCTTTCCCTACACGACGCTCTTCCGATCTTGCTGGCTCATAGAGACACATTC AGT (SEQ ID NO: 165) | 58 |
| | | 16 | CEP-F-R#3-miseq-R | R | GTGACTGGAGTTCAGACGTGTGCTCTTCCGATCTTGCTGGAGAGGATAGGACAGA GGA (SEQ ID NO: 166) | 58 |
| | | 17 | LCA10-2nd-9-F | F | CACTCTTTCCCTACACGACGCTCTTCCGATCTTTCCATTCTCCATGTCCTCTG (SEQ ID NO: 167) | 53 |
| | | 18 | CEP-F-R#5-miseq-R | R | GTGACTGGAGTTCAGACGTGTGCTCTTCCGATCTGGGTGACAGAATGAAACTCCA TCTCA (SEQ ID NO: 168) | 60 |

**[Table 14] PCR-related primer information**

| Use | Target region | No. | Name | Direction | Sequence | Length (nt) |
|---|---|---|---|---|---|---|
| | | 1 | LCA10-2nd-10-F | F | CACTCTTTCCCTACACGACGCTCTTCCGATCTGTGGCAGCCATTATTCCTGT (SEQ ID NO: 169) | 52 |
| | | 2 | LCA10-2nd-10-R | R | GTGACTGGAGTTCAGACGTGTGCTCTTCCGATCTGAACACTGTGGTCAGAAAACT CA (SEQ ID NO: 170) | 57 |
| | | 3 | LCA10-2nd-11-F | F | CACTCTTTCCCTACACGACGCTCTTCCGATCTATGTATGTTCTTTTGTACGTGCTC TT (SEQ ID NO: 171) | 58 |
| | | 4 | LCA10-2nd-11-R | R | GTGACTGGAGTTCAGACGTGTGCTCTTCCGATCTAAGACAGATATGTAAACACAT ATAATTTGA (SEQ ID NO: 172) | 64 |
| | | 5 | CEP-R-F#5-miseq-F | F | CACTCTTTCCCTACACGACGCTCTTCCGATCTGTCACTCCTGTCTACACTAGTTC TGC (SEQ ID NO: 173) | 58 |
| | | 6 | CEP-R-R#4-miseq-R | R | GTGACTGGAGTTCAGACGTGTGCTCTTCCGATCTACCATATTTGACACCACATGC ACTGT (SEQ ID NO: 174) | 60 |
| | | 7 | LCA10-2nd-13-F | F | CACTCTTTCCCTACACGACGCTCTTCCGATCTTTCCTTAATGGAATATAAGTCTTT TGA (SEQ ID NO: 175) | 59 |
| | | 8 | LCA10-2nd-13-R | R | GTGACTGGAGTTCAGACGTGTGCTCTTCCGATCTCCTTATTATCTATTCCATTCTT CACACA (SEQ ID NO: 176) | 62 |
| | | 9 | LCA10-2nd-14-F | F | CACTCTTTCCCTACACGACGCTCTTCCGATCTGGTTCATGAGACTAGAGGTCACG (SEQ ID NO: 177) | 55 |
| | | 10 | LCA10-2nd-14-R | R | GTGACTGGAGTTCAGACGTGTGCTCTTCCGATCTCTGGGCAACACAGTGAGACT (SEQ ID NO: 178) | 54 |
| | | 11 | LCA10-2nd-15-F | F | CACTCTTTCCCTACACGACGCTCTTCCGATCTGGTTCAAGCGATTCTTCTGC (SEQ ID NO: 179) | 52 |
| for 2nd PCR | R region | 12 | LCA10-2nd-15-R | R | GTGACTGGAGTTCAGACGTGTGCTCTTCCGATCTCCTGAAACTTCACTGGTCTCC (SEQ ID NO: 180) | 55 |
| | | 13 | LCA10-2nd-16-F | F | CACTCTTTCCCTACACGACGCTCTTCCGATCTAGCCTCAACTTCCCAAAGTG (SEQ ID NO: 181) | 52 |
| | | 14 | LCA10-2nd-16-R | R | GTGACTGGAGTTCAGACGTGTGCTCTTCCGATCTTCTTTTTCTCCCACCTACCC (SEQ ID NO: 182) | 54 |
| | | 15 | LCA10-2nd-17-F | F | CACTCTTTCCCTACACGACGCTCTTCCGATCTTTTAGAGTTACTAGGGAGAGAGT GATGA (SEQ ID NO: 183) | 60 |
| | | 16 | LCA10-2nd-17-R | R | GTGACTGGAGTTCAGACGTGTGCTCTTCCGATCTACACAGGAGGGAAAACAGGA (SEQ ID NO: 184) | 54 |
| | | 17 | LCA10-2nd-18-F | F | CACTCTTTCCCTACACGACGCTCTTCCGATCTTCCTGTGAAAAAGGATGAAAGG (SEQ ID NO: 185) | 54 |
| | | 18 | LCA10-2nd-18-R | R | GTGACTGGAGTTCAGACGTGTGCTCTTCCGATCTTGAAGAATAATACAAACAGGG TAATGA (SEQ ID NO: 186) | 61 |
| | | 19 | LCA10-2nd-19-F | F | CACTCTTTCCCTACACGACGCTCTTCCGATCTCTCCTGTTTTCCCTCCTGTG (SEQ ID NO: 187) | 52 |
| | | 20 | LCA10-2nd-19-R | R | GTGACTGGAGTTCAGACGTGTGCTCTTCCGATCTTTTGAGTGGAGTACCTGGAAA (SEQ ID NO: 188) | 55 |
| | | 21 | LCA10-2nd-20-F | F | CACTCTTTCCCTACACGACGCTCTTCCGATCTTGTCCTCTTTTTAGTCTCATTACC C (SEQ ID NO: 189) | 57 |
| | | 22 | LCA10-2nd-20-R | R | GTGACTGGAGTTCAGACGTGTGCTCTTCCGATCTGGAAAAAGGCTGGCGATATAA (SEQ ID NO: 190) | 55 |
| | | 23 | LCA10-2nd-21-F | F | CACTCTTTCCCTACACGACGCTCTTCCGATCTATATCGCCAGCCTTTTTCCT (SEQ ID NO: 191) | 52 |
| | | 24 | LCA10-2nd-21-R | R | GTGACTGGAGTTCAGACGTGTGCTCTTCCGATCTGCCTTGACTAGCTGGTTAGGT A (SEQ ID NO: 192) | 56 |

| | | | | | | |
|---|---|---|---|---|---|---|
| *The target region is divided into a forward region (F region) and a reverse region (R region), in which the F region and the R region correspond to the upstream 1654 bp portion and the downstream 2000 bp portion, respectively, based on the c.2991+1655A>G gene mutation. | | | | | | |

### 8. T-blunt end cloning

The target cassette or PCR product was cloned into a T-vector to identify vectorization of the cassette or the sequence of the PCR product. The All in One^{™} PCR Cloning Kit (VT202-020, Biofact) was used. Cloning was performed using the product or cassette DNA which was designed so that the length of DNA does not exceed 2 kb. A mixture having the following composition was prepared and subjected to ligation reaction.

**[Table 15] Composition information for cloning**

| Component | Volume | |
|---|---|---|
| 6X All in one buffer | 1 *µℓ* | Reaction at room temperature for 30 minutes |
| All in one vector | 1 *µℓ* | |
| PCR Product or Cassette | 4 *µℓ* | |
| Total | 6 *µℓ* | |

After allowing reaction to occur for 30 minutes, transformation was performed on E. coli C.P. cells. After completion of incubation on a LB Plate, positive colonies were identified through colony PCR and incubation was performed in 3 ml of LB medium. After Miniprep, sequencing was performed to finally identify sequence matching.

### 9. Vector construction

The following procedure was performed using the Cas14a1 Ver. 4.0 Dual gRNA vector (Korean Patent Application Nos. 10-2021-0050093 and 10-2021-0051552). Restriction enzyme ends of the vector to be cloned were checked, and a dual gRNA oligo was designed and ordered for custom fabrication. The custom-fabricated oligos were diluted to become 100 pmol. 4.5 µl each of forward and reverse primers was taken and put into a PCR tube, and then 1 µl of 10X annealing buffer was added to adjust the volume to 10 µl. Then, annealing was performed (annealing was performed under the conditions of 95°C, 5 minutes and -1°C/min from 95°C to 4°C). The Cas14a1 Ver. 4.0 Dual gRNA vector was prepared and digested with the following composition and conditions.

**[Table 16] Composition information for digestion**

| Reagent | Volume | |
|---|---|---|
| NEB 10X 2.1 buffer | 5 *µℓ* | Reaction at 37°C, 500 rpm for 2 hours. |
| Vector | 10 *µ*g | |
| Bbs I | 10 *µℓ* | |
| DW | Up to 50 µℓ | |
| Total | 50 *µℓ* | |

After the digestion, the digested vector was obtained through electrophoresis and gel elution. Ligation was performed using the digested vector and the annealed oligo.

**[Table 17] Composition information for ligation**

| Reagent | Volume | |
|---|---|---|
| DNA ligation mix (TAKARA) | 2 *µℓ* | Reaction at room temperature for 5 minutes |
| Annealed Oligo | 1.5 *µℓ* | |
| Bbsl cut vector | 0.5 *µℓ* | |
| Total | 4 *µℓ* | |

After the ligation, DH5a transformation was performed. After completion of incubation on a LB Plate, positive colonies were identified through colony PCR and incubation was performed in 3 ml of LB medium. After Miniprep, sequencing was performed to finally identify sequence matching.

### 10. DH5α transformation

The previously produced vector was transformed into E. coli to produce the vectors. DH5a competent cells were taken out and thawed on ice. The ligated vector was added up to 1/10 of the amount of DH5a and then reaction was allowed to occur on ice for 30 minutes. The resultant was subjected to heat shock at 42°C for 30 seconds, and then cooled on ice for 2 minutes. Incubation was performed at 37°C for 1 hour using 100 µl of LB medium or SOC medium. The resultant was smeared on an LB plate warmed to room temperature (which contains ampicillin or kanamycin depending on the vector), and incubation was performed at 37°C for 14 to 16 hours.

### 11. Production of ARPE19/HPV16-LCA10 cell line

ARPE19/HPV16 cells were seeded in a 100 mm dish on the day before transfection (in which seeding was performed so that the confluency reached 70% on the day of transfection). The next day, confluency was checked, and all cells were removed from the dish using trypsin. Washing was performed twice with PBS, and the supernatant was discarded. Mixing of 100 µl of NEON Transfection R buffer with DNA (vector 6 µg, donor 6 µg) was performed, and then the mixture was mixed well with the previously obtained cell pellet. Transfection was carried out under the conditions of 1350V, 30 ms, 2 pulses. Then, the resultant was placed in a 100 mm dish containing the prepared cell media, shaken well, and incubated for 24 hours. After performing the incubation for 24 hours, selection was carried out with puromycin (8 µg/ml). After carrying out the selection with puromycin for 2 weeks, the clones were transferred to a 96-well plate and genotyping was performed. After the genotyping, the selected cells were sub-cultured in a 24-well plate and analyzed by RT-PCR when the cells grew beyond a certain amount.

### 12. Screening of ARPE19/HPV16-LCA10 cells

The clones, which were selected for 2 weeks by puromycin (8 µg/ml), were checked for gene insertion through direct PCR and Sanger sequencing. The PCR process was carried out as follows. Direct PCR was performed twice, and then the PCR product was purified. A request for sequence analysis thereof was made to Macrogen. After the analysis, cells containing c.2991+1655A>G were checked, and the cells containing the mutation were named according to the following nomenclature: A to Z, A to H, numbers of 1 to 12 (A to Z: number of experiments, A being for first experiment, B for second experiment, C for third experiment ... in such an order up to Z; A to H: characters corresponding to rows of a 96-well plate (rows A to H from top); numbers of 1 to 12: numbers corresponding to columns in respective rows, for example, AC7: clone at row C, seventh column in a 96-well plate for first experiment). As a result of the sequence analysis, c.2991+1655A>G was identified in the LCA10-AC7 clone.

**[Table 18] First PCR-related information**

| **1st PCR** | | | **PCR Condition** | |
|---|---|---|---|---|
| 2X KOD One | 5 *µℓ* | 1 X | 95°C | 5min |
| F (10pmol) | 0.5 *µ*ℓ | 0.5 µM | 98°C | 10sec |
| R (10pmol) | 0.5 *µ*ℓ | 0.5 µM | 59°C | 5sec |
| Tween20 | 0.2 | | 68°C | 10sec |
| Cell lysate | 3.8 *µ*ℓ | | 68°C | 2min |
| Total | 10 *µ*ℓ | | Cycle | 33 |

**[Table 19] Second PCR-related information**

| **2nd PCR** | | | **PCR Condition** | |
|---|---|---|---|---|
| 2X KOD One | 5 *µℓ* | 1 X | 95°C | 5min |
| F (10pmol) | 0.5 *µℓ* | 1 µM | 98°C | 10sec |
| R (10pmol) | 0.5 *µ*ℓ | 1 µM | 59°C | 5sec |
| DW | 2 *µℓ* | | 68°C | 10sec |
| 1st PCR product | 2 *µℓ* | | 68°C | 2min |
| Total | 10 *µℓ* | | Cycle | 33 |

**[Table 20] Primer information**

| Target | Primer set | | Product size |
|---|---|---|---|
| c.2991+1655A>G site | Forward primer | CTTGGCTCACTGCAAGCTCCAC (SEQ ID NO: 193) | 397 bp |
| | Reverse primer | ACAGGGTAGGATTCATGTTTAGAATGATCA (SEQ ID NO: 194) | |

### Example 1. Selection of candidates for protospacer sequence and guide sequence

In order to decrease expression of the cryptic exon X caused by the c.2991+1655A>G gene mutation in the CEP290 gene, the target region was divided into a forward region (F region) and a reverse region (R region), in which the F region and the R region correspond to the upstream 1654 bp portion and the downstream 2000 bp portion, respectively, based on the c.2991+1655A>G gene mutation, and protospacer sequences corresponding to the respective regions were selected. Due to the nature of Cas14a1 in which TTTR is used as a PAM, among the existing Cas12a1 target sequences, protospacer sequences whose PAM is TTTR excluding TTTC were preferentially selected. The selected protospacer sequences are summarized in Tables 21 and 22. In order to easily distinguish the respective sequences, the sequences present in the F region were classified as F and numbered, and the sequences present in the R region were classified as R and numbered. These sequences are summarized in Tables 21 and 22.

**[Table 21] Candidates for protospacer sequence**

| Target region | Name | PAM (TTTR) | Protospacer sequence (20nt) | SEQ ID NO |
|---|---|---|---|---|
| | PS-FO1 | TTTA | TAGAAAATTATGCCTATTTA | 195 |
| | PS-F02 | TTTG | GCAAAAGCAGCAGAAAGCAA | 196 |
| | PS-F03 | TTTA | GTAGGAATCCTGAAAGCTAC | 197 |
| | PS-F04 | TTTA | CTGAGGACAGAACAAGCTCC | 198 |
| | PS-F05 | TTTG | GGAAAATGATAACGTTAAAC | 199 |
| | PS-F06 | TTTA | CTGAATGTGTCTCTATGAGC | 200 |
| | PS-F07 | TTTG | TTCATCTTCCTCATCAGAAA | 201 |
| | PS-F08 | TTTG | GATTATTCTTATCTAAGATC | 202 |
| | PS-F09 | TTTA | TCAATATTATTTAGAAGTAA | 203 |
| | PS-F10 | TTTA | CAGAGTGCATCCATGGTCCA | 204 |
| | PS-F11 | TTTG | TTCCTTGGAATGGATGTAGC | 205 |
| F region | PS-F12 | TTTA | ACCAGACATCTAAGAGAAAA | 206 |
| | PS-F13 | TTTA | TTCTACATTAAAATTCTACA | 207 |
| | PS-F14 | TTTG | TTCTGGGTACAGGGGTAAGA | 208 |
| | PS-F15 | TTTG | ATTCAGCTAAATCATGCAAG | 209 |
| | PS-F16 | TTTA | TAGTGGCTGAATGACTTCTG | 210 |
| | PS-F17 | TTTG | TTCCCACATAACACACACAC | 211 |
| | PS-F18 | TTTA | TAACTAAACTGAATGAATAA | 212 |
| | PS-F19 | TTTA | ATTAAAATCTGCCAAATCCC | 213 |
| | PS-F20 | TTTG | TGTTTGGGGGGATTTGGCAG | 214 |
| | PS-F21 | TTTG | TGTGATTCTTGAACCTTGTG | 215 |
| | PS-F22 | TTTA | TTCTACAATAAAAAATGATG | 216 |
| | PS-F23 | TTTG | CTGGCTCATAGAGACACATTC | 217 |
| | PS-F24 | TTTG | AGCTAATAGAATTAGATCTTA | 218 |

**[Table 22] Candidates for protospacer sequence**

| Target region | Name | PAM (TTTR) | Protospacer sequence (20nt) | SEQ ID NO |
|---|---|---|---|---|
| | PS-R01 | TTTG | AGAGGTAAAGGTTCATGAGA | 219 |
| | PS-R02 | TTTG | GCACAGAGTTCAAGCTAATA | 220 |
| | PS-R03 | TTTA | CATATCTGTCTTCCTTAATG | 221 |
| | PS-R04 | TTTA | ATGGAACAAAGTCACTCCTG | 222 |
| | PS-R05 | TTTA | CCCTTCAGGTAACCGGTAGC | 223 |
| | PS-R06 | TTTA | GAATGATCATTCTTGTGGCA | 224 |
| | PS-R07 | TTTA | AGGCGGGGAGTCACATGGGA | 225 |
| | PS-R08 | TTTA | CCTCTCAAAAAGCAACAATA | 226 |
| | PS-R09 | TTTG | AAACAGGAATAGAAATTCAC | 227 |
| | PS-R10 | TTTG | TTCCATTAAAAAAAGTATGC | 228 |
| | PS-R11 | TTTA | ATTTGTTTCTGTGTGTTGAG | 229 |
| | PS-R12 | TTTG | TATTTCCTTATTATCTATTC | 230 |
| R region | PS-R13 | TTTG | ATAGTAGAAGAAAAAAGAAA | 231 |
| | PS-R14 | TTTG | AGATCCCCTTGAAACACAGG | 232 |
| | PS-R15 | TTTA | CAATTCCTGTGTTTCAAGGG | 233 |
| | PS-R16 | TTTA | TCACTGAAATCCATTGCTTA | 234 |
| | PS-R17 | TTTA | TTTATCACTGAAATCCATTG | 235 |
| | PS-R18 | TTTG | AGTGGAGTACCTGGAAAATA | 236 |
| | PS-R19 | TTTA | GTCTCATTACCCTGTTTGTA | 237 |
| | PS-R20 | TTTA | TATACTATGAATACACAGGA | 238 |
| | PS-R21 | TTTG | TAACAAACAATAGGATTATC | 239 |
| | PS-R22 | TTTA | GAGTTACTAGGGAGAGAGTG | 240 |
| | PS-R23 | TTTA | AAGGAGACCAGTGAAGTTTC | 241 |
| | PS-R24 | TTTG | GATCTCAGGTAATCCGCCAG | 242 |
| | PS-R25 | TTTA | AGTAAAGTACATAATTATAG | 243 |
| | PS-R26 | TTTG | AGACCAGCCCGGCCAACATG | 244 |

Guide RNAs were designed based on the selected protospacer sequences. The guide RNA has a guide sequence that binds complementarily to a target sequence. Such a guide sequence may be designed using a protospacer sequence. The protospacer sequence is a sequence that binds complementarily to a target sequence, and correlation between the protospacer sequence and the target sequence is similar to correlation between the target sequence and the guide sequence. Due to this feature, when designing a guide sequence, the guide sequence may be generally designed using a protospacer sequence. That is, when designing a guide sequence that binds complementarily to a target sequence, the guide sequence may be designed as a nucleotide sequence having the same nucleotide sequence as the protospacer sequence. Here, the guide sequence is designed by replacing T with U in the nucleotide sequence of the protospacer sequence. Therefore, guide sequences were designed using the selected protospacer sequences, and these guide sequences are summarized in Tables 3 and 4.

### Example 2. Selection of guide sequence

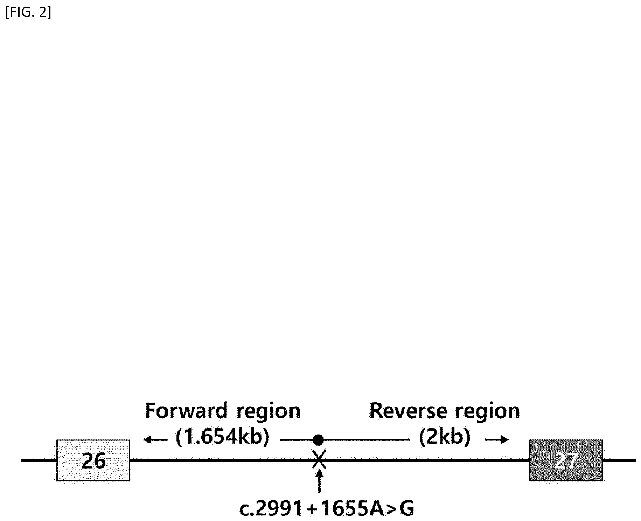

A nucleic acid sequence encoding each of guide RNAs designed based on the selected protospacer sequences was prepared as a cassette and transfected into HEK-293T cells using FuGENE HD along with a Cas14a1 vector. Here, the guide sequences of the guide RNAs are the same as in Tables 3 and 4, and 5'-ACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAA GUAACCCUCGAAACAAAUUCAGUGCUCCUCUCCAAUUCUGCACAAGAAA GUUGCAGAACCCGAAUAGAGCAAUGAAGGAAUGCAAC-3' (SEQ ID NO: 126) was used as a scaffold sequence of the guide RNAs. In addition, the guide RNA was designed to contain the sequence 5'-U₄AU₄-3', which is a U-rich tail sequence, at the 3' end. Using gDNA from the cells that were 60 to 72 hours after the transfection, indel efficiency was checked by NGS. As a result of the analysis, guide RNAs having the guide sequences F14, F16, F17, and R22, respectively, showed high indel efficiency (FIG. 2).

**[Table 23] Guide RNAs having respective guide sequences and indel efficiency (%) thereof caused by Cas14a1**

| Target region | Name | Guide sequence (20nt) | SEQ ID NO | NGS | %Indel |
|---|---|---|---|---|---|
| F region | Guide-F14 | UUCUGGGUACAGGGGUAAGA | 64 | O | 34.4 |
| | Guide-F16 | UAGUGGCUGAAUGACUUCUG | 66 | O | 17.07 |
| | Guide-F17 | UUCCCACAUAACACACACAC | 67 | O | 26.6 |
| R region | Guide-R22 | GAGUUACUAGGGAGAGAGUG | 96 | O | 26.73 |
| | Guide-R23 | AAGGAGACCAGUGAAGUUUC | 97 | O | 0.16 |
| | Guide-R24 | GAUCUCAGGUAAUCCGCCAG | 98 | O | 0.21 |

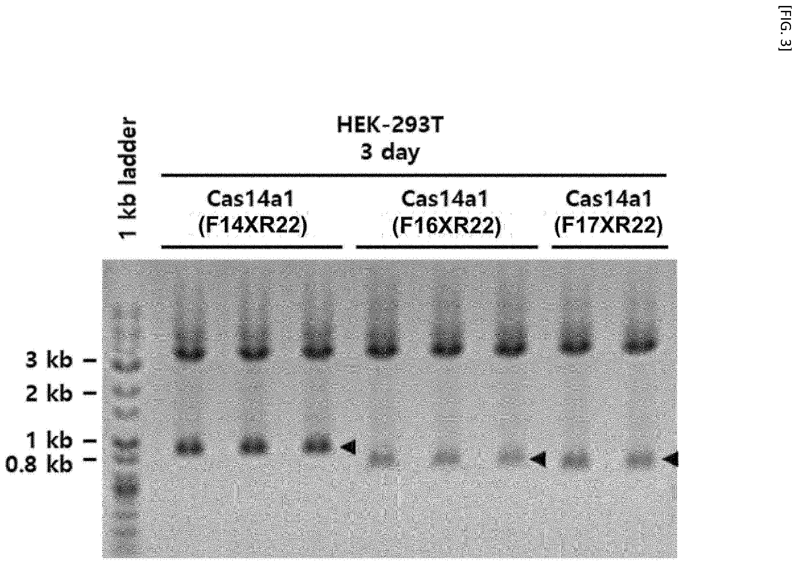

Based on these results, sets were constructed by combining a guide RNA having the guide sequence F14, F16, or F17 that targets the F region, and a guide RNA having the guide sequence R22 that targets the R region. The respective sets were compared for deletion efficiency of a partial region in the CEP290 gene. As a result, NT, which is a control with no treatment, showed a band of 3672 bp, the F14 X R22 set (that is, a guide RNA having F14 as a guide sequence and a guide RNA having R22 as a guide sequence) resulted in deletion of about 2500 bp and showed band of about 1170 bp, and the F16 X R22 set (that is, a guide RNA having F16 as a guide sequence and a guide RNA having R22 as a guide sequence) resulted in deletion of about 2700 bp and showed band of about 1000 bp. In addition, the F17 X R22 set (that is, a guide RNA having F17 as a guide sequence and a guide RNA having R22 as a guide sequence) resulted in deletion of about 2750 bp and showed a band of 950 bp. As a result of comparing intensity of the deletion bands, it was identified that the F14 X R22 set exhibited the best efficiency as in the NGS results (FIG. 3).

### Example 2-1. Identification of deletion of partial region in CEP290 gene

Nucleic acids encoding the guide RNA set, which previously showed high indel efficiency, that is, the F14 X R22 set (that is, a guide RNA having F14 as a guide sequence and a guide RNA having R22 as a guide sequence), were vectorized into a cassette through T-blunt end cloning, and deletion of a partial region in the CEP290 gene was additionally identified. EDIT101 (Morgan L. Maeder et al., Nature Medicine, 25, 229-233 (2019)) was used as a control for comparison of efficiency.

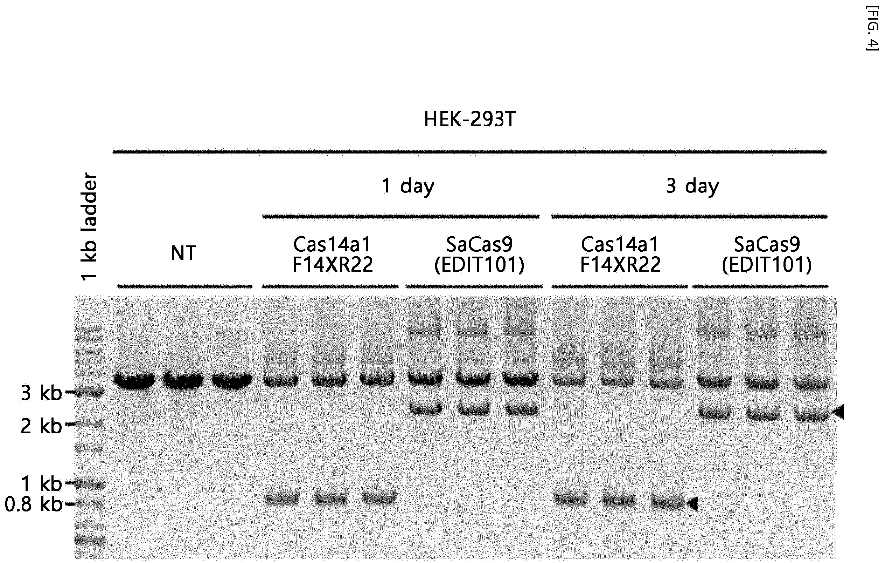

As a result of identifying deletion by transfection into HEK-293T, it was difficult to determine efficiency by intensity due to a large difference in size of deletion band between the experimental group and the control; however, as a result of comparing intensity between the band of the negative control NT with no treatment and a band of the same size (that is, a portion not cleaved by the CRISPR/Cas system), it was identified that Cas14a1 and F14 X R22 showed lower band intensity than EDIT101. As a result, it was predictable that Cas14a1 and F14 X R22 showed higher deletion efficiency (FIG. 4).

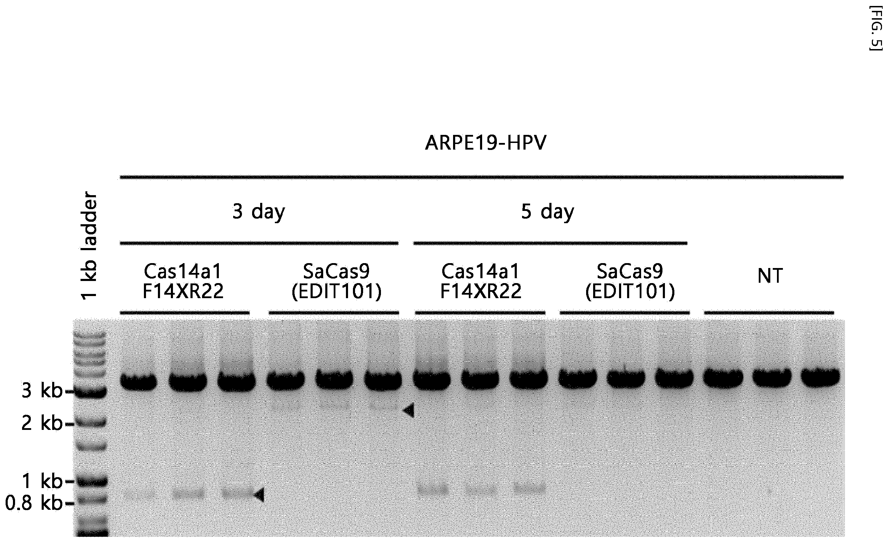

In addition, as a result of identifying deletion by transfection into ARPE19-HPY, although poor transfection efficiency was observed, from the viewpoint that when determined by intensity of deletion band, Cas14a1 and F14 X R22 showed higher intensity than EDIT101 despite its smaller size of deletion band, it was identified that Cas14a1 and F14 X R22 showed higher efficiency than EDIT101 (FIG. 5).

### Example 3. Selection of guide sequence

A nucleic acid sequence encoding each of the guide RNAs designed based on the selected protospacer sequences was prepared as a cassette (PCR amplicon), and transfected into HEK-293T cells using FuGENE HD along with a Cas14al vector. Here, the guide sequences of the guide RNAs are the same as in Tables 3 and 4, and 5'-ACCGCUUCACCAAAAGCUGUCCCUUAGGGGAUUAGAACUUGAGUGAAG GUGGGCUGCUUGCAUCAGCCUAAUGUCGAGAAGUGCUUUCUGGAAAGU AACCCUCGAAACAAAGAAAGGAAUGCAAC-3' (SEQ ID NO: 125) was used as a scaffold sequence of the guide RNAs. In addition, the guide RNA was designed to contain the sequence 5'-U₄AU₄-3', which is a U-rich tail sequence, at the 3' end. Using gDNA from the cells that were 60 to 72 hours after the transfection, indel efficiency was checked by NGS. As a result of the analysis, guide RNAs having the guide sequences F10, F14, F16, F17, R05, R07, R18, R20, R22, and R26, respectively, showed high indel efficiency.

**[Table 24] Indel efficiency (%) caused by guide RNAs having respective guide sequences and Cas14a1**

| Target region | Name | Guide sequence (20nt) | SEQ ID NO | NGS | %Indel |
|---|---|---|---|---|---|
| | Guide-F01 | UAGAAAAUUAUGCCUAUUUA | 51 | O | 0.01 |
| | Guide-F02 | GCAAAAGCAGCAGAAAGCAA | 52 | O | 0 |
| | Guide-F03 | GUAGGAAUCCUGAAAGCUAC | 53 | O | 0.01 |
| | Guide-F04 | CUGAGGACAGAACAAGCUCC | 54 | O | 0.14 |
| | Guide-F05 | GGAAAAUGAUAACGUUAAAC | 55 | O | 0.01 |
| | Guide-F06 | CUGAAUGUGUCUCUAUGAGC | 56 | O | 0.05 |
| | Guide-F07 | UUCAUCUUCCUCAUCAGAAA | 57 | O | 0 |
| | Guide-F08 | GAUUAUUCUUAUCUAAGAUC | 58 | O | 0.01 |
| | Guide-F09 | UCAAUAUUAUUUAGAAGUAA | 59 | O | 0 |
| | **Guide-F10** | **CAGAGUGCAUCCAUGGUCCA** | **60** | O | **34.1** |
| | Guide-F11 | UUCCUUGGAAUGGAUGUAGC | 61 | O | 1.44 |
| F region | Guide-F12 | ACCAGACAUCUAAGAGAAAA | 62 | O | 0.42 |
| | Guide-F13 | UUCUACAUUAAAAUUCUACA | 63 | O | 0.01 |
| | **Guide-F14** | **UUCUGGGUACAGGGGUAAGA** | **64** | O | **43.12** |
| | Guide-F15 | AUUCAGCUAAAUCAUGCAAG | 65 | O | 2.82 |
| | **Guide-F16** | **UAGUGGCUGAAUGACUUCUG** | **66** | O | **26.74** |
| | **Guide-F17** | **UUCCCACAUAACACACACAC** | **67** | O | **26.33** |
| | Guide-F18 | UAACUAAACUGAAUGAAUAA | 68 | O | 0.42 |
| | Guide-F19 | AUUAAAAUCUGCCAAAUCCC | 69 | O | 0.31 |
| | Guide-F20 | UGUUUGGGGGGAUUUGGCAG | 70 | O | 0.25 |
| | Guide-F21 | UGUGAUUCUUGAACCUUGUG | 71 | O | 0.02 |
| | Guide-F22 | UUCUACAAUAAAAAAUGAUG | 72 | O | 0 |
| | Guide-F23 | CUGGCUCAUAGAGACACAUUC | 73 | O | 0.97 |
| | Guide-F24 | AGCUAAUAGAAUUAGAUCUUA | 74 | O | 0.75 |

**[Table 25] Indel efficiency (%) caused by guide RNAs having respective guide sequences and Cas14a1**

| Target region | Name | Guide sequence (20nt) | SEQ ID NO | NGS | %Indel |
|---|---|---|---|---|---|
| | Guide-R01 | AGAGGUAAAGGUUCAUGAGA | 75 | O | 1.39 |
| | Guide-R02 | GCACAGAGUUCAAGCUAAUA | 76 | O | 0.04 |
| | Guide-R03 | CAUAUCUGUCUUCCUUAAUG | 77 | O | 1.47 |
| | Guide-R04 | AUGGAACAAAGUCACUCCUG | 78 | O | 1.53 |
| | **Guide-R05** | **CCCUUCAGGUAACCGGUAGC** | **79** | O | **10.12** |
| | Guide-R06 | GAAUGAUCAUUCUUGUGGCA | 80 | O | 0.01 |
| | **Guide-R07** | **AGGCGGGGAGUCACAUGGGA** | **81** | O | **15.85** |
| | Guide-R08 | CCUCUCAAAAAGCAACAAUA | 82 | O | 0.13 |
| | Guide-R09 | AAACAGGAAUAGAAAUUCAC | 83 | O | 0.01 |
| | Guide-R10 | UUCCAUUAAAAAAAGUAUGC | 84 | O | 0.03 |
| | Guide-R11 | AUUUGUUUCUGUGUGUUGAG | 85 | O | 0 |
| | Guide-R12 | UAUUUCCUUAUUAUCUAUUC | 86 | O | 0 |
| R region | Guide-R13 | AUAGUAGAAGAAAAAAGAAA | 87 | O | 0 |
| | Guide-R14 | AGAUCCCCUUGAAACACAGG | 88 | O | 0 |
| | Guide-R15 | CAAUUCCUGUGUUUCAAGGG | 89 | O | 0.02 |
| | Guide-R16 | UCACUGAAAUCCAUUGCUUA | 90 | O | 0.04 |
| | Guide-R17 | UUUAUCACUGAAAUCCAUUG | 91 | O | 0.02 |
| | **Guide-R18** | **AGUGGAGUACCUGGAAAAUA** | **92** | O | **26.76** |
| | Guide-R19 | GUCUCAUUACCCUGUUUGUA | 93 | X | - |
| | **Guide-R20** | **UAUACUAUGAAUACACAGGA** | **94** | O | **17.61** |
| | Guide-R21 | UAACAAACAAUAGGAUUAUC | 95 | O | 0.02 |
| | **Guide-R22** | **GAGUUACUAGGGAGAGAGUG** | **96** | O | **32.09** |
| | Guide-R23 | AAGGAGACCAGUGAAGUUUC | 97 | O | 0.24 |
| | Guide-R24 | GAUCUCAGGUAAUCCGCCAG | 98 | O | 0 |
| | Guide-R25 | AGUAAAGUACAUAAUUAUAG | 99 | O | 0.51 |
| | **Guide-R26** | **AGACCAGCCCGGCCAACAUG** | **100** | O | **7.9** |

Nucleic acids encoding the selected 10 guide RNAs were vectorized into cassettes through T-blunt end cloning, and indel efficiency thereof was checked by NGS. After vectorization, it was identified that the indel efficiency was increased. Among them, guide RNAs having the guide sequences F10, F14, R05, R07, R18, and R22, respectively, were selected in consideration of location from the c.2991+1655A>G gene mutation site and indel efficiency.

**[Table 26] Indel efficiency (%) caused by guide RNAs having respective guide sequences and Cas14a1**

| Target region | Name | Guide sequence (20nt) | SEQ ID NO | NGS | %Indel |
|---|---|---|---|---|---|
| F region | **Guide-F10** | **CAGAGUGCAUCCAUGGUCCA** | **60** | O | **62.12** |
| | **Guide-F14** | **UUCUGGGUACAGGGGUAAGA** | **64** | O | **60.66** |
| | Guide-F16 | UAGUGGCUGAAUGACUUCUG | 66 | O | 53.9 |
| | Guide-F17 | UUCCCACAUAACACACACAC | 67 | O | 51.6 |
| R region | **Guide-R05** | **CCCUUCAGGUAACCGGUAGC** | **79** | O | **10.87** |
| | **Guide-R07** | **AGGCGGGGAGUCACAUGGGA** | **81** | O | **19.88** |
| | **Guide-R18** | **AGUGGAGUACCUGGAAAAUA** | **92** | O | **61.41** |
| | Guide-R20 | UAUACUAUGAAUACACAGGA | 94 | O | 49.58 |
| | **Guide-R22** | **GAGUUACUAGGGAGAGAGUG** | **96** | O | **58.81** |
| | Guide-R26 | AGACCAGCCCGGCCAACAUG | 100 | O | 18.44 |

### Example 3-1. Optimization of guide sequence

The length of the guide sequences was adjusted using 19- to 25-mer protospacer sequences based on the PAM of each target. Cas14a1 ver.4.0 dual vectors containing nucleic acids encoding guide sequences of various lengths were constructed, and indel efficiency depending on the length of the guide sequence was checked (Tables 27 and 28).

**[Table 27] Indel efficiency (%) caused by guide RNAs having guide sequences of various lengths and Cas14al**

| Target region | Name | Guide sequence | SEQ ID NO | %Indel |
|---|---|---|---|---|
| | Guide-F10-19nt | CAGAGUGCAUCCAUGGUCC | 245 | 29.89 |
| | Guide-F10-20nt | CAGAGUGCAUCCAUGGUCCA | 60 | 25.98 |
| | Guide-F10-21nt | CAGAGUGCAUCCAUGGUCCAG | 246 | 30.23 |
| | Guide-F10-22nt | CAGAGUGCAUCCAUGGUCCAGG | 247 | 34.55 |
| | Guide-F10-23nt | CAGAGUGCAUCCAUGGUCCAGGA | 248 | 29.69 |
| | Guide-F10-24nt | CAGAGUGCAUCCAUGGUCCAGGAA | 249 | 28.23 |
| F region | Guide-F10-25nt | CAGAGUGCAUCCAUGGUCCAGGAAG | 250 | 17.8 |
| | Guide-F14-19nt | UUCUGGGUACAGGGGUAAG | 251 | 30.98 |
| | Guide-F14-20nt | UUCUGGGUACAGGGGUAAGA | 64 | 45.89 |
| | Guide-F14-21nt | UUCUGGGUACAGGGGUAAGAG | 252 | 33.98 |
| | Guide-F14-22nt | UUCUGGGUACAGGGGUAAGAGA | 253 | 36.91 |
| | Guide-F14-23nt | UUCUGGGUACAGGGGUAAGAGAA | 254 | 30.22 |
| | Guide-F14-24nt | UUCUGGGUACAGGGGUAAGAGAAA | 255 | 35.21 |
| | Guide-F14-25nt | UUCUGGGUACAGGGGUAAGAGAAAG | 256 | 25.35 |

**[Table 28] Indel efficiency (%) caused by guide RNAs having guide sequences of various lengths and Cas14al**

| Target region | Name | Guide sequence | SEQ ID NO | %Indel |
|---|---|---|---|---|
| | Guide-R05-19nt | CCCUUCAGGUAACCGGUAG | 257 | 3.86 |
| | Guide-R05-20nt | CCCUUCAGGUAACCGGUAGC | 79 | 8.51 |
| | Guide-R05-25nt | CCCUUCAGGUAACCGGUAGCUUUUG | 258 | 5.19 |
| | Guide-R07-19nt | AGGCGGGGAGUCACAUGGG | 259 | 13.47 |
| | Guide-R07-20nt | AGGCGGGGAGUCACAUGGGA | 81 | 15.18 |
| | Guide-R07-21nt | AGGCGGGGAGUCACAUGGGAG | 260 | 16.17 |
| | Guide-R07-23nt | AGGCGGGGAGUCACAUGGGAGUC | 261 | 16.27 |
| | Guide-R07-24nt | AGGCGGGGAGUCACAUGGGAGUCA | 262 | 14.55 |
| | Guide-R07-25nt | AGGCGGGGAGUCACAUGGGAGUCAC | 263 | 14.02 |
| R region | Guide-R18-20nt | AGUGGAGUACCUGGAAAAUA | 92 | 33.95 |
| | Guide-R18-21nt | AGUGGAGUACCUGGAAAAUAA | 264 | 37.51 |
| | Guide-R18-22nt | AGUGGAGUACCUGGAAAAUAAG | 265 | 35.78 |
| | Guide-R18-23nt | AGUGGAGUACCUGGAAAAUAAGC | 266 | 34.25 |
| | Guide-R18-24nt | AGUGGAGUACCUGGAAAAUAAGCC | 267 | 27.9 |
| | Guide-R18-25nt | AGUGGAGUACCUGGAAAAUAAGCCA | 268 | 21.53 |
| | Guide-R22-20nt | GAGUUACUAGGGAGAGAGUG | 96 | 37.03 |
| | Guide-R22-21nt | GAGUUACUAGGGAGAGAGUGA | 269 | 33.96 |
| | Guide-R22-23nt | GAGUUACUAGGGAGAGAGUGAUG | 270 | 34.37 |
| | Guide-R22-24nt | GAGUUACUAGGGAGAGAGUGAUGA | 271 | 35.54 |
| | Guide-R22-25nt | GAGUUACUAGGGAGAGAGUGAUGAA | 272 | 38.9 |

### Example 4. Production of ARPE19/HPV16-LCA10 cell line

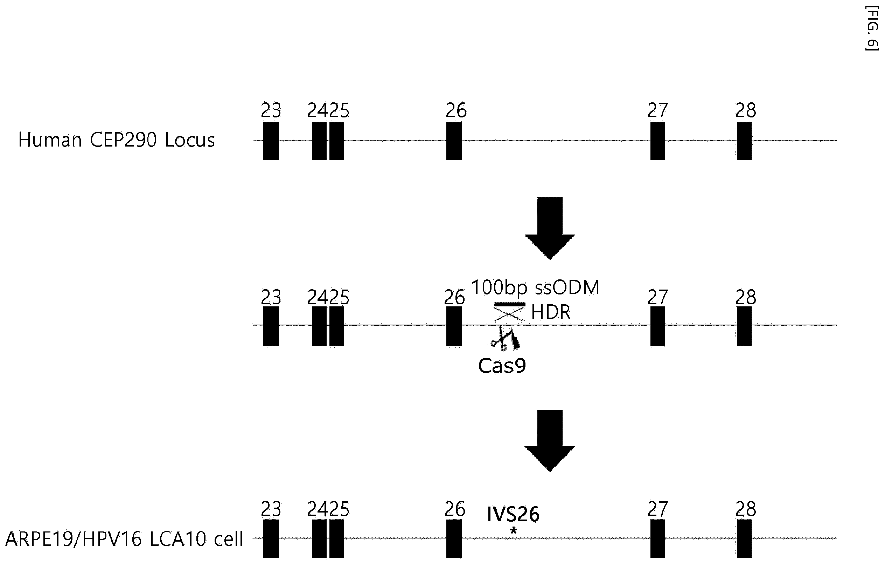

To identify effects obtained by artificially manipulating the CEP290 gene having the IVS26 mutation using the guide RNAs selected through the above-described screening, an ARPE19/HPV16-LCA10 cell line containing the CEP290 gene having the IVS26 mutation was produced. To briefly describe a strategy for producing the ARPE19/HPV16-LCA10 cell line, an experiment was designed in such a way that a CRIPSR/Cas9 system and a single-strand oligonucleotide (ssODN) were used to introduce a point mutation (c.2991+1655A>G) into intron 26 of the CEP290 locus (FIG. 6). To this end, 5'-ATCCCTATTGGCAGTGTCTT-3' (SEQ ID NO: 273) was used as a target sequence for the CRIPSR/Cas9 system, and GAGCCACCGCACCTGGCCCCAGTTGTAATTGTGAGTATCTCATAGATATCCC TATTGGCAGTGTCTTAGTTTTATTTTTTATTATCTTTATTGTGGCAGC-3' (SEQ ID NO: 274) was used as a donor sequence, that is, ssODN. Here, the underlined sequence in the donor sequence is a point mutation. To facilitate selection, a puromycin resistance gene was inserted together into a vector for expression of the CRISPR/Cas9 system used in the experiment. By transfecting the expression vector for the CRISPR/Cas9 system and ssODN together into ARPE19 cells, the target sequence was cleaved by the CRISPR/Cas9 system and repaired by HDR using ssODN, which caused a point mutation (c.2991+1655A) to be introduced into intron 26 of the CEP290 locus of interest.

The thus produced ARPE19/HPV16-LCA10 cells were first checked by PCR using the primer set as shown in Table 20. As a result, a band of 397 bp was identified in the candidate cells A2, A3, A7, C1, C2, C5 to C7, E8 to E12, and F1 to F12 (FIG. 7). The PCR products of the corresponding candidate cells were purified and a request for sequencing analysis thereof was made to Macrogen. As a result of the sequencing analysis, the c.2991+1655A>G mutation was identified in C7 cells (AC7 cells). For the other cells, deletion and insertion were identified in a site located after CCT that is a target portion of Cas9 (FIG. 8).

### Example 4-1. LCA10-AC7 clone expression analysis

RNA was extracted from a clone of the previously obtained AC7 cells (hereinafter referred to as LCA10-AC7 cells) and the ARPE19/HPV16 (WT) cells, and cDNA was synthesized therefrom (RNA extraction: Rneasy kit, Qiagen; cDNA synthesis: TOPscript RT DryMIX, enzynomics). RNA extraction and cDNA synthesis were performed according to the manufacturer's manual. The obtained cDNA was subjected to qRT-PCR and RT-PCR (1.5% agarose gel, 100v, electrophoresis for 20 minutes) using the following primers in an equal amount of 10 ng.

**[Table 29] Primer information for qRT-PCR**

| Target | Primer set | | Product size |
|---|---|---|---|
| Wt cDNA (exon 26) | CEP290 WT F#01 | CCATTTTCAAGATTGCAGCTCTCCAA (SEQ ID NO: 275) | 166 bp |
| | CEP290 WT R#01 | TCCAGGTGTTCCAAGTTACTTGTTCT (SEQ ID NO: 276) | |
| Mutation cDNA (exon 26~28) | CEP290 Mu F#02 | TGACTGCTAAGTACAGGGACATCTTG (SEQ ID NO: 277) | 173 bp |
| | CEP290 Mu R#02 | Atctgtaatcccagcactttaggagg (SEQ ID NO: 278) | |

**[Table 30] Primer information for RT-PCR**

| Target | Primer set | | Product size |
|---|---|---|---|
| 26-X-27 (26-27) | CEP290 WT F#02 | AGGAAATGGCCATTTTCAAGATTGCA (SEQ ID NO: 279) | 340 bp (212 bp) |
| | CEP290 cDNA Screening | AGACTCCACTTGTTCTTTTAAGGAG (SEQ ID NO: 280) | |

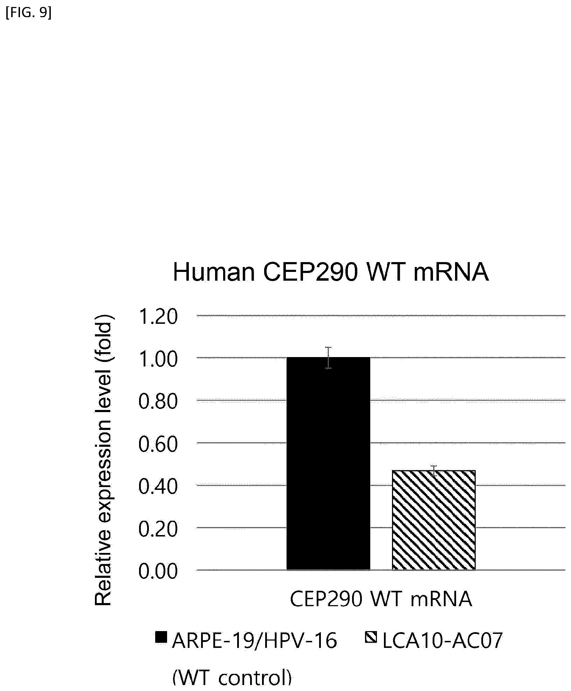

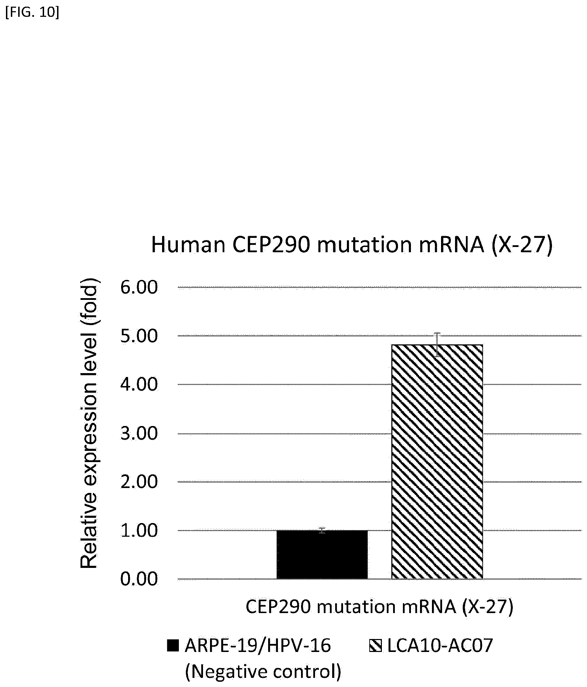

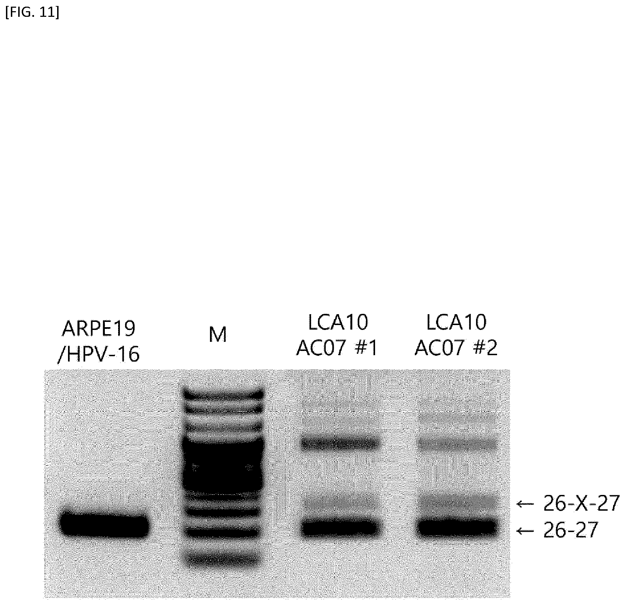

The qRT-PCR results were subjected to relative quantification with WT through the 2^-ddCt method. ARPE-19/HPV16 CEP290 WT mRNA and LCA10-AC07 CEP290 WT mRNA were compared (FIG. 9), and ARPE-19/HPV16 CEP290 mutation mRNA (X-27) and LCA10-AC07 CEP290 mutation mRNA (X-27) were compared (FIG. 10). As a result, due to generation of the C.2991+1655A>G mutation, it was identified that expression of the WT mRNA and the mutantion mRNA showed a different tendency from ARPE-19/HPV16 WT. In a case where cryptic exon X was expressed due to generation of the C.2991+1655A>G mutation, a ratio of WT decreased and a ratio of mutation mRNA increased among the total CEP290 mRNA. Therefore, in a case where the corresponding portion is corrected with a genetic scissor, a ratio of WT increases and a ratio of mutation mRNA decreases, again (Morgan L. Maeder et al., Nature Medicine, 25, 229-233 (2019)). In addition, as a result of RT-PCR, the band 26-27 (212 bp) was detected in both WT and the LCA10-AC7 cells, and the band 26-X-27 (340 bp) was detected only in the LCA10-AC7 cells, indicating that the cryptic exon X was generated by a point mutation (c.2991+1655A>G) in intron 26 of the CEP290 locus in the LCA10-AC7 cells (FIG. 11).

### Example 4-2. Additional production of ARPE19/HPV16-LCA10 cell line

Since it is difficult to identify accuracy of the in vitro assay using only the previously obtained LCA10-AC7 cells, additional production was performed to obtain additional ARPE19/HPV16-LCA10 cells. The production method is the same as the previously described production method. As a result of the additional production, a point mutation (c.2991+1655A>G) was identified in intron 26 of the CEP290 locus in LCA10-BC8 cells and LCA10-BD5 cells (FIG. 12).

RNA was extracted from each clone of the obtained LCA10-AC7 cells, LCA10-BC8 cells, and LCA10-BD5 cells and from the ARPE19/HPV16 (WT) cells, and cDNA was synthesized therefrom (RNA extraction: Rneasy kit, Qiagen; cDNA synthesis: TOPscript RT DryMIX, enzynomics). RNA extraction and cDNA synthesis were performed according to the manufacturer's manual. The obtained cDNA was subjected to qRT-PCR and RT-PCR (1.5% agarose gel, 100v, electrophoresis for 20 minutes) using the following primers in an equal amount of 10 ng. The RT-PCR-related primer information is the same as the primer information in Table 29.

**[Table 31] qRT-PCR-related primer information**

| Target | Primer set | | Product size |
|---|---|---|---|
| Mutation cDNA (exon 26~28) | CEP290 Mu F#03 | tagagatggggtttcaccttgttagc (SEQ ID NO: 281) | 164 bp |
| | CEP290 cDNA Screening | AGACTCCACTTGTTCTTTTAAGGAG (SEQ ID NO: 282) | |

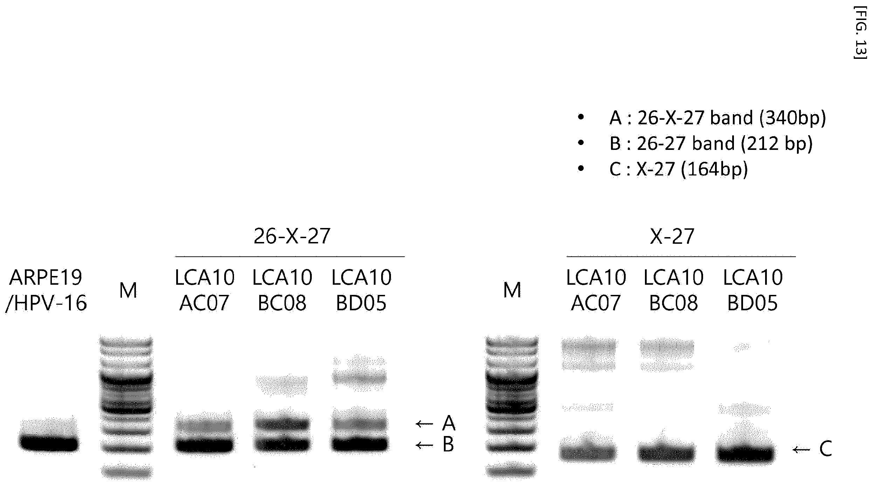

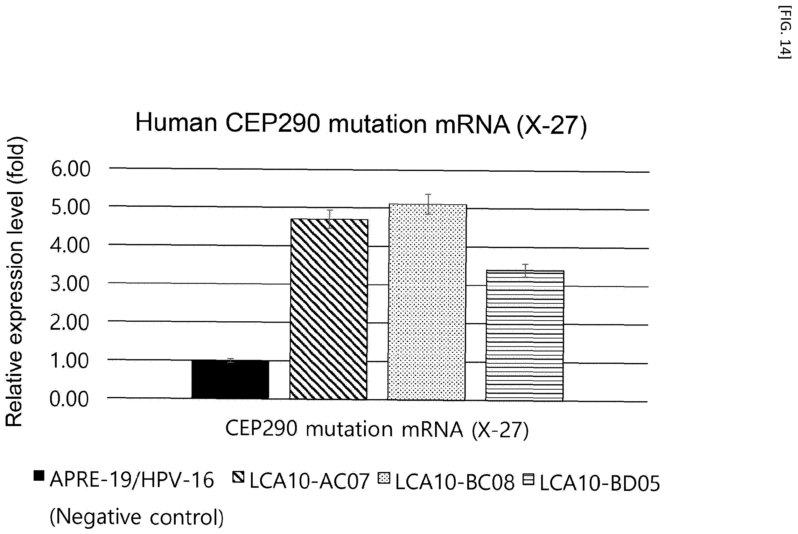

As a result of RT-PCR, the band 26-27 (212 bp) was detected in all of WT, the LCA10-AC7 cells, the LCA10-BC8 cells, and the LCA10-BD5 cells; the band 26-X-27 (340 bp) was detected only in the LCA10-AC7 cells, the LCA10-BC8 cells, and the LCA10-BD5 cells; and the band X-27 (164 bp) was also detected in the LCA10-AC7 cells, the LCA10-BC8 cells, and the LCA10-BD5 cells. From the results, it was identified that cryptic exon X was generated by a point mutation (c.2991+1655A>G) in intron 26 of the CEP290 locus in the LCA10-AC7 cells, the LCA10-BC8 cells, and the LCA10-BD5 cells (FIG. 13). In addition, an expression level of cryptic exon X was checked in the LCA10-BC8 cells and the LCA10-BDS cells. Here, each of the expression levels was compared relative to the expression of the cryptic exon X mRNA of the LCA10-AC7 cells that were produced first. As a result, it was identified that the LCA10-BC8 cells showed slightly increased expression of cryptic exon X as compared with the LCA10-AC7 cells, whereas the LCA10-BD5 cells showed approximately 30% lower expression of cryptic exon X than the LCA10-AC7 cells (FIG. 14).

### Example 5. Identification of efficiency of LCA10 Cas12f1 therapeutic agent

As a result of performing comparative analysis of efficiency of the dual gRNA vector and the one vector using the previously produced LCA10-AC7 cells, LCA10-BC8 cells, and LCA10-BD5 cells, the most significant result was obtained in the BC8 clone. Based on these results, efficiency was evaluated using the LCA10-BC8 cells to identify efficiency of a Cas12f1-based LCA10 therapeutic agent. Here, for the guide RNAs used in the experiment, a guide RNA having F14 as a guide sequence and a guide RNA having R22 as a guide sequence, which were previously selected through screening, were used. The two guide RNAs were intended to be used to remove the point mutation (c.2991+1655A>G) present in intron 26 of the CEP290 locus, thereby inhibiting generation of cryptic exon X.

To identify efficiency of the LCA10 Cas12f1 therapeutic agent, LCA10-BC8 cells were seeded one day before transfection so that the confluency reached 70% the next day. The next day, Cas12f1_LCA10_F14_vector (1 µg) + Cas12f1_LCA10_R22_vector (1 µg) + FuGENE HD 6 µg were mixed, and reaction was allowed to occur at room temperature for 20 minutes. Then, transfection was performed. After 72 hours, RNA was extracted from the transfected LCA10-BC8 cells and the ARPE19/HPV16 (WT) cells, and cDNA was synthesized therefrom (RNA extraction: Rneasy kit, Qiagen; cDNA synthesis: TOPscript RT DryMIX, enzynomics). RNA extraction and cDNA synthesis were performed according to the manufacturer's manual. The obtained cDNA was subjected to qRT-PCR and RT-PCR using the following primers in an equal amount of 10 ng. The RT-PCR-related primer information is the same as the primer information in Table 29, and the qRT-PCR-related primer information is the same as the primer information in Table 30. The qRT-PCR results were subjected to relative comparative quantification with WT through the 2^-ddCt method. The RT-PCR results were subjected to electrophoresis on 2% agarose gel (Etbr) at 135 V for 15 minutes. The cDNA was sequenced to identify a signal of the cryptic exon.

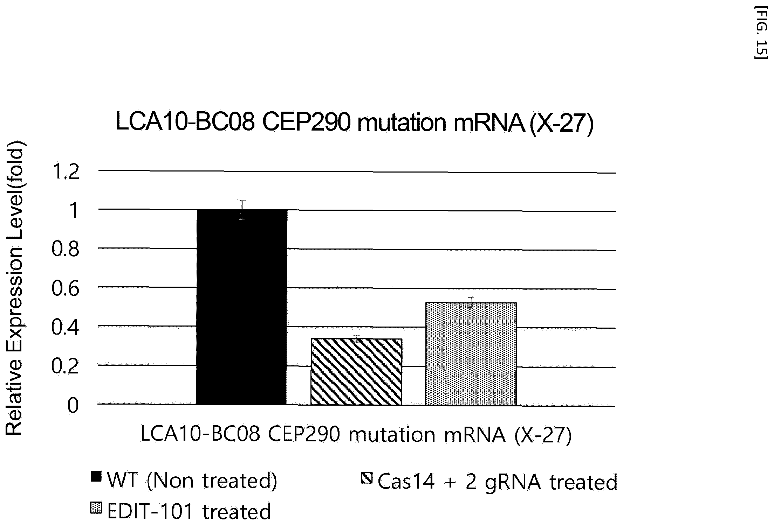

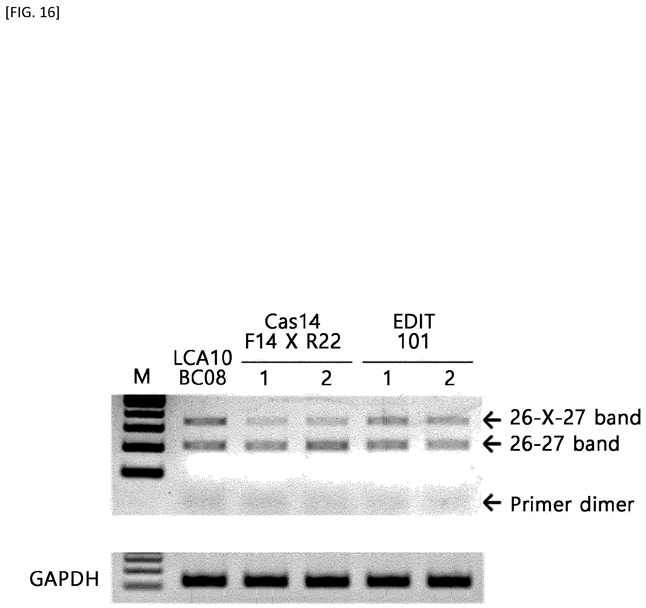

As a result, it was identified that the guide RNA having F14 as a guide sequence and the guide RNA having R22 as a guide sequence resulted in decreased expression of X-27 of the CEP290 gene (FIG. 15). In addition, it was identified that the two guide RNAs resulted in a decreased amount of the band 26-X-27 even in RT-PCR (FIG. 16). In addition, it was also identified that the two guide RNAs resulted in deletion of the target region. Here, EDIT101 was used as a comparator group.

### Example 6. Production and screening of 661W mouse LCA10 disease model cell line

### Example 6-1. Production of 661W mouse LCA10 disease model cell line

A 661W mouse LCA10 disease model cell line was produced. The procedure was performed similarly to the method described in the section <Production of ARPE19/HPV16-LCA10 cell line>, except that 661W mouse cells were used instead of ARPE19/HPV16 cells. In addition, unlike the ARPE19 cells for which introduction of a point mutation was performed, in a case of the 661W mouse cells, insertion of the entire sequence of human exon 26 to exon 27, which contains the IVS26 mutation, was performed using a homology-directed repair (HDR) method.

Specifically, exon 25 and exon 26 of the mouse CEP290 gene were targeted to delete the corresponding sequences, and insertion of the entire sequence of human exon 26 to exon 27, which contains the IVS26 mutation, was performed with a donor sequence of SEQ ID NO: 476 through the repair (HDR) method. In addition, instead of the puromycin resistance gene used in Example 4, a neomycin resistance gene was inserted together into the right homology arm of the donor sequence to allow for selection.

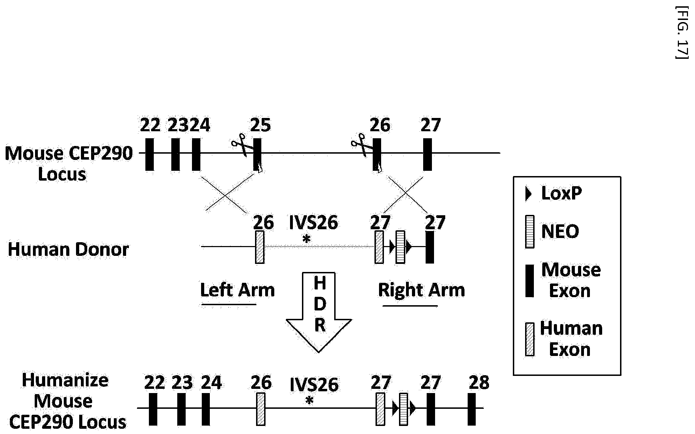

A schematic diagram for production of the 661W/LCA10 cells is shown in FIG. 17.

### Example 6-2. Screening of 661W/LCA10 cells

Screening of the 661W/LCA10 cells was performed similarly to the method described in the section <12. Screening of ARPE19/HPV16-LCA10 cells>.

Here, instead of puromycin, neomycin (600 µg/ml) was used to perform selection.

DNA of the selected clone was extracted, and it was identified whether the sequence insertion was properly performed through PCR using 1) Inner Primer F (5'-TGTGGCAGCCATTATTCCTGTCTCTA-3' (SEQ ID NO: 487)) and Inner Primer R (5'-GCAACACAGTGAGACTCTGTTTCAAA-3' (SEQ ID NO: 488)), 2) Left Arm Primer F (5'-TCTTCTGTGGCTATCATGAGAGCGTC-3' (SEQ ID NO: 491)) and Left Arm Primer R (5'-TGGGTACAGGGGTAAGAGAAAGGGAT-3' (SEQ ID NO: 492)), and 3) Right Arm Primer F (5'-GGAACTATGATTCAAGATGAGATTTGGGTA-3' (SEQ ID NO: 489)) and Right Arm Primer R (5'-TTGGACACTTCAACTTTGAGCTCC-3' (SEQ ID NO: 490)), respectively.

Then, Sanger sequencing was performed to identify whether the entire sequence was inserted correctly.

The cells containing the mutation were named according to the following nomenclature: A to H, numbers of 1 to 12 (A to H: characters corresponding to rows of a 96-well plate; numbers of 1 to 12: numbers corresponding to columns in respective rows, for example, E08: clone at row E, eighth column in a 96-well plate).

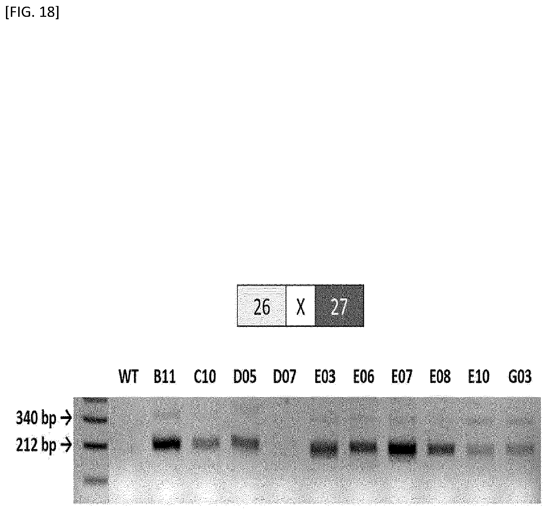

Then, for each clone, expression of 26-X-27 (X means cryptic exon X) was checked through RT-PCR (FIG. 18). The primer set used is as shown in Table 32.

**[Table 32]**

| Primer set | | | | |
|---|---|---|---|---|
| Target | PCR product | Primer name | Sequence | Product size |
| Human CEP290 | **26-X-27** | CEP290 WT F#02 | AGGAAATGGCCATTTTCAAGATTGCA (SEQ ID NO: 279) | 212 bp (340 bp) |
| exon 26-27 | | Screening R#02 | AGACTCCACTTGTTCTTTTAAGGAG (SEQ ID NO: 280) | |

Then, from the viewpoint that mouse cell lines show expression of Y and Z in addition to cryptic exon X, in order to select a cell line with high expression of cryptic exon X, expression of X-27 was checked through RT-PCR and the optimal clone was selected. The primer set used is as shown in Table 33.

**[Table 33]**

| Primer set | | | | |
|---|---|---|---|---|
| Target | PCR product | Primer name | Sequence | Product size |
| Human CEP290 | **X-27** | CEP290 Mu F#3 | tagagatggggtttcaccttgttagc (SEQ ID NO: 281) | 164 bp |
| cryptic exon X | | CEP290 cDNA Screening R#02 | AGACTCCACTTGTTCTTTTAAGGAG (SEQ ID NO: 280) | |

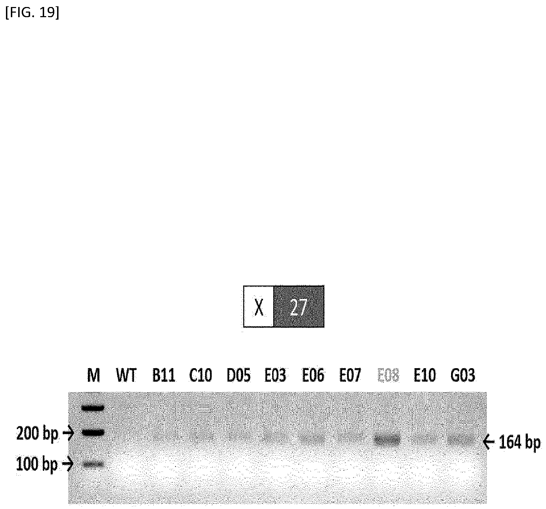

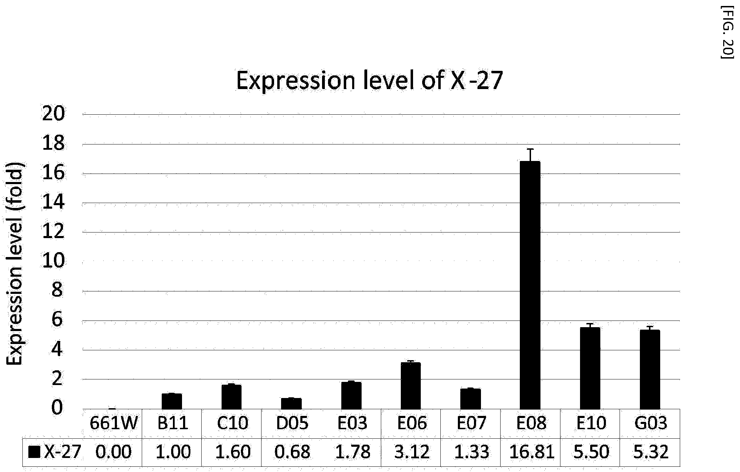

As a result of the experiment, the clone 661W/LCA10-E08 having the highest expression level of X-27 was selected and used for the experiments (FIGS. 19 and 20).

### Example 7. Identification of deletion of partial region of CEP290 gene for 661W/LCA10 cell line

For the 661W/LCA10-E08 cell line produced in Example 6, deletion of a partial region of the CEP290 gene caused by the LCA10 Cas12f1 therapeutic agent was identified.

Specifically, the procedure was performed similarly to the method described in the section <Example 2-1. Identification of deletion of partial region in CEP290 gene>, except that 1) a one-vector system was used which is capable of expressing all of a Cas12f1 (Cas14a1) protein, a guide RNA targeting the F region, and a guide RNA targeting the R region in a form of a single vector, 2) the 661W/LCA10-E08 cells produced in Example 6 were used instead of the HEK-293T cells, and 3) the guide sequences F14 X R18 and F14 X R22 were used to identify the deletion.

The primer sets used for RT-PCR are as shown in Table 34.

**[Table 34]**

| Primer set | | | |
|---|---|---|---|
| Target | Primer name | Sequence | Product size |
| Human CEP290 | CEP290 qPCR F#1 | TGGACCAGACCCTTTGTAGTTATCTT (SEQ ID NO: 483) | 197 bp |
| | CEP290 qPCR R#1 | TGAGCCAGCAAAAGCTTTTGAGCT (SEQ ID NO: 484) | |
| | CEP290 64 qPCR F#01 | CTTACATCCTCCTTACTGCCACAAGA (SEQ ID NO: 485) | 195 bp |
| | CEP290 64 qPCR R#01 | actgtggtcagaaaactcagctagtt (SEQ ID NO: 486) | |

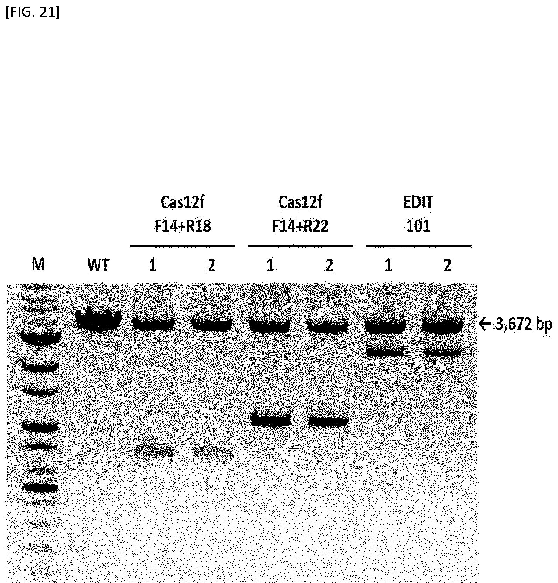

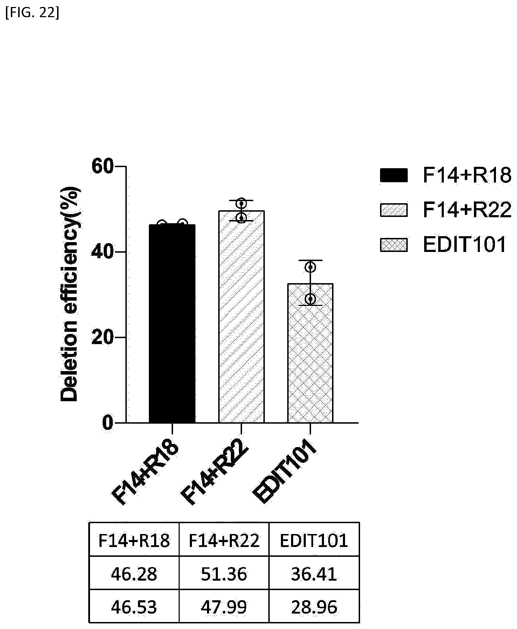

As a result of the experiment, upon comparison of signal intensity between the band of WT, which is a negative control with no treatment, and the bands of the same size, it was identified that the signal intensity of the bands of Cas12f1 F14 + R18 and Cas12f1 F14 + R22 was lower than EDIT101 (FIG. 21). In addition, as a result of identifying deletion through q-PCR, it was found that deletion efficiency of Cas12f1 F14+R18 and Cas12f1 F14+R22 was higher than EDIT101 (FIG. 22).

### Example 8. Target screening for Cas12f1 variants

### Example 8-1. Target screening 1 for Cas12f1 variants

In order to screen an efficient target sequence for the Cas12f1 variant of SEQ ID NO: 288 (transposon-associated transposase B derived from Candidatus Woesearchaeota archaeon; also known as TnpB), an experiment was conducted similarly to that described in the section <Example 2. Selection of guide sequence>.

Here, the experiment was conducted in such a way that 1) 5'-ACCGCTTCACCAAAAGCTGTCCCTTAGGGGATTAGAACTTGAGTGAAGGTG GGCTGCTTGCATCAGCCTAATGTCGAGAAGTGCTTTCTTCGGAAAGTAACC CTCGAAACAAAGAAAGGAATGCAAC-3' (SEQ ID NO: 477) was used as a scaffold sequence of the guide RNAs, 2) 5'-UUUUAUUUU-3' was used as a U-rich tail sequence, and 3) F10, F11, F12, F14, F15, F16, F17, F18, F23, F24, R01, R03, R04, R05, R07, R18, R20, R22, R23, and R26 in Tables 3 and 4 were used as guide sequences, respectively.

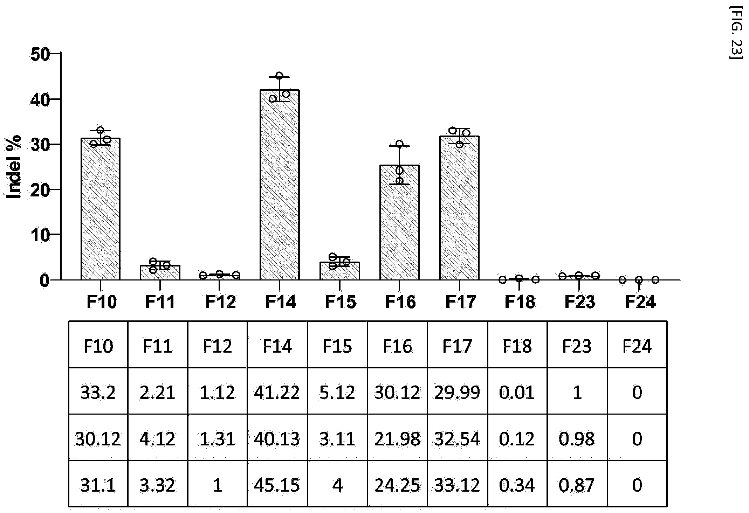

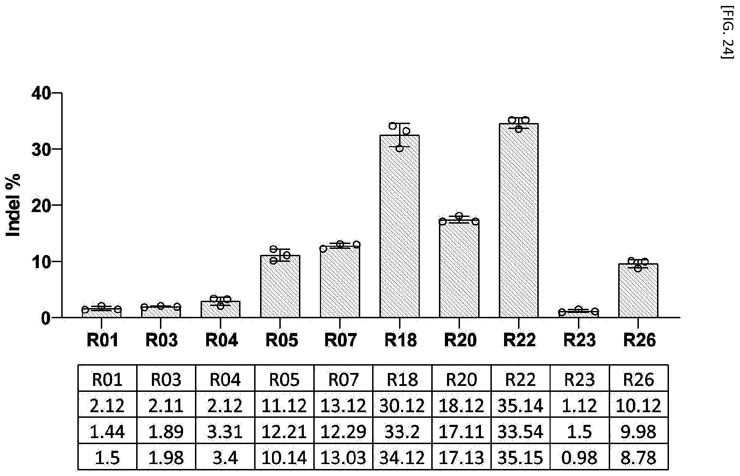

As a result of the experiment, indel efficiency was observed in most of the targets except F24, and in particular, high indel efficiency was observed in F10, F14, F16, F17, R18, and R22 (FIGS. 23 and 24).

### Example 8-2. Target screening 2 for Cas12f1 variants

For the targets F14, R18, and R22, which showed high indel efficiency in Example 8-1, different guide sequences were compared in terms of efficiency.

Specifically, an experiment was conducted similarly to that described in the section <Example 2. Selection of guide sequence>, except that 1) each of 5'-ACCGCTTCACCAAAAGCTGTCCCTTAGGGGATTAGAACTTGAGTGAAGGTG GGCTGCTTGCATCAGCCTAATGTCGAGAAGTGCTTTCTTCGGAAAGTAACC CTCGAAACAAAGAAAGGAATGCAAC-3' (SEQ ID NO: 477) (denoted as ver. 4.0), and 5'-ACCGCUUCACCGAGUGAAGGUGGGCUGCUUGCAUCAGCCUAAUGUCGAG AAGUGCUUUCUUCGGAAAGUAACCCUCGAAACAAAgaaaGGAAUGCAAC-3' (SEQ ID NO: 478) (denoted as ver 4.1) was used as a scaffold sequence of the guide RNAs, 2) 5'-UUUUAUUUU-3' was used as a U-rich tail sequence, and 3) F14, F18, and R22 in Tables 3 and 4 were used as guide sequences, respectively.

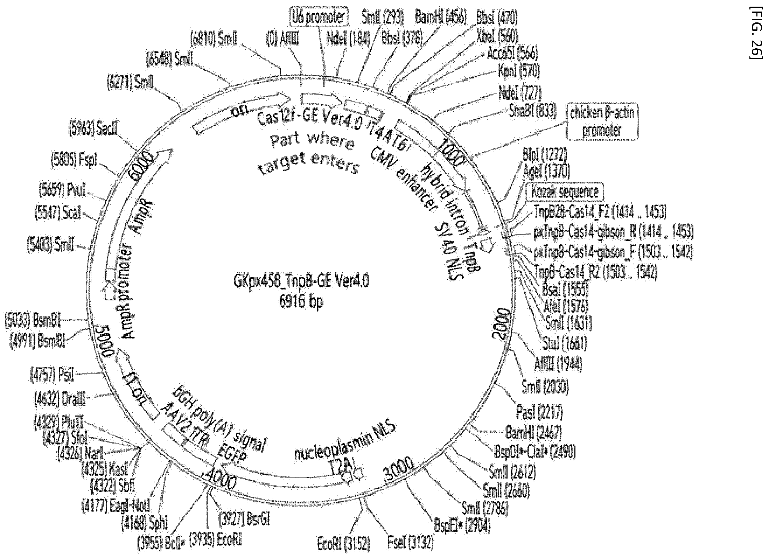

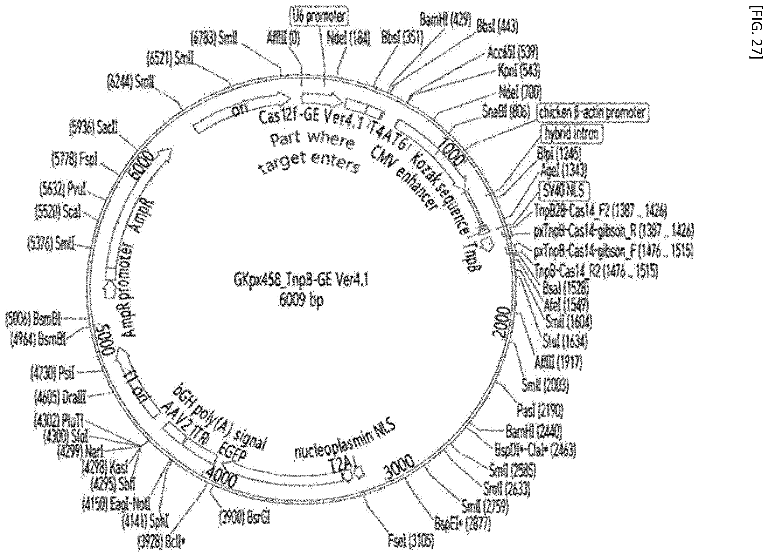

In addition, a one-vector system expressing the Cas12f1 variant of SEQ ID NO: 288 and a guide RNA was constructed and used for transfection. Specifically, the guide sequences were cloned using the px458-TnpB-GE vector (TnpB, gRNA scaffold Bbsl cloning) and the Bbsl enzyme, and used (FIGS. 26 and 27).

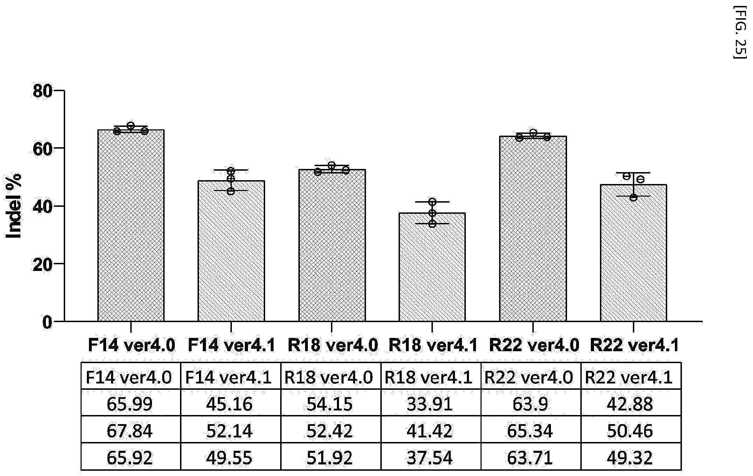

As a result of the experiment, significant indel efficiency was observed for both the guide RNA scaffolds of SEQ ID NO: 477 (ver 4.0) and SEQ ID NO: 478 (ver. 4.1); and in general, it was found that slightly higher indel efficiency was observed in a case where the guide RNA scaffold of SEQ ID NO: 477 was used (FIG. 25).

### Example 8-3. Target screening 3 for Cas12f1 variants

In order to optimize the length of the guide sequence, an experiment was conducted similarly to that described in the section <Example 3-1. Optimization of guide sequence>.

Specifically, the experiment was conducted in such a way that 1) nucleic acids encoding the Cas12f1 variant of SEQ ID NO: 288, the guide RNA scaffold sequence of SEQ ID NO: 477, and the U-rich tail of 5'-UUUUAUUUU-3' were used, 2) guide sequences were cloned using the px458-TnpB-GE vectors (TnpB, gRNA scaffold Bbsl cloning), which contain nucleic acids encoding guide sequences of various lengths, and the Bbsl enzyme, and 3) among the guide sequences shown in Tables 27 and 28, each of the guide sequences of Guide-F14-19nt to Guide-F14-25nt, Guide-R18-20nt to Guide-R18-25nt, and Guide-R22-20nt to Guide-R22-25nt was used.

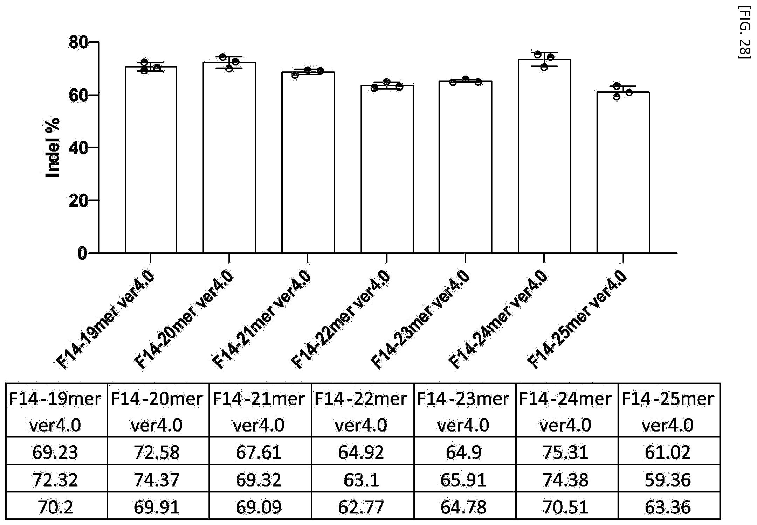

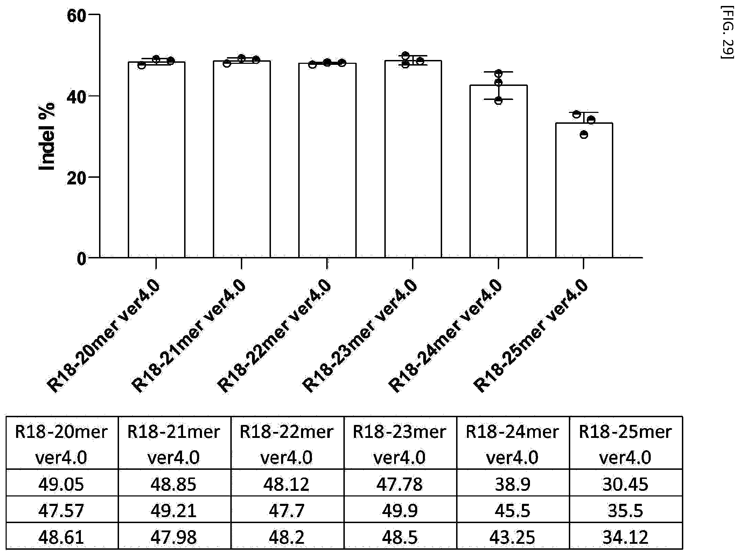

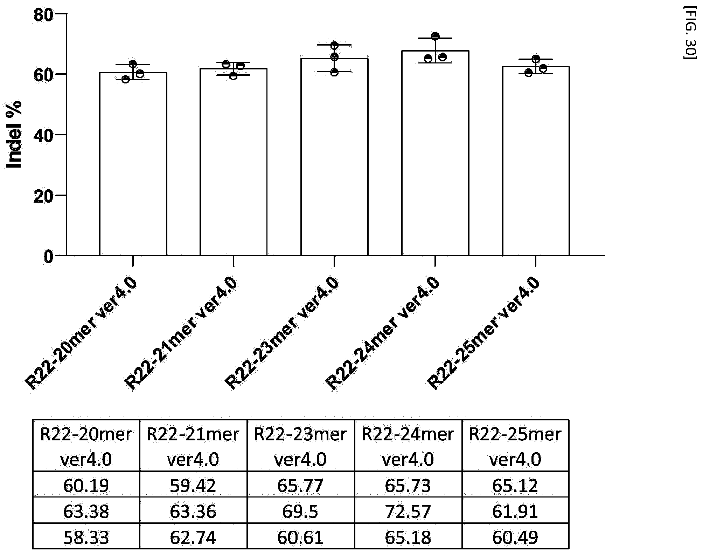

As a result of the experiment, all showed high activity for various lengths of spacer (FIGS. 28 to 30).

### Example 9. Identification of efficiency of LCA10 Cas12f1 variant therapeutic agent

In order to identify efficiency of the Cas12f1 variant-based LCA10 therapeutic agent based on the results of Example 8, evaluation of efficiency was performed using the ARPE19/LCA10-BC08 cell line and the 661W/LCA10-E08 cell line.

Specifically, an experiment was performed similarly to that described in the section <Example 5. Identification of efficiency of LCA10 Cas12f1 therapeutic agent>, except that 1) the ARPE19/LCA10-BC08 cells and the 661W/LCA10-E08 cells were used, 2) the px458-TnpB-GE vector (TnpB, gRNA scaffold Bbsl cloning), which contains a nucleic acid encoding the Cas12f1 variant of SEQ ID NO: 288 and a nucleic acid encoding two guide RNAs, was used, 3) each guide RNA had, as components, the guide RNA scaffold sequence of SEQ ID NO: 477 and the U-rich tail of 5'-UUUUAUUUU-3', and 4) (F14 and F18) and (F14 and R22) were used as combinations of the two guide RNAs.

Here, the method of identifying an expression level of X-27 for the ARPE19/LCA10-BC08 cell line was the same as that described in the section <Example 5. Identification of efficiency of LCA10 Cas12f1 therapeutic agent>, and the method of identifying an expression level of X-27 for 661W/LCA10-E08 was the same as that described in the section <Example 6-2. Screening of 661W/LCA10 cells>.

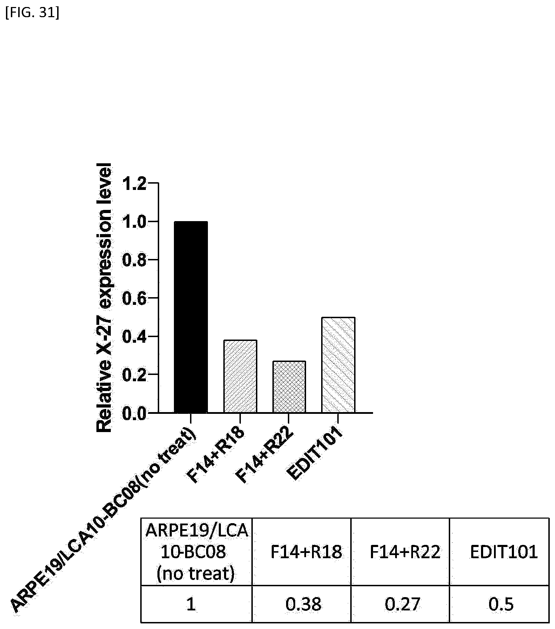

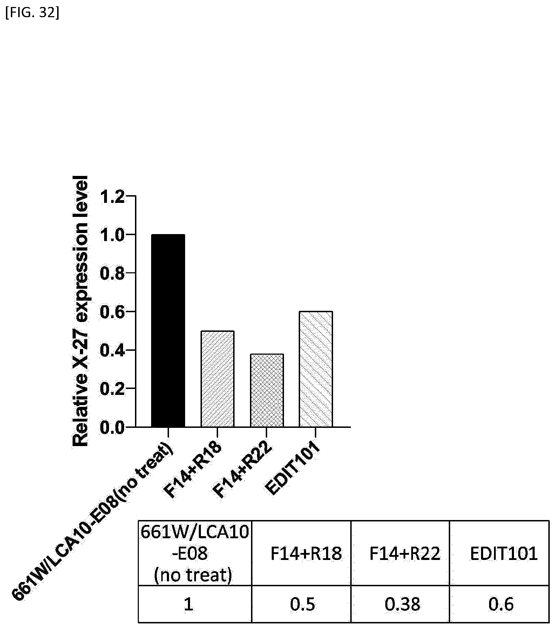

As a result of the experiment, it was found that the LCA10 Cas12f1 variant therapeutic agent of the present disclosure significantly decreased an expression level of X-27 in both the ARPE19/LCA10-BC08 cell line and the 661W/LCA10-E08 cell line, and showed a superior effect to that of the comparator group EDIT101 (FIGS. 31 and 32).

### Example 10. Identification of effect of improving deletion efficiency, caused by factor for decreasing NHEJ activity

This example was intended to identify an effect of improving deletion efficiency in a case of additionally using a factor for decreasing non-homologous end joining DNA repair pathway (NHEJ) activity together with a LCA10 Cas12f1 therapeutic agent and a LCA10 Cas12f1 variant therapeutic agent.

An experiment was conducted in HEK293T cells similarly to that described in the section <Example 2-1. Identification of deletion of partial region in CEP290 gene>.

Here, each of the following one-vector systems was used: 1) a one-vector system that contains a Cas12f1 protein, a guide RNA targeting F14, a guide RNA targeting R22, and a nucleic acid encoding a factor for decreasing NHEJ activity, and 2) a one-vector system that contains the Cas12f1 protein variant of SEQ ID NO: 288, a guide RNA targeting F14, a guide RNA targeting R22, and a nucleic acid encoding a factor for decreasing NHEJ activity.

Each of the guide RNAs contained the guide RNA scaffold region of SEQ ID NO: 477 and the U-rich tail of 5'-UUUUAUAUUUU-3'.

In addition, the experiment was conducted in such a way that for the factor for decreasing NHEJ activity used, one or two units of nucleic acid encoding shDCLRE1C-3 of SEQ ID NO: 473 were included in the vector, and shscramble was used as a comparator group.

As a result of the experiment, it was found that higher deletion efficiency was observed in a case where the factor for decreasing NHEJ activity was used together with the LCA10 Cas12f1 therapeutic agent or the LCA10 Cas12f1 variant therapeutic agent (FIG. 33).

## Claims

1. A guide RNA for a CRISPR/Cas12f1 system, comprising:
a scaffold region represented by a nucleotide sequence selected from SEQ ID NO: 125, SEQ ID NO: 126, SEQ ID NO: 477, and SEQ ID NO: 478;
a spacer region comprising, as a guide sequence, a nucleotide sequence selected from SEQ ID NOS: 51 to 100 and SEQ ID NOS: 245 to 272; and
a U-rich tail,
wherein the scaffold region, the spacer region, and the U-rich tail are sequentially linked to each other in a 5' to 3' direction,
the scaffold region is a region capable of interacting with a Cas12f1 protein,
the spacer region is capable of forming a complementary bond with a target sequence present in CEP290 gene, and
the U-rich tail is represented by a sequence selected from 5'-(UaN)dUe-3', 5'-UaVUaVUe-3', and 5'-UaVUaVUaVUe-3' wherein N is A, C, G or U, each V is independently A, C or G, a is an integer from 0 to 4, d is an integer from 0 to 3, and e is an integer from 1 to 10.

2. A DNA encoding the guide RNA of claim 1.

3. A guide RNA-Cas12f1 protein complex, comprising:
a first Cas12f1 protein represented by an amino acid sequence selected from SEQ ID NO: 129 or SEQ ID NO: 288;
a second Cas12f1 protein having the same amino acid sequence as the first Cas12f1 protein; and
a guide RNA in which a scaffold region represented by a nucleotide sequence selected from SEQ ID NO: 125, SEQ ID NO: 126, SEQ ID NO: 477, and SEQ ID NO: 478, a spacer region comprising, as a guide sequence, a nucleotide sequence selected from SEQ ID NOS: 51 to 100 and SEQ ID NOS: 245 to 272, and a U-rich tail are sequentially linked to each other in a 5' to 3' direction,
wherein the first Cas12f1 protein and the second Cas12f1 protein form a dimer,
the scaffold region of the guide RNA and the dimer formed by the Cas12f1 proteins interact with each other to form a complex,
the U-rich tail is represented by a sequence selected from 5'-(UaN)dUe-3', 5'-UaVUaVUe-3', and 5'-UaVUaVUaVUe-3' wherein N is A, C, G or U, each V is independently A, C or G, a is an integer from 0 to 4, d is an integer from 0 to 3, and e is an integer from 1 to 10, and
the spacer region is capable of forming a complementary bond with a target sequence present in CEP290 gene.

4. A vector comprising:
(i) a nucleic acid that encodes a Cas12f1 protein represented by an amino acid sequence selected from SEQ ID NO: 129 or SEQ ID NO: 288; and
a nucleic acid that encodes a guide RNA, in which a scaffold region represented by a nucleotide sequence selected from SEQ ID NO: 125, SEQ ID NO: 126, SEQ ID NO: 477, and SEQ ID NO: 478, a spacer region comprising, as a guide sequence, a nucleotide sequence selected from SEQ ID NOS: 51 to 100 and SEQ ID NOS: 245 to 272, and a U-rich tail are sequentially linked to each other, wherein the U-rich tail is represented by a sequence selected from 5'-(UaN)dUe-3', 5'-UaVUaVUe-3', and 5'-UaVUaVUaVUe-3' wherein N is A, C, G or U, each V is independently A, C or G, a is an integer from 0 to 4, d is an integer from 0 to 3, and e is an integer from 1 to 10, or
(ii) a nucleic acid that encodes a Cas12f1 protein represented by an amino acid sequence selected from SEQ ID NO: 129 or SEQ ID NO: 288; and
a nucleic acid that encodes a first guide RNA in which a first scaffold region represented by a nucleotide sequence selected from SEQ ID NO: 125, SEQ ID NO: 126, SEQ ID NO: 477, and SEQ ID NO: 478, a first spacer region comprising, as a guide sequence, a nucleotide sequence selected from SEQ ID NOS: 51 to 74 and SEQ ID NOS: 245 to 256, and a first U-rich tail are sequentially linked to each other; and
a nucleic acid that encodes a second guide RNA in which a second scaffold region represented by a nucleotide sequence selected from SEQ ID NO: 125, SEQ ID NO: 126, SEQ ID NO: 477, and SEQ ID NO: 478, a second spacer region comprising, as a guide sequence, a nucleotide sequence selected from SEQ ID NOS: 75 to 100 and SEQ ID NOS: 257 to 272, and a second U-rich tail are sequentially linked to each other, wherein the first U-rich tail and the second U-rich tail are each independently represented by a sequence selected from 5'-(UaN)dUe-3', 5'-UaVUaVUe-3', and 5'-UaVUaVUaVUe-3' wherein N is A, C, G or U, each V is independently A, C or G, a is an integer from 0 to 4, d is an integer from 0 to 3, and e is an integer from 1 to 10.

5. The vector of claim 4, wherein
i) the vector is one or more selected from: plasmid; mRNA (transcript); PCR amplicon; retroviral (retrovirus) vector; lentiviral (lentivirus) vector; adenoviral (adenovirus) vector; adeno-associated viral (adeno-associated virus; AAV) vector; vaccinia viral (vaccinia virus) vector; poxviral (poxvirus) vector; and herpes simplex viral (herpes simplex virus) vector; and/or
ii) the vector is a single vector.

6. The vector of claim 4 or claim 5, further comprising at least one unit of a shRNA, which decreases activity of one or more genes selected from ATM1, XRCC4, XLF-1, XRCC6, LIG4, and DCLRE1C, or a nucleic acid encoding the shRNA,
preferably wherein the vector is as defined in claim 4(ii),
wherein the first spacer region of the first guide RNA comprises, as a guide sequence, the nucleotide sequence of SEQ ID NO: 64,
the second spacer region of the second guide RNA comprises, as a guide sequence, the nucleotide sequence of SEQ ID NO: 92 or SEQ ID NO: 96,
the vector comprises two units of the nucleic acid encoding the shRNA, and
the vector is an adeno-associated viral vector and is a single vector.

7. A CRISPR/Cas12f1 composition comprising:
(i) a Cas12f1 protein represented by an amino acid sequence selected from SEQ ID NO: 129 or SEQ ID NO: 288, or a nucleic acid encoding the Cas12f1 protein; and
a guide RNA in which a scaffold region represented by a nucleotide sequence selected from SEQ ID NO: 125, SEQ ID NO: 126, SEQ ID NO: 477, and SEQ ID NO: 478, a spacer region comprising, as a guide sequence, a nucleotide sequence selected from SEQ ID NOS: 51 to 100 and SEQ ID NOS: 245 to 272, and a U-rich tail are sequentially linked to each other, or a nucleic acid encoding the guide RNA,
wherein the Cas12f1 protein and the scaffold region of the guide RNA are capable of interacting with each other to form a complex,
the spacer region of the guide RNA is capable of forming a complementary bond with a target sequence present in CEP290 gene, and
the U-rich tail is represented by a sequence selected from 5'-(UaN)dUe-3', 5'-UaVUaVUe-3', and 5'-UaVUaVUaVUe-3' wherein N is A, C, G or U, each V is independently A, C or G, a is an integer from 0 to 4, d is an integer from 0 to 3, and e is an integer from 1 to 10, or
(ii) a Cas12f1 protein represented by an amino acid sequence selected from SEQ ID NO: 129 or SEQ ID NO: 288, or a nucleic acid encoding the Cas12f1 protein; and
a first guide RNA in which a first scaffold region represented by a nucleotide sequence selected from SEQ ID NO: 125, SEQ ID NO: 126, SEQ ID NO: 477, and SEQ ID NO: 478, a first spacer region comprising, as a guide sequence, a nucleotide sequence selected from SEQ ID NOS: 51 to 74 and SEQ ID NOS: 245 to 256, and a first U-rich tail are sequentially linked to each other, or a nucleic acid encoding the first guide RNA; and
a second guide RNA in which a second scaffold region represented by a nucleotide sequence selected from SEQ ID NO: 125, SEQ ID NO: 126, SEQ ID NO: 477, and SEQ ID NO: 478, a second spacer region comprising, as a guide sequence, a nucleotide sequence selected from SEQ ID NOS: 75 to 100 and SEQ ID NOS: 257 to 272, and a second U-rich tail are sequentially linked to each other, or a nucleic acid encoding the second guide RNA,
wherein the Cas12f1 protein and the first scaffold region are capable of interacting with each other to form a complex,
the Cas12f1 protein and the second scaffold region are capable of interacting with each other to form a complex,
the first spacer region and the second spacer region are each independently capable of forming a complementary bond with a target sequence present in CEP290 gene, and
the first U-rich tail and the second U-rich tail are each independently represented by a sequence selected from 5'-(UaN)dUe-3', 5'-UaVUaVUe-3', and 5'-UaVUaVUaVUe-3' wherein N is A, C, G or U, each V is independently A, C or G, a is an integer from 0 to 4, d is an integer from 0 to 3, and e is an integer from 1 to 10.

8. The CRISPR/Cas12f1 composition of claim 7, further comprising at least one unit of a shRNA, which decreases activity of one or more genes selected from ATM1, XRCC4, XLF-1, XRCC6, LIG4, and DCLRE1C, or a nucleic acid encoding the shRNA.

9. The CRISPR/Cas12f1 composition of claim 8, wherein the CRISPR/Cas12f1 composition comprises the first guide RNA and the second guide RNA,
the first spacer region of the first guide RNA comprises, as a guide sequence, the nucleotide sequence of SEQ ID NO: 64,
the second spacer region of the second guide RNA comprises, as a guide sequence, the nucleotide sequence of SEQ ID NO: 92 or SEQ ID NO: 96, and
the CRISPR/Cas12f1 composition comprises two units of the shRNA or the nucleic acid encoding the shRNA.

10. An *in vitro* or *ex vivo* method for editing CEP290 gene that comprises IVS26 mutation (c.2991 + 1655A>G), comprising:
introducing a CRISPR/Cas12f1 composition into a human cell, a non-human animal cell having the CEP290 gene, a cell obtained from a human with LCA10 disease, or a cell obtained from a non-human animal with LCA10 disease, and
wherein the CRISPR/Cas12f1 composition comprises:
a Cas12f1 protein represented by an amino acid sequence selected from SEQ ID NO: 129 or SEQ ID NO: 288, or a nucleic acid encoding the Cas12f1 protein; and
a first guide RNA in which a first scaffold region represented by a nucleotide sequence selected from SEQ ID NO: 125, SEQ ID NO: 126, SEQ ID NO: 477, and SEQ ID NO: 478, a first spacer region comprising, as a guide sequence, a nucleotide sequence selected from SEQ ID NOS: 51 to 74 and SEQ ID NOS: 245 to 256, and a first U-rich tail are sequentially linked to each other, or a nucleic acid encoding the first guide RNA; and
a second guide RNA in which a second scaffold region represented by a nucleotide sequence selected from SEQ ID NO: 125, SEQ ID NO: 126, SEQ ID NO: 477, and SEQ ID NO: 478, a second spacer region comprising, as a guide sequence, a nucleotide sequence selected from SEQ ID NOS: 75 to 100 and SEQ ID NOS: 257 to 272, and a second U-rich tail are sequentially linked to each other, or a nucleic acid encoding the second guide RNA,
wherein the Cas12f1 protein and the first scaffold region are capable of interacting with each other to form a complex,
the Cas12f1 protein and the second scaffold region are capable of interacting with each other to form a complex,
the first spacer region of the first guide RNA of the CRISPR/Cas12f1 composition targets a region between the 3' end of exon 26 of the CEP290 gene and the IVS26 mutation (c.2991 + 1655A>G) of the CEP290 gene,
the second spacer region of the second guide RNA of the CRISPR/Cas12f1 composition targets a region between the IVS26 mutation (c.2991 + 1655A>G) of the CEP290 gene and the 5' end of exon 27 of the CEP290 gene,
the first U-rich tail and the second U-rich tail are each independently represented by a sequence selected from 5'-(UaN)dUe-3', 5'-UaVUaVUe-3', and 5'-UaVUaVUaVUe-3' wherein N is A, C, G or U, each V is independently A, C or G, a is an integer from 0 to 4, d is an integer from 0 to 3, and e is an integer from 1 to 10, and
introduction of the CRISPR/Cas12f1 composition to the subject induces removal of a partial nucleic acid sequence, which comprises the IVS26 mutation (c.2991 + 1655A>G), in a region of intron 26 of the CEP290 gene.

11. The method of claim 10, wherein the CRISPR/Cas12f1 composition further comprises at least one unit of a shRNA, which decreases activity of one or more genes selected from ATM1, XRCC4, XLF-1, XRCC6, LIG4, and DCLRE1C, or a nucleic acid encoding the shRNA.

12. The method of claim 11, wherein the first spacer region of the first guide RNA of the CRISPR/Cas12f1 composition comprises, as a guide sequence, the nucleotide sequence of SEQ ID NO: 64,
the second spacer region of the second guide RNA of the CRISPR/Cas12f1 composition comprises, as a guide sequence, the nucleotide sequence of SEQ ID NO: 92 or SEQ ID NO: 96, and
the CRISPR/Cas12f1 composition further comprises two units of the shRNA or the nucleic acid encoding the shRNA.

13. A pharmaceutical composition comprising the composition of any one of claims 7 to 9 for use in treating LCA10 disease.

14. The pharmaceutical composition for use of claim 13, wherein the subject is a human with LCA10 disease or a non-human animal with LCA10 disease.

## Patentansprüche

1. Guide-RNA für ein CRISPR/Cas12f1-System, umfassend:
eine Scaffold-Region, dargestellt durch eine aus SEQ ID NO: 125, SEQ ID NO: 126, SEQ ID NO: 477 und SEQ ID NO: 478 ausgewählte Nukleotidsequenz;
eine Spacer-Region, die als eine Guide-Sequenz eine aus SEQ ID NOS: 51 bis 100 und SEQ ID NOS: 245 bis 272 ausgewählte Nukleotidsequenz umfasst; und
einen U-reichen Schwanz,
wobei die Scaffold-Region, die Spacer-Region und der U-reiche Schwanz in einer 5'- zu 3'-Richtung sequentiell aneinander gekoppelt sind,
die Scaffold-Region eine Region ist, die dazu fähig ist, mit einem Cas12f1-Protein zu interagieren,
die Spacer-Region dazu fähig ist, eine komplementäre Bindung mit einer im CEP290-Gen vorhandenen Zielsequenz zu bilden, und
der U-reiche Schwanz durch eine aus 5'-(UaN)dUe-3', 5'-UaVUaVUe-3' und 5'-UaVUaVUaVUe-3' ausgewählte Sequenz dargestellt ist, wobei N A, C, G oder U ist, jedes V unabhängig A, C oder G ist, a eine ganze Zahl von 0 bis 4 ist, d eine ganze Zahl von 0 bis 3 ist und e eine ganze Zahl von 1 bis 10 ist.

2. DNA, die die Guide-RNA nach Anspruch 1 codiert.

3. Guide-RNA-Cas12f1-Proteinkomplex, umfassend:
ein erstes Cas12f1-Protein, dargestellt durch eine aus SEQ ID NO: 129 oder SEQ ID NO: 288 ausgewählte Aminosäuresequenz;
ein zweites Cas12f1-Protein, das dieselbe Aminosäuresequenz wie das erste Cas12f1-Protein aufweist; und
eine Guide-RNA, in der eine Scaffold-Region, dargestellt durch eine aus SEQ ID NO: 125, SEQ ID NO: 126, SEQ ID NO: 477 und SEQ ID NO: 478 ausgewählte Nukleotidsequenz, eine Spacer-Region, die als eine Guide-Sequenz eine aus SEQ ID NOS: 51 bis 100 und SEQ ID NOS: 245 bis 272 ausgewählte Nukleotidsequenz umfasst, und ein U-reicher Schwanz in einer 5'- zu 3'-Richtung sequentiell aneinander gekoppelt sind,
wobei das erste Cas12f1-Protein und das zweite Cas12f1-Protein ein Dimer bilden,
die Scaffold-Region der Guide-RNA und das durch die Cas12f1-Proteine gebildete Dimer miteinander interagieren, um einen Komplex zu bilden,
der U-reiche Schwanz durch eine aus 5'-(UaN)dUe-3', 5'-UaVUaVUe-3' und 5'-UaVUaVUaVUe-3' ausgewählte Sequenz dargestellt ist, wobei N A, C, G oder U ist, jedes V unabhängig A, C oder G ist, a eine ganze Zahl von 0 bis 4 ist, d eine ganze Zahl von 0 bis 3 ist und e eine ganze Zahl von 1 bis 10 ist, und
die Spacer-Region dazu fähig ist, eine komplementäre Bindung mit einer im CEP290-Gen vorhandenen Zielsequenz zu bilden.

4. Vektor, umfassend:
(i) eine Nukleinsäure, die ein Cas12f1-Protein codiert, dargestellt durch eine aus SEQ ID NO: 129 oder SEQ ID NO: 288 ausgewählte Aminosäuresequenz; und
eine Nukleinsäure, die eine Guide-RNA codiert, in der eine Scaffold-Region, dargestellt durch eine aus SEQ ID NO: 125, SEQ ID NO: 126, SEQ ID NO: 477 und SEQ ID NO: 478 ausgewählte Nukleotidsequenz, eine Spacer-Region, die als eine Guide-Sequenz eine aus SEQ ID NOS: 51 bis 100 und SEQ ID NOS: 245 bis 272 ausgewählte Nukleotidsequenz umfasst, und ein U-reicher Schwanz sequentiell aneinander gekoppelt sind, wobei der U-reiche Schwanz durch eine aus 5'-(UaN)dUe-3', 5'-UaVUaVUe-3' und 5'-UaVUaVUaVUe-3' ausgewählte Sequenz dargestellt ist, wobei N A, C, G oder U ist, jedes V unabhängig A, C oder G ist, a eine ganze Zahl von 0 bis 4 ist, d eine ganze Zahl von 0 bis 3 ist und e eine ganze Zahl von 1 bis 10 ist, oder
(ii) eine Nukleinsäure, die ein Cas12f1-Protein codiert, dargestellt durch eine aus SEQ ID NO: 129 oder SEQ ID NO: 288 ausgewählte Aminosäuresequenz; und
eine Nukleinsäure, die eine erste Guide-RNA codiert, in der eine erste Scaffold-Region, dargestellt durch eine aus SEQ ID NO: 125, SEQ ID NO: 126, SEQ ID NO: 477 und SEQ ID NO: 478 ausgewählte Nukleotidsequenz, eine erste Spacer-Region, die als eine Guide-Sequenz eine aus SEQ ID NOS: 51 bis 74 und SEQ ID NOS: 245 bis 256 ausgewählte Nukleotidsequenz umfasst, und ein erster U-reicher Schwanz sequentiell aneinander gekoppelt sind; und
eine Nukleinsäure, die eine zweite Guide-RNA codiert, in der eine zweite Scaffold-Region, dargestellt durch eine aus SEQ ID NO: 125, SEQ ID NO: 126, SEQ ID NO: 477 und SEQ ID NO: 478 ausgewählte Nukleotidsequenz, eine zweite Spacer-Region, die als eine Guide-Sequenz eine aus SEQ ID NOS: 75 bis 100 und SEQ ID NOS: 257 bis 272 ausgewählte Nukleotidsequenz umfasst, und ein zweiter U-reicher Schwanz sequentiell aneinander gekoppelt sind, wobei der erste U-reiche Schwanz und der zweite U-reiche Schwanz jeweils unabhängig durch eine aus 5'-(UaN)dUe-3', 5'-UaVUaVUe-3' und 5'-UaVUaVUaVUe-3' ausgewählte Sequenz dargestellt sind, wobei N A, C, G oder U ist, jedes V unabhängig A, C oder G ist, a eine ganze Zahl von 0 bis 4 ist, d eine ganze Zahl von 0 bis 3 ist und e eine ganze Zahl von 1 bis 10 ist.

5. Vektor nach Anspruch 4, wobei
i) der Vektor eines oder mehrere der folgenden ist: Plasmid; mRNA (Transkript); PCR-Amplicon; retroviraler (Retrovirus-)Vektor; lentiviraler (Lentivirus-)Vektor; adenoviraler (Adenovirus-)Vektor; Adeno-assoziierter viraler Vektor (Adenoassoziiertes Virus; AAV-Vektor); Vaccinia-viraler (Vaccinia-Virus-)Vektor; poxviraler (Poxvirus-)Vektor; und Herpes-simplex-viraler (Herpes-simplex-Virus-)Vektor; und/oder
ii) der Vektor ein einzelner Vektor ist.

6. Vektor nach Anspruch 4 oder Anspruch 5, weiter umfassend mindestens eine Einheit einer shRNA, die die Aktivität von einem oder mehreren aus ATM1, XRCC4, XLF-1, XRCC6, LIG4 und DCLRE1C ausgewählten Genen verringert, oder eine die shRNA codierende Nukleinsäure,
bevorzugt wobei der Vektor wie in Anspruch 4(ii) definiert ist,
wobei die erste Spacer-Region der ersten Guide-RNA als eine Guide-Sequenz die Nukleotidsequenz von SEQ ID NO: 64 umfasst,
die zweite Spacer-Region der zweiten Guide-RNA als eine Guide-Sequenz die Nukleotidsequenz von SEQ ID NO: 92 oder SEQ ID NO: 96 umfasst,
der Vektor zwei Einheiten der die shRNA codierenden Nukleinsäure umfasst, und
der Vektor ein Adeno-assoziierter viraler Vektor ist und ein einzelner Vektor ist.

7. CRISPR/Cas12f1-Zusammensetzung, umfassend:
(i) ein Cas12f1-Protein, dargestellt durch eine aus SEQ ID NO: 129 oder SEQ ID NO: 288 ausgewählte Aminosäuresequenz, oder eine das Cas12f1-Protein codierende Nukleinsäure; und
eine Guide-RNA, in der eine Scaffold-Region, dargestellt durch eine aus SEQ ID NO: 125, SEQ ID NO: 126, SEQ ID NO: 477 und SEQ ID NO: 478 ausgewählte Nukleotidsequenz, eine Spacer-Region, die als eine Guide-Sequenz eine aus SEQ ID NOS: 51 bis 100 und SEQ ID NOS: 245 bis 272 ausgewählte Nukleotidsequenz umfasst, und ein U-reicher Schwanz sequentiell aneinander gekoppelt sind, oder eine die Guide-RNA codierende Nukleinsäure,
wobei das Cas12f1-Protein und die Scaffold-Region der Guide-RNA dazu fähig sind, miteinander zu interagieren, um einen Komplex zu bilden,
die Spacer-Region der Guide-RNA dazu fähig ist, eine komplementäre Bindung mit einer im CEP290-Gen vorhandenen Zielsequenz zu bilden, und
der U-reiche Schwanz durch eine aus 5'-(UaN)dUe-3', 5'-UaVUaVUe-3' und 5'-UaVUaVUaVUe-3' ausgewählte Sequenz dargestellt ist, wobei N A, C, G oder U ist, jedes V unabhängig A, C oder G ist, a eine ganze Zahl von 0 bis 4 ist, d eine ganze Zahl von 0 bis 3 ist und e eine ganze Zahl von 1 bis 10 ist, oder
(ii) ein Cas12f1-Protein, dargestellt durch eine aus SEQ ID NO: 129 oder SEQ ID NO: 288 ausgewählte Aminosäuresequenz, oder eine das Cas12f1-Protein codierende Nukleinsäure; und
eine erste Guide-RNA, in der eine erste Scaffold-Region, dargestellt durch eine aus SEQ ID NO: 125, SEQ ID NO: 126, SEQ ID NO: 477 und SEQ ID NO: 478 ausgewählte Nukleotidsequenz, eine erste Spacer-Region, die als eine Guide-Sequenz eine aus SEQ ID NOS: 51 bis 74 und SEQ ID NOS: 245 bis 256 ausgewählte Nukleotidsequenz umfasst, und ein erster U-reicher Schwanz sequentiell aneinander gekoppelt sind, oder eine die erste Guide-RNA codierende Nukleinsäure; und
eine zweite Guide-RNA, in der eine zweite Scaffold-Region, dargestellt durch eine aus SEQ ID NO: 125, SEQ ID NO: 126, SEQ ID NO: 477 und SEQ ID NO: 478 ausgewählte Nukleotidsequenz, eine zweite Spacer-Region, die als eine Guide-Sequenz eine aus SEQ ID NOS: 75 bis 100 und SEQ ID NOS: 257 bis 272 ausgewählte Nukleotidsequenz umfasst, und ein zweiter U-reicher Schwanz sequentiell aneinander gekoppelt sind, oder eine die zweite Guide-RNA codierende Nukleinsäure,
wobei das Cas12f1-Protein und die erste Scaffold-Region dazu fähig sind, miteinander zu interagieren, um einen Komplex zu bilden,
das Cas12f1-Protein und die zweite Scaffold-Region dazu fähig sind, miteinander zu interagieren, um einen Komplex zu bilden,
die erste Spacer-Region und die zweite Spacer-Region jeweils unabhängig dazu fähig sind, eine komplementäre Bindung mit einer im CEP290-Gen vorhandenen Zielsequenz zu bilden, und
der erste U-reiche Schwanz und der zweite U-reiche Schwanz jeweils unabhängig durch eine aus 5'-(UaN)dUe-3', 5'-UaVUaVUe-3' und 5'-UaVUaVUaVUe-3' ausgewählte Sequenz dargestellt sind, wobei N A, C, G oder U ist, jedes V unabhängig A, C oder G ist, a eine ganze Zahl von 0 bis 4 ist, d eine ganze Zahl von 0 bis 3 ist und e eine ganze Zahl von 1 bis 10 ist.

8. CRISPR/Cas12f1-Zusammensetzung nach Anspruch 7, weiter umfassend mindestens eine Einheit einer shRNA, die die Aktivität von einem oder mehreren aus ATM1, XRCC4, XLF-1, XRCC6, LIG4 und DCLRE1C ausgewählten Genen verringert, oder eine die shRNA codierende Nukleinsäure.

9. CRISPR/Cas12f1-Zusammensetzung nach Anspruch 8, wobei die CRISPR/Cas12f1-Zusammensetzung die erste Guide-RNA und die zweite Guide-RNA umfasst,
die erste Spacer-Region der ersten Guide-RNA als eine Guide-Sequenz die Nukleotidsequenz von SEQ ID NO: 64 umfasst,
die zweite Spacer-Region der zweiten Guide-RNA als eine Guide-Sequenz die Nukleotidsequenz von SEQ ID NO: 92 oder SEQ ID NO: 96 umfasst, und
die CRISPR/Cas12f1-Zusammensetzung zwei Einheiten der shRNA oder der die shRNA codierenden Nukleinsäure umfasst.

10. *In-vitro-* oder *Ex-vivo-*Verfahren zum Editieren eines CEP290-Gens, das eine IVS26-Mutation (c.2991 + 1655A>G) umfasst, umfassend:
Einführen einer CRISPR/Cas12f1-Zusammensetzung in eine menschliche Zelle, eine nichtmenschliche tierische Zelle mit dem CEP290-Gen, eine aus einem Menschen mit LCA10-Erkrankung erhaltene Zelle oder eine aus einem nichtmenschlichen Tier mit LCA10-Erkrankung erhaltene Zelle, und
wobei die CRISPR/Cas12f1-Zusammensetzung umfasst:
ein Cas12f1-Protein, dargestellt durch eine aus SEQ ID NO: 129 oder SEQ ID NO: 288 ausgewählte Aminosäuresequenz, oder eine das Cas12f1-Protein codierende Nukleinsäure; und
eine erste Guide-RNA, in der eine erste Scaffold-Region, dargestellt durch eine aus SEQ ID NO: 125, SEQ ID NO: 126, SEQ ID NO: 477 und SEQ ID NO: 478 ausgewählte Nukleotidsequenz, eine erste Spacer-Region, die als eine Guide-Sequenz eine aus SEQ ID NOS: 51 bis 74 und SEQ ID NOS: 245 bis 256 ausgewählte Nukleotidsequenz umfasst, und ein erster U-reicher Schwanz sequentiell aneinander gekoppelt sind, oder eine die erste Guide-RNA codierende Nukleinsäure; und
eine zweite Guide-RNA, in der eine zweite Scaffold-Region, dargestellt durch eine aus SEQ ID NO: 125, SEQ ID NO: 126, SEQ ID NO: 477 und SEQ ID NO: 478 ausgewählte Nukleotidsequenz, eine zweite Spacer-Region, die als eine Guide-Sequenz eine aus SEQ ID NOS: 75 bis 100 und SEQ ID NOS: 257 bis 272 ausgewählte Nukleotidsequenz umfasst, und ein zweiter U-reicher Schwanz sequentiell aneinander gekoppelt sind, oder eine die zweite Guide-RNA codierende Nukleinsäure,
wobei das Cas12f1-Protein und die erste Scaffold-Region dazu fähig sind, miteinander zu interagieren, um einen Komplex zu bilden,
das Cas12f1-Protein und die zweite Scaffold-Region dazu fähig sind, miteinander zu interagieren, um einen Komplex zu bilden,
die erste Spacer-Region der ersten Guide-RNA der CRISPR/Cas12f1-Zusammensetzung auf eine Region zwischen dem 3'-Ende von Exon 26 des CEP290-Gens und der IVS26-Mutation (c.2991 + 1655A>G) des CEP290-Gens abzielt,
die zweite Spacer-Region der zweiten Guide-RNA der CRISPR/Cas12f1-Zusammensetzung auf eine Region zwischen der IVS26-Mutation (c.2991 + 1655A>G) des CEP290-Gens und dem 5'-Ende von Exon 27 des CEP290-Gens abzielt,
der erste U-reiche Schwanz und der zweite U-reiche Schwanz jeweils unabhängig durch eine aus 5'-(UaN)dUe-3', 5'-UaVUaVUe-3' und 5'- UaVUaVUaVUe-3' ausgewählte Sequenz dargestellt sind, wobei N A, C, G oder U ist, jedes V unabhängig A, C oder G ist, a eine ganze Zahl von 0 bis 4 ist, d eine ganze Zahl von 0 bis 3 ist und e eine ganze Zahl von 1 bis 10 ist, und
das Einführen der CRISPR/Cas12f1-Zusammensetzung in das Subjekt die Entfernung einer partiellen Nukleinsäuresequenz induziert, die die IVS26-Mutation (c.2991 + 1655A>G) umfasst, in einer Region von Intron 26 des CEP290-Gens.

11. Verfahren nach Anspruch 10, wobei die CRISPR/Cas12f1-Zusammensetzung weiter mindestens eine Einheit einer shRNA umfasst, die die Aktivität von einem oder mehreren aus ATM1, XRCC4, XLF-1, XRCC6, LIG4 und DCLRE1C ausgewählten Genen verringert, oder eine die shRNA codierende Nukleinsäure.

12. Verfahren nach Anspruch 11, wobei die erste Spacer-Region der ersten Guide-RNA der CRISPR/Cas12f1-Zusammensetzung als eine Guide-Sequenz die Nukleotidsequenz von SEQ ID NO: 64 umfasst,
die zweite Spacer-Region der zweiten Guide-RNA der CRISPR/Cas12f1-Zusammensetzung als eine Guide-Sequenz die Nukleotidsequenz von SEQ ID NO: 92 oder SEQ ID NO: 96 umfasst, und
die CRISPR/Cas12f1-Zusammensetzung weiter zwei Einheiten der shRNA oder der die shRNA codierenden Nukleinsäure umfasst.

13. Pharmazeutische Zusammensetzung, die die Zusammensetzung nach einem der Ansprüche 7 bis 9 zur Verwendung bei der Behandlung von LCA10-Erkrankung umfasst.

14. Pharmazeutische Zusammensetzung zur Verwendung nach Anspruch 13, wobei das Subjekt ein Mensch mit LCA10-Erkrankung oder ein nichtmenschliches Tier mit LCA10-Erkrankung ist.

## Revendications

1. ARN guide pour un système CRISPR/Cas12f1, comprenant :
une région échafaudage, représentée par une séquence nucléotidique sélectionnée parmi SEQ ID NO : 125, SEQ ID NO : 126, SEQ ID NO : 477 et SEQ ID NO : 478 ;
une région espaceur, comprenant, en tant que séquence guide, une séquence nucléotidique sélectionnée parmi SEQ ID NOs : 51 à 100 et SEQ ID NOs : 245 à 272 ; et
une queue riche en U,
dans lequel la région échafaudage, la région espaceur et la queue riche en U sont liées séquentiellement les unes aux autres dans une direction 5' à 3',
la région échafaudage est une région apte à interagir avec une protéine Cas12f1,
la région espaceur est apte à former une liaison complémentaire avec une séquence cible présente dans le gène CEP290, et
la queue riche en U est représentée par une séquence sélectionnée parmi 5'-(UaN)dUe-3', 5'- UaVUaVUe-3' et 5'-UaVUaVUaVUe-3', dans laquelle N est A, C, G ou U, chaque V est indépendamment A, C ou G, a est un nombre entier de 0 à 4, d est un nombre entier de 0 à 3, et e est un nombre entier de 1 à 10.

2. ADN codant l'ARN guide selon la revendication 1.

3. Complexe protéine Cas12f1-ARN guide, comprenant :
une première protéine Cas12f1 représentée par une séquence d'acides aminés sélectionnée parmi SEQ ID NO : 129 ou SEQ ID NO : 288 ;
une deuxième protéine Cas12f1, présentant la même séquence d'acides aminés que la première protéine Cas12f1 ; et
un ARN guide, dans lequel une région échafaudage représentée par une séquence nucléotidique sélectionnée parmi SEQ ID NO : 125, SEQ ID NO : 126, SEQ ID NO : 477 et SEQ ID NO : 478, une région espaceur comprenant, en tant que séquence guide, une séquence nucléotidique sélectionnée parmi SEQ ID NOs : 51 à 100 et SEQ ID NOs : 245 à 272, et une queue riche en U, sont liées séquentiellement les unes aux autres dans une direction 5' à 3',
dans lequel la première protéine Cas12f1 et la deuxième protéine Cas12f1 forment un dimère,
la région échafaudage de l'ARN guide et le dimère formé par les protéines Cas12f1 interagissent l'un avec l'autre de manière à former un complexe,
la queue riche en U est représentée par une séquence sélectionnée parmi 5'-(UaN)dUe-3', 5'-UaVUaVUe-3' et 5'-UaVUaVUaVUe-3', dans lequel N est A, C, G ou U, chaque V est indépendamment A, C ou G, a est un nombre entier de 0 à 4, d est un nombre entier de 0 à 3, et e est un nombre entier de 1 à 10, et
la région espaceur est apte à former une liaison complémentaire avec une séquence cible présente dans le gène CEP290.

4. Vecteur comprenant :
(i) un acide nucléique, qui code une protéine Cas12f1 représentée par une séquence d'acides aminés sélectionnée parmi SEQ ID NO : 129 ou SEQ ID NO : 288 ; et
un acide nucléique, qui code un ARN guide, dans lequel une région échafaudage représentée par une séquence nucléotidique sélectionnée parmi SEQ ID NO : 125, SEQ ID NO : 126, SEQ ID NO : 477 et SEQ ID NO : 478, une région espaceur comprenant, en tant que séquence guide, une séquence nucléotidique sélectionnée parmi SEQ ID NOs : 51 à 100 et SEQ ID NOs : 245 à 272, et une queue riche en U, sont liées séquentiellement les unes aux autres, dans lequel la queue riche en U est représentée par une séquence sélectionnée parmi 5'-(UaN)dUe-3', 5'-UaVUaVUe-3' et 5'-UaVUaVUaVUe-3', dans laquelle N est A, C, G ou U, chaque V est indépendamment A, C ou G, a est un nombre entier de 0 à 4, d est un nombre entier de 0 à 3, et e est un nombre entier de 1 à 10, ou
(ii) un acide nucléique qui code une protéine Cas12f1 représentée par une séquence d'acides aminés sélectionnée parmi SEQ ID NO : 129 ou SEQ ID NO : 288 ; et
un acide nucléique qui code un premier ARN guide dans lequel une première région échafaudage représentée par une séquence nucléotidique sélectionnée parmi SEQ ID NO : 125, SEQ ID NO : 126, SEQ ID NO : 477 et SEQ ID NO : 478, une première région espaceur comprenant, en tant que séquence guide, une séquence nucléotidique sélectionnée parmi SEQ ID NOs : 51 à 74 et SEQ ID NOs : 245 à 256, et une première queue riche en U sont liées séquentiellement les unes aux autres ; et
un acide nucléique qui code un deuxième ARN guide dans lequel une deuxième région échafaudage représentée par une séquence nucléotidique sélectionnée parmi SEQ ID NO : 125, SEQ ID NO : 126, SEQ ID NO : 477 et SEQ ID NO : 478, une deuxième région espaceur comprenant, en tant que séquence guide, une séquence nucléotidique sélectionnée parmi SEQ ID NOs : 75 à 100 et SEQ ID NOs : 257 à 272, et une deuxième queue riche en U sont liées séquentiellement les unes aux autres, dans lequel la première queue riche en U et la deuxième queue riche en U sont chacune indépendamment représentées par une séquence sélectionnée parmi 5'-(UaN)dUe-3', 5'-UaVUaVUe-3' et 5'-UaVUaVUaVUe-3', dans laquelle N est A, C, G ou U, chaque V est indépendamment A, C ou G, a est un nombre entier de 0 à 4, d est un nombre entier de 0 à 3, et e est un nombre entier de 1 à 10.

5. Vecteur selon la revendication 4, dans lequel
i) le vecteur est un ou plusieurs éléments sélectionnés parmi : plasmide ; ARNm (transcrit) ; amplicon PCR ; vecteur rétroviral (rétrovirus) ; vecteur lentiviral (lentivirus) ; vecteur adénoviral (adénovirus) ; vecteur viral adéno-associé (virus adéno-associé ; AAV) ; vecteur viral de la vaccine (virus de la vaccine) ; vecteur poxviral (poxvirus) ; et vecteur viral de l'herpès simplex (virus de l'herpès simplex) ; et/ou
ii) le vecteur est un vecteur unique.

6. Vecteur selon la revendication 4 ou la revendication 5, comprenant en outre au moins une unité d'un shRNA, qui diminue l'activité d'un ou plusieurs gènes sélectionnés parmi ATM1, XRCC4, XLF-1, XRCC6, LIG4 et DCLRE1C, ou un acide nucléique codant le shRNA,
dans lequel, de préférence, le vecteur est tel que défini dans la revendication 4(ii),
dans lequel la première région espaceur du premier ARN guide comprend, en tant que séquence guide, la séquence nucléotidique de SEQ ID NO : 64,
la deuxième région espaceur du deuxième ARN guide comprend, en tant que séquence guide, la séquence nucléotidique de SEQ ID NO : 92 ou SEQ ID NO : 96,
le vecteur comprend deux unités de l'acide nucléique codant le shRNA, et
le vecteur est un vecteur viral adéno-associé et est un vecteur unique.

7. Composition CRISPR/Cas12f1, comprenant :
(i) une protéine Cas12f1 représentée par une séquence d'acides aminés sélectionnée parmi SEQ ID NO : 129 ou SEQ ID NO : 288, ou un acide nucléique codant la protéine Cas12f1 ; et
un ARN guide dans lequel une région échafaudage représentée par une séquence nucléotidique sélectionnée parmi SEQ ID NO : 125, SEQ ID NO : 126, SEQ ID NO : 477 et SEQ ID NO : 478, une région espaceur comprenant, en tant que séquence guide, une séquence nucléotidique sélectionnée parmi SEQ ID NOs : 51 à 100 et SEQ ID NOs : 245 à 272, et une queue riche en U, sont liées séquentiellement les unes aux autres, ou un acide nucléique codant l'ARN guide,
dans lequel la protéine Cas12f1 et la région échafaudage de l'ARN guide sont aptes à interagir l'une avec l'autre pour former un complexe,
la région espaceur de l'ARN guide est apte à former une liaison complémentaire avec une séquence cible présente dans le gène CEP290, et
la queue riche en U est représentée par une séquence sélectionnée parmi 5'-(UaN)dUe-3', 5'-UaVUaVUe-3' et 5'-UaVUaVUaVUe-3', dans laquelle N est A, C, G ou U, chaque V est indépendamment A, C ou G, a est un nombre entier de 0 à 4, d est un nombre entier de 0 à 3, et e est un nombre entier de 1 à 10, ou
(ii) une protéine Cas12f1 représentée par une séquence d'acides aminés sélectionnée parmi SEQ ID NO : 129 ou SEQ ID NO : 288, ou un acide nucléique codant la protéine Cas12f1 ; et
un premier ARN guide dans lequel une première région échafaudage représentée par une séquence nucléotidique sélectionnée parmi SEQ ID NO : 125, SEQ ID NO : 126, SEQ ID NO : 477 et SEQ ID NO : 478, une première région espaceur comprenant, en tant que séquence guide, une séquence nucléotidique sélectionnée parmi SEQ ID NOs : 51 à 74 et SEQ ID NOs : 245 à 256, et une première queue riche en U, sont liées séquentiellement les unes aux autres, ou un acide nucléique codant le premier ARN guide ; et
un deuxième ARN guide, dans lequel une deuxième région échafaudage représentée par une séquence nucléotidique sélectionnée parmi SEQ ID NO : 125, SEQ ID NO : 126, SEQ ID NO : 477 et SEQ ID NO : 478, une deuxième région espaceur comprenant, en tant que séquence guide, une séquence nucléotidique sélectionnée parmi SEQ ID NOs : 75 à 100 et SEQ ID NOs : 257 à 272, et une deuxième queue riche en U, sont liées séquentiellement les unes aux autres, ou un acide nucléique codant le deuxième ARN guide,
dans lequel la protéine Cas12f1 et la première région échafaudage sont aptes à interagir l'une avec l'autre pour former un complexe,
la protéine Cas12f1 et la deuxième région échafaudage sont aptes à interagir l'une avec l'autre pour former un complexe,
la première région espaceur et la deuxième région espaceur sont chacune indépendamment aptes à former une liaison complémentaire avec une séquence cible présente dans le gène CEP290, et
la première queue riche en U et la deuxième queue riche en U sont chacune indépendamment représentées par une séquence sélectionnée parmi 5'-(UaN)dUe-3', 5'-UaVUaVUe-3' et 5'-UaVUaVUaVUe-3', dans laquelle N est A, C, G ou U, chaque V est indépendamment A, C ou G, a est un nombre entier de 0 à 4, d est un nombre entier de 0 à 3, et e est un nombre entier de 1 à 10.

8. Composition CRISPR/Cas12f1 selon la revendication 7, comprenant en outre au moins une unité d'un shRNA, qui diminue l'activité d'un ou plusieurs gènes sélectionnés parmi ATM1, XRCC4, XLF-1, XRCC6, LIG4 et DCLRE1C, ou un acide nucléique codant le shRNA.

9. Composition CRISPR/Cas12f1 selon la revendication 8, dans laquelle la composition CRISPR/Cas12f1 comprend le premier ARN guide et le deuxième ARN guide,
la première région espaceur du premier ARN guide comprend, en tant que séquence guide, la séquence nucléotidique de SEQ ID NO : 64,
la deuxième région espaceur du deuxième ARN guide comprend, en tant que séquence guide, la séquence nucléotidique de SEQ ID NO : 92 ou SEQ ID NO : 96, et
la composition CRISPR/Cas12f1 comprend deux unités du shRNA ou de l'acide nucléique codant le shRNA.

10. Procédé *in vitro* ou *ex vivo* d'édition du gène CEP290 qui comprend la mutation IVS26 (c.2991 + 1655A>G), comprenant :
l'introduction d'une composition CRISPR/Cas12f1 dans une cellule humaine, une cellule d'animal non humain présentant le gène CEP290, une cellule obtenue à partir d'un humain atteint de la maladie LCA10, ou une cellule obtenue à partir d'un animal non humain atteint de la maladie LCA10, et
dans lequel la composition CRISPR/Cas12f1 comprend :
une protéine Cas12f1 représentée par une séquence d'acides aminés sélectionnée parmi SEQ ID NO : 129 ou SEQ ID NO : 288, ou un acide nucléique codant la protéine Cas12f1 ; et
un premier ARN guide dans lequel une première région échafaudage représentée par une séquence nucléotidique sélectionnée parmi SEQ ID NO : 125, SEQ ID NO : 126, SEQ ID NO : 477 et SEQ ID NO : 478, une première région espaceur comprenant, en tant que séquence guide, une séquence nucléotidique sélectionnée parmi SEQ ID NOs : 51 à 74 et SEQ ID NOs : 245 à 256, et une première queue riche en U, sont liées séquentiellement les unes aux autres, ou un acide nucléique codant le premier ARN guide ; et
un deuxième ARN guide dans lequel une deuxième région échafaudage représentée par une séquence nucléotidique sélectionnée parmi SEQ ID NO : 125, SEQ ID NO : 126, SEQ ID NO : 477 et SEQ ID NO : 478, une deuxième région espaceur comprenant, en tant que séquence guide, une séquence nucléotidique sélectionnée parmi SEQ ID NOs : 75 à 100 et SEQ ID NOs : 257 à 272, et une deuxième queue riche en U, sont liées séquentiellement les unes aux autres, ou un acide nucléique codant le deuxième ARN guide,
dans lequel la protéine Cas12f1 et la première région échafaudage sont aptes à interagir l'une avec l'autre pour former un complexe,
la protéine Cas12f1 et la deuxième région échafaudage sont aptes à interagir l'une avec l'autre pour former un complexe,
la première région espaceur du premier ARN guide de la composition CRISPR/Cas12f1 cible une région entre l'extrémité 3' de l'exon 26 du gène CEP290 et la mutation IVS26 (c.2991 + 1655A>G) du gène CEP290,
la deuxième région espaceur du deuxième ARN guide de la composition CRISPR/Cas12f1 cible une région entre la mutation IVS26 (c.2991 + 1655A>G) du gène CEP290 et l'extrémité 5' de l'exon 27 du gène CEP290,
la première queue riche en U et la deuxième queue riche en U sont chacune indépendamment représentées par une séquence sélectionnée parmi 5'-(UaN)dUe-3', 5'-UaVUaVUe-3' et 5'-UaVUaVUaVUe-3', dans laquelle N est A, C, G ou U, chaque V est indépendamment A, C ou G, a est un nombre entier de 0 à 4, d est un nombre entier de 0 à 3, et e est un nombre entier de 1 à 10, et
l'introduction de la composition CRISPR/Cas12f1 dans le sujet induit l'élimination d'une séquence partielle d'acide nucléique, qui comprend la mutation IVS26 (c.2991 + 1655A>G), dans une région de l'intron 26 du gène CEP290.

11. Procédé selon la revendication 10, dans lequel la composition CRISPR/Cas12f1 comprend en outre au moins une unité d'un shRNA, qui diminue l'activité d'un ou plusieurs gènes sélectionnés parmi ATM1, XRCC4, XLF-1, XRCC6, LIG4 et DCLRE1C, ou un acide nucléique codant le shRNA.

12. Procédé selon la revendication 11, dans lequel la première région espaceur du premier ARN guide de la composition CRISPR/Cas12f1 comprend, en tant que séquence guide, la séquence nucléotidique de SEQ ID NO : 64,
la deuxième région espaceur du deuxième ARN guide de la composition CRISPR/Cas12f1 comprend, en tant que séquence guide, la séquence nucléotidique de SEQ ID NO : 92 ou SEQ ID NO : 96, et
la composition CRISPR/Cas12f1 comprend en outre deux unités du shRNA ou de l'acide nucléique codant le shRNA.

13. Composition pharmaceutique comprenant la composition selon l'une quelconque des revendications 7 à 9 destinée au traitement de la maladie LCA10.

14. Composition pharmaceutique destinée au traitement selon la revendication 13, dans laquelle le sujet est un humain atteint de la maladie LCA10 ou un animal non humain atteint de la maladie LCA10.
